# EUROPEAN PATENT APPLICATION

(11) **EP 4 595 954 A1**
(43) Date of publication of application: **06.08.2025**
(21) Application number: 24155402.1
(22) Date of filing: 01.02.2024
(51) Int. Cl.: A61K 9/28, A61K 31/785, A61K 47/32, A61K 47/38

(54) **COMPOSITIONS AND METHODS FOR TREATING BILE ACID MALABSORPTION**

(71) Applicant: Cosmo Technologies Ltd., Dublin D02KV60 Dublin (IE)
(72) Inventor: LONGO, Luigi Maria, 20045 Milan (IT); PAGANI, Stefania, 20045 Milan (IT)
(74) Representative: FRKelly

(57) **Abstract**

Pharmaceutical compositions comprising bile acid sequestrants, their use in treating bile acid malabsorption, and associated conditions such as bile acid diarrhea and colitis are described herein. Methods for treating bile acid malabsorption and associated conditions are also described.

## Description

### Field of the Invention

The present invention relates to pharmaceutical compositions comprising bile acid sequestrants, and their use in treating bile acid malabsorption, and associated conditions such as bile acid diarrhea and bile acid colitis, as well as methods for treating bile acid malabsorption and associated conditions.

### Background

Bile acids (also called bile salts) are the main functional components of bile, which is produced in the liver and stored in the gallbladder. After a meal, this stored bile is secreted into the duodenum, where it exerts its functional role in digestion. Bile acids are natural surfactants, which solubilize lipids and lipophilic substances through formation of micelles.

Bile acids are endogenous surfactants synthetized in the liver by the cytochrome P450-mediated oxidation of cholesterol and play a key role in the absorption of dietary fats from the small intestine. They are conjugated with the amino acids taurine or glycine, or with a sulfate or a glucuronide, and are then transported across canalicular membrane of the hepatocytes into the bile and stored in the gallbladder, from which they are secreted into the duodenum to solubilize fats contained into the diet.

After each meal, bile acids are released into duodenum, the first tract of the small intestine, where they exert their role as physiological detergents molecules, facilitating the absorption of lipids and liposoluble nutrients, including liposoluble vitamins, from the small intestine. Bile acid are ionized in the lumen of the small intestine, and the ionization increases their water solubility and renders bile acids impermeable to cell membranes, allowing them to reach the critical micellar concentration, which is the concentration above which micelles are formed. The micelles are needed in the small intestine for absorption of lipids and lipophilic substances such as some vitamins.

Once the bile acids have exerted their role in the duodenum and jejunum, about 95% of the secreted amount is reabsorbed by active transport in the ileum and delivered back to the liver via the portal vein. This process, known as enterohepatic circulation of bile acid, may involve hepatic conjugation and intestinal deconjugation. As an effect, only about 5% of the secreted amount is lost into the feces. Daily loss of Bile acids is compensated by de novo synthesis in the liver and thus, a constant bile acid pool is maintained. The intestinal bile acid uptake is mainly mediated by the apical sodium dependent bile salt transporter (ASBT). *(*Li T. et al., Regulation of Bile Acid and Cholesterol Metabolism by PPARs. Hindavvi Publishing Corporation, Vol 2009, Article ID 501739*).*

In humans, the bile acid pool consists of primary bile acids and secondary bile acids. The primary bile acids are synthesized from cholesterol exclusively in the liver through two pathways, the classic pathway (accounting for about 90% of bile acid synthesis) and the alternative pathway (accounting for about the remaining 10%). The secondary bile acids are synthesized from primary bile acids by bacterial enzymes of the natural microbiota resident into the intestine. (Li T. et al., Regulation of Bile Acid and Cholesterol Metabolism by PPARs. Hindavvi Publishing Corporation, Vol 2009, Article ID 501739*).*

Enterohepatic circulation of bile acids is an efficient process capable of cycling these important compounds between the liver and intestine multiple times during the day.

Abnormal bile acids homeostasis and lack of, or impaired, reabsorption into the bloodstream, can exacerbate several disorders including Crohn's disease (CD), hepatic microvascular dysplasia, inflammatory bowel disease (IBD), irritable bowel syndrome (IBS), colonic cancer, cholestasis, insufficient control of blood glucose and cardiovascular disease. Subsequently, lack of, or impaired, reabsorption of bile acids in the ileum and the enterohepatic bile acid circulation resulting in watery diarrhea. Bile acids in the large bowel cause abnormally high levels of water and salts to get into the large bowel from the bloodstream, causing watery diarrhea.

Bile Acid Malabsorption (BAM) is a defect in this enterohepatic circulation of bile acids, whereby increased proportions of the secreted bile acids are not reabsorbed in the ileum and reach the colon consequently. Bile acids in the colon activate increase fluid secretion, increase colonic motility and thereby decrease colonic transit time. Overall, this generates a chronic watery diarrhea (also known as Bile Acid Diarrhea), accompanied by further symptoms such as bloating, pain, faecal urgency and faecal incontinence. It is currently estimated that about 1% of the population has Bile Acid Diarrhea, and that about 30% of all irritable bowel syndrome with diarrhea (IBS-D) cases are due to Bile Acid Malabsorption. Bile Acid Malabsorption has also been associated with other diseases and conditions, such as microscopic colitis, Crohn's disease, HIV-related enteritis, persistent diarrhea following bacterial infection and exocrine pancreatic insufficiency.

Currently, Bile Acid Diarrhea is subdivided in 3 main types: type 1 results from terminal ileal resection/bypass or disease (for example: Crohn's disease or ileal resection), which results in failure of enterohepatic recycling of bile acids, and excess amounts entering the colon. Type 2 often referred to as primary (or idiopathic) bile acid diarrhea, is the most common cause of bile acid malabsorption and may account for at least 30% of individuals, who would otherwise be labeled as having IBS-D or functional diarrhea. Despite much investigation, the etiology and pathogenesis of type 2 Bile Acid Diarrhea is poorly understood. Type 3 includes causes not included with types 1 and 2 that may interfere with normal bile acid cycling, small intestinal motility, or composition of ileal contents: for example, small intestine bacterial overgrowth, chronic pancreatitis, celiac disease, etc. *(*DiBaise, Nutrition Issues in Practical Gastroenterology, Series #198, Practical Gastroenterology 2020). There is also a type 4, which is the excess of bile acid production as side effect of therapy with metformin.

To treat the diarrheic effects generated by the excess of bile acids in the colon, bile acid sequestrants substances are commonly used in clinical practice, although these products have no approval for such use and are approved for lowering the cholesterol in the blood. Cholestyramine, colestipol, and colesevelam are the three main bile acid sequestrants currently available in the US and European markets.

Currently used sequestrant substances are available as granules for suspension or capsule shape tablets that require a very high amount of active ingredient with several administrations per day. This creates issues with patient compliance.

First-generation bile acid sequestrants (or resins) such as cholestyramine or colestipol are available as powders or granules. However, patients are often intolerant to those sequestrants, due to side effects such as nausea, bloating, and constipation. Moreover, an efficacious treatment of BAD requires the administration of very high doses of these bile acid sequestrants: cholestyramine up to 36 g per day; colestipol up to 30 g per day (Wilcox et al., Aliment Pharmacol Ther 2014; 39: 923-939). These very high doses of cholestyramine or colestipol are not tolerated by many patients, because these compounds are in form of powders and have a very low palatability. Discontinuation rates have been reported to range from 34% to 60%. Colesevelam (and salts thereof, e.g., hydrochloride salt) is a second-generation bile acid sequestrant, which is commercially available as immediate release tablets or granules. Even though the recommended dose of colesevelam for treating BAD is reduced compared to cholestyramine and colestipol, it remains high and corresponds to 6 or 7 tablets per day. Given the chronicity of the treatment, there are patient compliance issues reported for colesevelam, because of the high number of tablets per day. When administered in form of granules, colesevelam is known to generate a so called "sand effect" in the mouth that can results particularly unpleasant for most of patients that are not encouraged to continue the therapy day by day. There is an immediate need for an improved formulation of bile acid sequestrants capable of increasing the patient's compliance; in particular, there is an immediate need for a formulation of bile acid sequestrants in form of tablets, with a high drug content of active substance per dosage unit while maintaining the dimensions suitable to allow for easy administration.

Currently available bile acid sequestrant is released in the upper GI tract. As such, the use of such compositions for treatment of BAM has several drawbacks, such as negative drug-drug interactions, decreased absorption of fat-soluble vitamins, and a negative impact on the absorption of other medications. Moreover, the available formulations of bile acid sequestrants have some adverse reactions (e.g., nausea, dyspepsia and bloating), which are due to their unspecific effect in the upper GI tract.

WO 2021/163007 (Viscera) provides a colesevelam colon specific drug delivery system for use in the treatment of cholestasis and/or cholestatic pruritis. Described therein is an oral drug delivery system comprising: a) a core comprising a therapeutically effective amount of at least one active agent present in an amount of from about 35% to about 65% w/w of the tablet core, and a drug release controlling component capable of providing release of the active agent primarily in a region selected from the group consisting of the lower gastrointestinal tract, the large intestine, the jejunum, the ileum, the cecum, the colon, the rectum, and combinations thereof, b) an outer coating encasing the tablet core, and optionally a plasticizer, wherein after ingestion by a patient the active agent is released primarily in the region selected from the group consisting of the lower gastrointestinal tract, the large intestine, the jejunum, the ileum, the cecum, the colon, the rectum, and combinations thereof.

The recommended dose for colesevelam for treating bile acid malabsorption is high (3.75 g per day), which corresponds to around 6 tablets comprising 625 mg colesevelam hydrochloride per day. The tablets can be administered either in a single non-fractionated administration or in multiple fractionated administrations, e.g., six tablets once per day, or three tablets twice per day, or two tablets thrice per day. Given the chronicity of the disease, and the high number of tablets per day, patient compliance issues often occur when treating with colesevelam.

The current commercially available formulations of bile acid sequestrants are characterized by immediate release in the stomach. The bile acid sequestrants therefore exert their pharmacological action starting in the duodenum: once the bile acids are released by the gallbladder in the duodenum, they are bound by the sequestrants and cannot exert their physiological digestive role of solubilization and absorption-enhancement of lipids and liposoluble vitamins. This leads to nutritional disorders and deficiencies of liposoluble vitamins.

Additionally, the effect on the bile acids in the upper GI is suboptimal when the disease to be treated is caused by an excess of bile acids entering the colon; in other words, the currently available formulations do not target only the excess of bile acids entering the colon in patient with bile acid malabsorption/bile acid diarrhea, but target the whole pool of bile acids released from the gallbladder and transiting the upper small intestine segments, i.e., the duodenum and jejunum.

Furthermore, bile acid sequestrants are non-specific, in that they bind a lot of different substances, preventing their absorption from the small intestine. Importantly, bile acid sequestrants are known to bind several drugs administered orally, a characteristic which has an impact on the systemic bioavailability of those drugs, because, once these are bound to the resin polymer chain, they are not absorbed and are excreted in the feces. Most orally administered drugs are absorbed in the upper GI tract, i.e., in the stomach, or in the duodenum or in the jejunum. The currently available formulations of bile acid sequestrants, which release the resin in the stomach, have a significant interaction with drugs absorbed in the upper GI tract, which leads to the need of administering these drugs at least 4 hours prior to bile acid sequestrants, leading to difficulties for the physician to draw an effective treatment schedule for patients taking several concomitant medications.

Finally, the currently available formulations of bile acid sequestrants, although in general are regarded to as safe, have some adverse reactions (e.g., nausea, dyspepsia, bloating), which are due to their effect in, and transit throughout, the whole GI tract, from the stomach to the colon.

There is therefore the need for new treatment options for bile acid malabsorption and bile acid diarrhea which overcome the above highlighted limitations of the currently available treatments.

### Summary of the Invention

The present invention provides pharmaceutical compositions which resolve the above-mentioned drawbacks. The pharmaceutical compositions described herein are characterized by a high drug load, allowing for reduced number of administrations per day, a delivery of the active substance in the low intestinal districts to avoid unwanted interference with the natural role of the bile acids.

According to a first aspect of the present invention, there is provided a unit dose pharmaceutical composition for oral administration comprising a tablet core and a gastro-resistant coating covering the tablet core, wherein the tablet core comprises colesevelam or a pharmaceutically acceptable salt thereof, wherein the colesevelam or a pharmaceutically acceptable salt thereof is at least 66 wt% of the tablet core, and wherein the colesevelam or a pharmaceutically acceptable salt thereof is present in the tablet core in an amount of from about 700 mg to about 1400 mg.

Optionally, the colesevelam or a pharmaceutically acceptable salt thereof, for example, colesevelam hydrochloride, is present in the tablet core in an amount of from 850 mg to 1200 mg

Optionally, the colesevelam or a pharmaceutically acceptable salt thereof, for example, colesevelam hydrochloride, is at least 70 wt%, or at least 75 wt%, or at least 76 wt%, or at least 80 wt%, or at least 81 wt% of the tablet core, optionally, at least 82 wt% of the tablet core. For example, the amount of colesevelam and/or pharmaceutically acceptable salts thereof (such as colesevelam hydrochloride) accounts for at least 66 wt% of the tablet core, such as an amount of from 66 wt% to 90 wt% of the tablet core, optionally in an amount of from 70 wt% to 90 wt% of the tablet core, optionally, in an amount of from 75 wt% to 90 wt% of the tablet core, such as from 76 wt% to 90 wt% of the tablet core, suitably 80 wt% to 90 wt% of the tablet core, such as from 81 wt% to 90 wt% of the tablet core, optionally, from 82 wt% to 90 wt% of the tablet core.

The weight percentages of colesevelam or a pharmaceutically acceptable salt(s) thereof refers to the weight percent of anhydrous colesevelam or a pharmaceutically acceptable salt(s) thereof, such as colesevelam hydrochloride. For example, "at least 66 wt% colesevelam and/or pharmaceutically acceptable salt thereof" implies "at least 66 wt% anhydrous colesevelam and/or pharmaceutically acceptable salt thereof," and in particular embodiments "at least 66 wt% anhydrous colesevelam hydrochloride." Hydrated forms of colesevelam may also be used, however, the amounts employed are adjusted to compensate for the amount of water present i.e., when using hydrated forms of colesevelam, the amount of water/hydrate present in the hydrated colesevelam does not contribute to the weight percentage of colesevelam based on dry weight.

Suitably, hydrated forms of colesevelam or pharmaceutically acceptable salts thereof that may be used in the present invention comprise less than about 20 wt% water based on the total weight of the hydrated colesevelam or pharmaceutically acceptable salt thereof, preferably less than about 10 wt%, e.g., about 1 wt%, or about 2% wt%, or 3 wt%, or 4 wt%, or 5 wt%, or 6 wt%, or 7 wt%, or 8 wt%, or 9 wt%, water based on the total weight of the hydrated colesevelam or pharmaceutically acceptable salt thereof or any fractions of the above integers. When using hydrated forms of colesevelam, the amount of water present in the hydrated colesevelam (shall be compensated for, thus in order to achieve 0.9 X g of colesevelam (based on dry weight colesevelam), X g of a hydrated colesevelam comprising 10% water would be required.

Optionally, the tablet core comprising colesevelam or a pharmaceutically acceptable salt thereof, for example, colesevelam hydrochloride, further comprises one or more of a diluent, a binder, a disintegrant, a glidant or a lubricant, preferably wherein the tablet core comprises a diluent, a binder, a glidant and a lubricant, and optionally a disintegrant.

Optionally, the tablet core comprising colesevelam or a pharmaceutically acceptable salt thereof, for example, colesevelam hydrochloride, comprises a diluent or mixture of two or more diluents selected from the group comprising: lactose, a sugar such as sucrose and fructose, dextrose, dextrates, dextrans, a polyol such as sorbitol, mannitol and xylitol, dibasic calcium phosphate, tribasic calcium phosphate, calcium sulphate, sodium chloride, starch (e.g., pregelatinized starch), and cellulose or a derivative thereof (e.g., methyl, ethyl or hydroxyethyl cellulose).

Preferably, the tablet core comprising colesevelam or a pharmaceutically acceptable salt thereof, for example, colesevelam hydrochloride, comprises a diluent or mixture of two or more diluents selected from the group comprising: cellulose and derivatives thereof, lactose, calcium phosphate, starch, modified starch, and mannitol.

Optionally, the diluent is microcrystalline cellulose, optionally the diluent is a porous microcrystalline cellulose having an average particle size of in the range of from 80 to 110 µm and an angle of repose of less than about 40°, preferably, said porous microcrystalline cellulose has an average particle size of about 90 µm and an angle of repose of about 34°.

Optionally, the diluent is present in an amount of from 6 to 21 wt% of the tablet core, preferably from 8 to 18 wt% of the tablet core, optionally the diluent is present in an amount of from 9 to 13 wt% of the tablet core.

Optionally, the tablet core comprising colesevelam or a pharmaceutically acceptable salt thereof, for example, colesevelam hydrochloride, comprises a binder selected from the group comprising: polyvinyl alcohol, a natural gum, gelatin, a polymethacrylate, cellulose or a derivative thereof (e.g., ethyl cellulose) and copovidone, or any combination thereof. Preferably the binder is copovidone or polyvinyl alcohol. More preferably, the binder is copovidone.

Optionally, the tablet core comprising colesevelam or a pharmaceutically acceptable salt thereof, for example, colesevelam hydrochloride, comprises a binder present in an amount of from 1 to 7 wt% of the tablet core, optionally the binder is present in an amount of from 2 to 6 wt% of the tablet core, such as in an amount of from 3 to 5 wt% of the tablet core.

Optionally, the tablet core comprising colesevelam or a pharmaceutically acceptable salt thereof, for example, colesevelam hydrochloride, comprises a disintegrant selected from the group comprising: sodium carboxymethyl cellulose, cross-linked sodium carboxymethyl cellulose, bentonite, alginic acid or a derivative or salt thereof, and crospovidone, or any combination thereof. Preferably, the disintegrant is crospovidone or cross-linked sodium carboxymethyl cellulose. More preferably, the disintegrant is crospovidone.

Optionally, the tablet core comprising colesevelam or a pharmaceutically acceptable salt thereof, for example, colesevelam hydrochloride, comprises a disintegrant present in an amount of from 0.01 to 2 wt% of the tablet core, optionally the disintegrant is present in an amount of from 0.1 to 1 wt% of the tablet core.

Optionally, the tablet core comprising colesevelam or a pharmaceutically acceptable salt thereof, for example, colesevelam hydrochloride, comprises a glidant selected from the group comprising: talc, starch, magnesium oxide, silica (silicon dioxide) and a silicate, such as magnesium or calcium silicate, or any combination thereof. Preferably, the glidant is silica, colloidal silicon dioxide, talc or magnesium oxide. More preferably, the glidant is colloidal silicon dioxide.

Optionally, the tablet core comprising colesevelam or a pharmaceutically acceptable salt thereof, for example, colesevelam hydrochloride, comprises a glidant present in an amount of from 0.1 to 2 wt% of the tablet core, optionally the glidant is present in an amount of from 0.5 to 1.5 wt% of the tablet core, further, optionally, the glidant is present in an amount of from 0.7 to 1.2 wt% of the tablet core.

Optionally, the tablet core comprising colesevelam or a pharmaceutically acceptable salt thereof, for example, colesevelam hydrochloride, comprises a lubricant selected from the group comprising: stearic acid or a salt thereof (e.g., magnesium, calcium or zinc stearate), polyethylene glycol, fumaric acid or a salt thereof, glyceryl behenate, glyceryl palmitostearate, wax, and sodium benzoate or any combination thereof. Preferably, the lubricant is stearic acid or a salt thereof, fumaric acid or a salt thereof, or polyethylene glycol.

Optionally, the tablet core comprising colesevelam or a pharmaceutically acceptable salt thereof, for example, colesevelam hydrochloride, comprises a lubricant present in an amount of from 0.1 to 2 wt% of the tablet core, optionally the lubricant is present in an amount of from 0.5 to 1.5 wt% of the tablet core, further optionally, the lubricant is present in an amount of from 0.7 to 1.2 wt% of the tablet core.

Optionally, the tablet core comprising colesevelam or a pharmaceutically acceptable salt thereof, for example, colesevelam hydrochloride, comprises microcrystalline cellulose, copovidone, colloidal silicon dioxide, magnesium stearate, and optionally crospovidone.

Optionally, the tablet core comprises:
colesevelam or a pharmaceutically acceptable salt thereof, for example, colesevelam hydrochloride,
microcrystalline cellulose in an amount of from 9 to 13 wt% of the tablet core,
copovidone in an amount of from 3 to 4 wt% of the tablet core,
colloidal silicon dioxide in an amount of from 0.5 to 1.5 wt% of the tablet core,
magnesium stearate in an amount of from 0.5 to 1.5 wt% of the tablet core, and optionally, crospovidone in an amount of from 0.1 to 1 wt% of the tablet core.

Optionally, the tablet core comprising colesevelam or a pharmaceutically acceptable salt thereof, for example, colesevelam hydrochloride, further comprises a sub-coating between the tablet core and the gastro-resistant coating, optionally, wherein the sub-coating comprises hydroxypropyl cellulose.

Optionally, the gastro-resistant coating comprises methacrylic acid/methyl methacrylate (1:1) copolymer.

Optionally, the gastro-resistant coating comprises methacrylic acid/methyl methacrylate (1:2) copolymer.

Optionally, the gastro-resistant coating comprises a mixture of methacrylic acid/methyl methacrylate (1:1) copolymer and of methacrylic acid/methyl methacrylate (1:2) copolymer, wherein methacrylic acid/methyl methacrylate (1:1) copolymer is present in a ratio ranging from 0.5 to 1 to 3 to 1 with respect to the other copolymer, preferably in a ratio of 1 to 1 with respect to the other copolymer, more preferably in a ratio of 2 to 1 with respect to the other copolymer.

Optionally, the gastro-resistant coating of the unit dose starts breaking or dissolving at or above a pH of 6, preferably at or above a pH of 6.5, more preferably at a pH of 6.8.

Optionally, the tablet core comprising colesevelam or a pharmaceutically acceptable salt thereof, for example, colesevelam hydrochloride, has a hardness of greater than about 70 N, optionally greater than about 80 N, optionally greater than about 90 N, optionally greater than about 120 N as determined in accordance with European Pharmacopoeia 2.9.8 or with US Pharmacopoeia monograph <1217>. The tablet core may have a hardness in the range of from 70 N to 450 N, such as from 80 N to 400 N, optionally from 90 N to 370 N, such as from 100 N to 330 N when measured in accordance with US Pharmacopeia monograph <1217> or with European Pharmacopoeia monograph 2.9.8.

Optionally, the tablet comprising the table core described above has a friability of less than about 1%, preferably less than about 0.5%, more preferably of less than 0.1% as determined in accordance with US Pharmacopoeia monograph <1216> or with European Pharmacopoeia monograph 2.9.7. For example, the tablet core may have a friability of 0.001 to 1%, or from 0.01 to 0.5%, such as from 0.01 to 0.1% when measured in accordance with US Pharmacopeia monograph <1216> or with European Pharmacopoeia monograph 2.9.7.

Optionally, the tablet core comprising colesevelam or a pharmaceutically acceptable salt thereof, for example, colesevelam hydrochloride, has a length of less than about 25 ± 0.5 mm, preferably less than about 24 ± 0.5 mm, more preferably of less than 23 mm ± 0.5 mm. More preferably, the tablet has a length of 22.5 ± 0.5 mm. Optionally, the tablet core has a length of less than 22 mm ± 0.5 mm, or less than 21 mm ± 0.5 mm, or less than 20 mm ± 0.5 mm.

Optionally, the tablet core comprising colesevelam or a pharmaceutically acceptable salt thereof, for example, colesevelam hydrochloride, has a thickness of less than about 11.5 mm ± 0.5 mm, preferably of less than about 10.5 mm ± 0.5 mm, more preferably of less than about 9.5 mm ± 0.5 mm, more preferably of less than or equal to about 8.5 mm ± 0.5 mm. Optionally, the tablet core has a thickness of less than about 8.0 mm ± 0.5 mm, or less than about 7.5 mm ± 0.5 mm, or less than about 7.0 mm ± 0.5 mm.

Optionally, the tablet core comprising colesevelam or a pharmaceutically acceptable salt thereof, for example, colesevelam hydrochloride, has a length of about 22 mm ± 0.5 mm and a thickness of about 10.5 mm ± 0.5 mm.

Optionally, the unit dose pharmaceutical composition further comprises a sub-coating between the tablet core and the gastro-resistant coating, optionally, wherein the sub-coating comprises hydroxypropyl cellulose.

Optionally, the tablet core comprising colesevelam or a pharmaceutically acceptable salt thereof, for example, colesevelam hydrochloride, is formed by direct compression.

Optionally, the gastro-resistant coating is formulated for delayed release, optionally, wherein the gastro-resistant coating is formulated for release in the ileum.

Optionally, the gastro-resistant coating is formulated for delayed/extended release, optionally, wherein the gastro-resistant coating is formulated for delayed release in the ileum and extended release continuing into the colon.

According to a second aspect of the present invention, there is provided a method of manufacturing a unit dose pharmaceutical composition for oral administration comprising the steps of:
providing a tablet core comprising colesevelam or a pharmaceutically acceptable salt thereof, such as colesevelam hydrochloride,
wherein the colesevelam or a pharmaceutically acceptable salt thereof (for example colesevelam hydrochloride) is at least 66 wt% of the tablet core,
wherein the colesevelam or a pharmaceutically acceptable salt thereof (for example colesevelam hydrochloride) is present in the **tablet core** in an amount of from about 700 mg to about 1400 mg; and
coating the tablet core with a gastro-resistant coating, and optionally a sub-coating.

There is also provided a method of manufacturing a unit dose pharmaceutical composition for oral administration comprising coating a tablet core with a gastro-resistant coating, and optionally a sub-coating, wherein the colesevelam or a pharmaceutically acceptable salt thereof is at least 66 wt% of the tablet core, wherein the colesevelam or a pharmaceutically acceptable salt thereof is present in the tablet core in an amount of from about 700 mg to about 1400 mg.

Optionally, the colesevelam or a pharmaceutically acceptable salt thereof, for example colesevelam hydrochloride, is present in the tablet core in an amount of from 850 mg to 1200 mg

Optionally, the method of manufacture comprises the steps of:
(i) combining colesevelam and/or pharmaceutically acceptable salts thereof, with a diluent, a binder, a glidant, a lubricant and optionally a disintegrant to form a mixture;
(ii) tableting the mixture of step (i) to form the tablet cores; and
(iii) coating the tablet cores formed in step (ii) with a gastro-resistant coating, and optionally a sub-coating.

Optionally, the method of manufacture comprises bulk manufacture of a plurality of unit dose pharmaceutical compositions for oral administration, wherein the plurality of unit dose pharmaceutical compositions has a uniformity of mass of less than ±3%, preferably less than ±2%, more preferably less than ±1.7%.

According to a third aspect of the present invention, there is provided a unit dose pharmaceutical composition for oral administration prepared according to the method according to the second aspect of the present invention.

Optionally, the active substance is released from the tablet core in a region selected from the group consisting of the lower gastrointestinal tract, the large intestine, the jejunum, the ileum, the cecum, the colon, the rectum, and combinations thereof.

Optionally, the active substance is released from the tablet core at pH of at least 5, such as 5.1, or 5.2, or 5.3, or 5.4, or 5.5, or 5.6, or 5.7, or 5.8, or 5.9, or 6, or 6.1, or 6.2, or 6.3, or 6.4, or 6.5, or 6.6, or 6.7, or 6.8, or 6.9, or 7, or 7.1, or 7.2, or 7.3, or 7.4, or 7.5. Preferably, the active substance is released from the tablet core at pH in the range of about 6.5 to about 7.2, more preferably in the range between 6.8 to about 7. According to a preferred aspect, the active substance is released from the tablet core at pH of 6.8. According to another preferred aspect, the active substance is released from the tablet core at pH of 7. According to a further preferred aspect, the active substance is released from the tablet core at pH of 7.2.

Optionally, the pharmaceutical composition may comprise colesevelam hydrochloride, microcrystalline cellulose, copovidone, crospovidone, colloidal silicone dioxide, and magnesium stearate.

Optionally, the pharmaceutical composition may comprise microcrystalline cellulose, wherein the microcrystalline cellulose is present in an amount ranging from 8 % to 15 % by weight with respect to the weight of the tablet core, more preferably from 9 % to 13 % by weight with respect to the weight of tablet core.

Optionally, the pharmaceutical composition may comprise copovidone, wherein the copovidone is present in an amount ranging from 1 % to 5 % by weight with respect to the weight of the tablet core, more preferably from 3 % to 4 % by weight with respect to the weight of tablet core.

Optionally, the pharmaceutical composition may comprise crospovidone, wherein the crospovidone is present in an amount ranging from 0.01 % to 2 % by weight with respect to the weight of the tablet core, more preferably from 0.1 % to 1 % by weight with respect to the weight of the tablet core.

Optionally, the pharmaceutical composition may comprise colloidal silicon dioxide, wherein the colloidal silicon dioxide is present in an amount ranging from 0.1 % to 2 % by weight with respect to the weight of the tablet core, more preferably from 0.5 % to 1 % by weight with respect to the weight of the tablet core.

Optionally, the pharmaceutical composition may comprise magnesium stearate, wherein the magnesium stearate is present in an amount ranging from 0.1 % to 2 % by weight with respect to the weight of the tablet core, more preferably from 0.5 % to 1 % by weight with respect to the weight of the tablet core.

Optionally the pharmaceutical composition is formulated for release in the ileum.

Optionally, pharmaceutical composition is formulated for release starting and reaching 100% release in the ileum prior to entering the ascending colon.

Optionally, the pharmaceutical composition is formulated for release starting in the ileum and continuing into the initial portion of the colon.

Optionally, the pharmaceutical composition is formulated for release starting in the ileum and throughout the ascending colon.

Optionally, the pharmaceutical composition is formulated for release starting in the ileum and throughout the ascending colon and transverse colon.

Optionally, the pharmaceutical composition is formulated for release starting in the ileum and throughout the ascending colon, transverse colon and descending colon.

Optionally, the pharmaceutical composition is formulated for release starting in the ileum and throughout the ascending colon, transverse colon, descending colon and sigmoid colon.

Optionally, the pharmaceutical composition is formulated for release starting in the ileum and throughout the ascending colon, transverse colon, optionally the descending colon, optionally the sigmoid colon, and optionally the rectum.

Optionally, the pharmaceutical composition comprises colesevelam or a salt thereof present in an amount of at least 68% w/w of the tablet core.

Optionally, the pharmaceutical composition comprises colesevelam or a salt thereof, for example colesevelam hydrochloride, present in an amount of at least 70%, at least 75%, at least 76%, at least 77%, at least 78%, at least 79%, at least 80%, at least 81%, at least 82%, at least 83%, at least 84%, at least 85%, at least 90%, or at least 95% w/w of the tablet core. In particular aspects, the pharmaceutical composition comprises colesevelam or a salt thereof, for example colesevelam hydrochloride, in an amount ranging from at least about 70% w/w to about 95% w/w of the tablet core.

Optionally, the pharmaceutical composition comprises a gastro-resistant coating.

Optionally, the gastro-resistant coating comprises methacrylic acid/methyl methacrylate copolymers (EUDRAGIT^{®} (S) and/or EUDRAGIT^{®} (L)); said gastro-resistant coating may optionally further comprise cellulose derivatives, such as cellulose aceto-phthalate, and ethyl cellulose.

Optionally, the pharmaceutical composition is in the form of a tablet having friability equal to or less than 1.5%.

Optionally, the pharmaceutical composition is in the form of a tablet, wherein the tablet core has a hardness of more than about 80 N.

Optionally, the tablet core has a length of less than about 25 ± 0.5 mm, preferably less than about 24 ± 0.5 mm, more preferably of less than 23 mm ± 0.5 mm.

Optionally, the tablet core has a thickness of less than about 11.5 mm ± 0.5 mm, preferably of less than about 10.5 mm ± 0.5 mm, more preferably of less than about 9.5 mm ± 0.5 mm, more preferably of less than or equal to about 8.5 mm ± 0.5 mm.

Optionally, the tablet core comprises about 900 mg colesevelam or a pharmaceutically acceptable salt thereof, for example about 900 mg colesevelam hydrochloride.

Optionally, the tablet core comprises about 1200 mg colesevelam or a pharmaceutically acceptable salt thereof, for example about 1200 mg colesevelam hydrochloride.

Suitably, the oral pharmaceutical compositions disclosed herein releases not more than about 50% of the amount of colesevelam, or a pharmaceutically acceptable salt thereof, when the pharmaceutical composition is placed in a United States Pharmacopeia (USP) dissolution apparatus ∥ complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 2 hours, wherein the dissolution apparatus comprises an aqueous phosphate buffer (optionally 1000 mL of said buffer) at pH 6.8 and at a temperature of 37 ± 0.5 °C, and wherein the aqueous solution is stirred at a rate of 100 rpm.

Suitably, any of the oral pharmaceutical compositions disclosed herein, may release not more than about 45% of the amount of colesevelam, or a pharmaceutically acceptable salt thereof, when the pharmaceutical composition is placed in a USP dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 2 hours, wherein the dissolution apparatus comprises an aqueous phosphate buffer (optionally 1000 mL of said buffer) at pH 6.8 and at a temperature of 37 ± 0.5 °C, and wherein the aqueous solution is stirred at a rate of 100 rpm.

Any of the oral pharmaceutical compositions disclosed herein may release not more than about 40% of the amount of colesevelam, or a pharmaceutically acceptable salt thereof, when the pharmaceutical composition is placed in a USP dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 2 hours, wherein the dissolution apparatus comprises an aqueous phosphate buffer (optionally 1000 mL of said buffer) at pH 6.8 and at a temperature of 37 ± 0.5 °C, and wherein the aqueous solution is stirred at a rate of 100 rpm.

Suitably, the oral pharmaceutical compositions disclosed herein releases not more than about 35% of the amount of colesevelam, or a pharmaceutically acceptable salt thereof, when the pharmaceutical composition is placed in a United States Pharmacopeia (USP) dissolution apparatus ∥ complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 2 hours, wherein the dissolution apparatus comprises an aqueous phosphate buffer (optionally 1000 mL of said buffer) at pH 6.8 and at a temperature of 37 ± 0.5 °C, and wherein the aqueous solution is stirred at a rate of 100 rpm.

Suitably, the oral pharmaceutical compositions disclosed herein releases not more than about 30% of the amount of colesevelam, or a pharmaceutically acceptable salt thereof, when the pharmaceutical composition is placed in a United States Pharmacopeia (USP) dissolution apparatus ∥ complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 2 hours, wherein the dissolution apparatus comprises an aqueous phosphate buffer (optionally 1000 mL of said buffer) at pH 6.8 and at a temperature of 37 ± 0.5 °C, and wherein the aqueous solution is stirred at a rate of 100 rpm.

Suitably, any of the oral pharmaceutical compositions disclosed herein, may release not more than about 25% of the amount of colesevelam, or a pharmaceutically acceptable salt thereof, when the pharmaceutical composition is placed in a USP dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 2 hours, wherein the dissolution apparatus comprises an aqueous phosphate buffer (optionally 1000 mL of said buffer) at pH 6.8 and at a temperature of 37 ± 0.5 °C, and wherein the aqueous solution is stirred at a rate of 100 rpm.

Any of the oral pharmaceutical compositions disclosed herein may release not more than about 20% of the amount of colesevelam, or a pharmaceutically acceptable salt thereof, when the pharmaceutical composition is placed in a USP dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 2 hours, wherein the dissolution apparatus comprises an aqueous phosphate buffer (optionally 1000 mL of said buffer) at pH 6.8 and at a temperature of 37 ± 0.5 °C, and wherein the aqueous solution is stirred at a rate of 100 rpm.

Suitably, the oral pharmaceutical compositions disclosed herein releases not more than about 50% of the amount of colesevelam, or a pharmaceutically acceptable salt thereof, when the pharmaceutical composition is placed in a United States Pharmacopeia (USP) dissolution apparatus ∥ complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 3 hours, wherein the dissolution apparatus comprises an aqueous phosphate buffer (optionally 1000 mL of said buffer) at pH 6.8 and at a temperature of 37 ± 0.5 °C, and wherein the aqueous solution is stirred at a rate of 100 rpm.

Suitably, any of the oral pharmaceutical compositions disclosed herein, may release not more than about 45% of the amount of colesevelam, or a pharmaceutically acceptable salt thereof, when the pharmaceutical composition is placed in a USP dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 3 hours, wherein the dissolution apparatus comprises an aqueous phosphate buffer (optionally 1000 mL of said buffer) at pH 6.8 and at a temperature of 37 ± 0.5 °C, and wherein the aqueous solution is stirred at a rate of 100 rpm.

Any of the oral pharmaceutical compositions disclosed herein may release not more than about 40% of the amount of colesevelam, or a pharmaceutically acceptable salt thereof, when the pharmaceutical composition is placed in a USP dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 3 hours, wherein the dissolution apparatus comprises an aqueous phosphate buffer (optionally 1000 mL of said buffer) at pH 6.8 and at a temperature of 37 ± 0.5 °C, and wherein the aqueous solution is stirred at a rate of 100 rpm.

Suitably, the oral pharmaceutical compositions disclosed herein releases not more than about 35% of the amount of colesevelam, or a pharmaceutically acceptable salt thereof, when the pharmaceutical composition is placed in a United States Pharmacopeia (USP) dissolution apparatus ∥ complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 3 hours, wherein the dissolution apparatus comprises an aqueous phosphate buffer (optionally 1000 mL of said buffer) at pH 6.8 and at a temperature of 37 ± 0.5 °C, and wherein the aqueous solution is stirred at a rate of 100 rpm.

Suitably, the oral pharmaceutical compositions disclosed herein releases not more than about 30% of the amount of colesevelam, or a pharmaceutically acceptable salt thereof, when the pharmaceutical composition is placed in a United States Pharmacopeia (USP) dissolution apparatus ∥ complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 3 hours, wherein the dissolution apparatus comprises an aqueous phosphate buffer (optionally 1000 mL of said buffer) at pH 6.8 and at a temperature of 37 ± 0.5 °C, and wherein the aqueous solution is stirred at a rate of 100 rpm.

Suitably, any of the oral pharmaceutical compositions disclosed herein, may release not more than about 25% of the amount of colesevelam, or a pharmaceutically acceptable salt thereof, when the pharmaceutical composition is placed in a USP dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 3 hours, wherein the dissolution apparatus comprises an aqueous phosphate buffer (optionally 1000 mL of said buffer) at pH 6.8 and at a temperature of 37 ± 0.5 °C, and wherein the aqueous solution is stirred at a rate of 100 rpm.

Any of the oral pharmaceutical compositions disclosed herein may release not more than about 20% of the amount of colesevelam, or a pharmaceutically acceptable salt thereof, when the pharmaceutical composition is placed in a USP dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 3 hours, wherein the dissolution apparatus comprises an aqueous phosphate buffer (optionally 1000 mL of said buffer) at pH 6.8 and at a temperature of 37 ± 0.5 °C, and wherein the aqueous solution is stirred at a rate of 100 rpm.

Suitably, the oral pharmaceutical compositions disclosed herein releases not more than about 50% of the amount of colesevelam, or a pharmaceutically acceptable salt thereof, when the pharmaceutical composition is placed in a United States Pharmacopeia (USP) dissolution apparatus ∥ complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 4 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Suitably, the oral pharmaceutical compositions disclosed herein releases not more than about 45% of the amount of colesevelam, or a pharmaceutically acceptable salt thereof, when the pharmaceutical composition is placed in a United States Pharmacopeia (USP) dissolution apparatus ∥ complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 4 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Suitably, the oral pharmaceutical compositions disclosed herein releases not more than about 40% of the amount of colesevelam, or a pharmaceutically acceptable salt thereof, when the pharmaceutical composition is placed in a United States Pharmacopeia (USP) dissolution apparatus ∥ complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 4 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Suitably, the oral pharmaceutical compositions disclosed herein releases not more than about 35% of the amount of colesevelam, or a pharmaceutically acceptable salt thereof, when the pharmaceutical composition is placed in a United States Pharmacopeia (USP) dissolution apparatus ∥ complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 4 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Suitably, the oral pharmaceutical compositions disclosed herein releases not more than about 30% of the amount of colesevelam, or a pharmaceutically acceptable salt thereof, when the pharmaceutical composition is placed in a United States Pharmacopeia (USP) dissolution apparatus ∥ complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 4 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Suitably, any of the oral pharmaceutical compositions disclosed herein, may release not more than about 25% of the amount of colesevelam, or a pharmaceutically acceptable salt thereof, when the pharmaceutical composition is placed in a USP dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 4 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Suitably, any of the oral pharmaceutical compositions disclosed herein, may release not more than about 20% of the amount of colesevelam, or a pharmaceutically acceptable salt thereof, when the pharmaceutical composition is placed in a USP dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 4 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Optionally, the pharmaceutical composition releases not more than about 50% of the amount of colesevelam, or a pharmaceutically acceptable salt thereof, when the pharmaceutical composition is placed in a USP dissolution apparatus II complying with USP <711> for 1 hour, wherein the dissolution apparatus comprises an aqueous phosphate buffer (optionally 1000 mL of said buffer) at pH 6.8 and at a temperature of 37 ± 0.5 °C, and wherein the aqueous solution is stirred at a rate of 100 rpm.

Optionally, the pharmaceutical composition releases not more than about 45% of the amount of colesevelam, or a pharmaceutically acceptable salt thereof, when the pharmaceutical composition is placed in a USP dissolution apparatus II complying with USP <711> for 1 hour, wherein the dissolution apparatus comprises an aqueous phosphate buffer (optionally 1000 mL of said buffer) at pH 6.8 and at a temperature of 37 ± 0.5 °C, and wherein the aqueous solution is stirred at a rate of 100 rpm.

Optionally, the pharmaceutical composition releases not more than about 40% of the amount of colesevelam, or a pharmaceutically acceptable salt thereof, when the pharmaceutical composition is placed in a USP dissolution apparatus II complying with USP <711> for 1 hour, wherein the dissolution apparatus comprises an aqueous phosphate buffer (optionally 1000 mL of said buffer) at pH 6.8 and at a temperature of 37 ± 0.5 °C, and wherein the aqueous solution is stirred at a rate of 100 rpm.

Optionally, the pharmaceutical composition releases not more than about 35% of the amount of colesevelam, or a pharmaceutically acceptable salt thereof, when the pharmaceutical composition is placed in a USP dissolution apparatus II complying with USP <711> for 1 hour, wherein the dissolution apparatus comprises an aqueous phosphate buffer (optionally 1000 mL of said buffer) at pH 6.8 and at a temperature of 37 ± 0.5 °C, and wherein the aqueous solution is stirred at a rate of 100 rpm.

Optionally, the pharmaceutical composition releases not more than about 30% of the amount of colesevelam, or a pharmaceutically acceptable salt thereof, when the pharmaceutical composition is placed in a USP dissolution apparatus II complying with USP <711> for 1 hour, wherein the dissolution apparatus comprises an aqueous phosphate buffer (optionally 1000 mL of said buffer) at pH 6.8 and at a temperature of 37 ± 0.5 °C, and wherein the aqueous solution is stirred at a rate of 100 rpm.

Optionally, the pharmaceutical composition releases not more than about 25% of the amount of colesevelam, or a pharmaceutically acceptable salt thereof, when the pharmaceutical composition is placed in a USP dissolution apparatus II complying with USP <711> for 1 hour, wherein the dissolution apparatus comprises an aqueous phosphate buffer (optionally 1000 mL of said buffer) at pH 6.8 and at a temperature of 37 ± 0.5 °C, and wherein the aqueous solution is stirred at a rate of 100 rpm.

Optionally, the pharmaceutical composition releases not more than about 20% of the amount of colesevelam, or a pharmaceutically acceptable salt thereof, when the pharmaceutical composition is placed in a USP dissolution apparatus II complying with USP <711> for 1 hour, wherein the dissolution apparatus comprises an aqueous phosphate buffer (optionally 1000 mL of said buffer) at pH 6.8 and at a temperature of 37 ± 0.5 °C, and wherein the aqueous solution is stirred at a rate of 100 rpm.

Optionally, the pharmaceutical composition releases not less than about 80% of the amount of colesevelam, or a pharmaceutically acceptable salt thereof, when the pharmaceutical composition is placed in a USP dissolution apparatus II complying with USP <711> for 2 hours, wherein the dissolution apparatus comprises an aqueous phosphate buffer (optionally 1000 mL of said buffer) at pH 6.8 and at a temperature of 37 ± 0.5 °C, and wherein the aqueous solution is stirred at a rate of 100 rpm.

Optionally, the pharmaceutical composition releases not less than about 80% of the amount of colesevelam, or a pharmaceutically acceptable salt thereof, when the pharmaceutical composition is placed in a USP dissolution apparatus II complying with USP <711> for 3 hours, wherein the dissolution apparatus comprises an aqueous phosphate buffer (optionally 1000 mL of said buffer) at pH 6.8 and at a temperature of 37 ± 0.5 °C, and wherein the aqueous solution is stirred at a rate of 100 rpm.

Optionally, the pharmaceutical composition releases not less than about 80% of the amount of colesevelam, or a pharmaceutically acceptable salt thereof, when the pharmaceutical composition is placed in a USP dissolution apparatus II complying with USP <711> for 4 hours, wherein the dissolution apparatus comprises an aqueous phosphate buffer (optionally 1000 mL of said buffer) at pH 6.8 and at a temperature of 37 ± 0.5 °C, and wherein the aqueous solution is stirred at a rate of 100 rpm.

Optionally, the oral pharmaceutical compositions of the present invention have a dissolution rate lower than about 50% over about 1 hour, when measured at about pH 6.8.

Optionally, the oral pharmaceutical compositions of the present invention have a dissolution rate lower than about 45% over about 1 hour, when measured at about pH 6.8.

Optionally, the oral pharmaceutical compositions of the present invention have a dissolution rate lower than about 40% over about 1 hour, when measured at about pH 6.8.

Optionally, the oral pharmaceutical compositions of the present invention have a dissolution rate lower than about 35% over about 1 hour, when measured at about pH 6.8.

Optionally, the oral pharmaceutical compositions of the present invention have a dissolution rate lower than about 30% over about 1 hour, when measured at about pH 6.8.

Optionally, the oral pharmaceutical compositions of the present invention have a dissolution rate lower than about 25% over about 1 hour, when measured at about pH 6.8.

Optionally, the oral pharmaceutical compositions of the present invention have a dissolution rate lower than about 20% over about 1 hour, when measured at about pH 6.8.

Optionally, the oral pharmaceutical compositions of the present invention have a dissolution rate of greater than or equal to about 80% of colesevelam, or a pharmaceutically acceptable salt thereof, over about 2 hours, when measured at about pH 6.8.

Optionally, the oral pharmaceutical compositions of the present invention have a dissolution rate of greater than or equal to about 80% of colesevelam, or a pharmaceutically acceptable salt thereof, over about 3 hours, when measured at about pH 6.8.

Optionally, the oral pharmaceutical compositions of the present invention have a dissolution rate of greater than or equal to about 80% of colesevelam, or a pharmaceutically acceptable salt thereof, over about 4 hours, when measured at about pH 6.8.

According to the invention, the dissolutions are measured with the with the USP dissolution apparatus type II equipped with a paddle at 100 rpm at 37°±2° C.

According to a fourth aspect of the present invention, there is provided use of a pharmaceutical composition in the treatment of bile acid malabsorption.

According to a fifth aspect of the present invention, there is provided use of a pharmaceutical composition in the treatment of bile acid diarrhea.

Optionally, this bile acid diarrhea could be idiopathic (e.g., primary bile acid diarrhea) or secondary to an underlying disease. Optionally, such underlying disease can be: Crohn's disease, ileal resection or radiation ileitis, chronic pancreatitis, celiac disease, cholecystectomy, small intestine bacterial overgrowth, irritable bowel syndrome subtype diarrhea (IBS-D) and similar.

According to a sixth aspect of the present invention, there is provided use of a pharmaceutical composition in the treatment of irritable bowel syndrome subtype diarrhea (IBS-D).

According to a seventh aspect of the present invention, there is provided use of a pharmaceutical composition in the treatment of irritable microscopic colitis.

According to an eighth aspect of the present invention, there is provided a method of treating bile acid malabsorption.

Optionally, the method comprises administering a therapeutically effective amount of a pharmaceutical composition to a subject in need thereof.

According to a ninth aspect of the present invention, there is provided a method of treating bile acid diarrhea.

Optionally, this bile acid diarrhea is idiopathic (e.g., primary bile acid diarrhea) or secondary to an underlying disease. Optionally, such underlying disease can be: Crohn's disease, ileal resection or radiation ileitis, chronic pancreatitis, celiac disease, cholecystectomy, small intestine bacterial overgrowth, irritable bowel syndrome subtype diarrhea (IBS-D) and similar.

Optionally, the method comprises administering a therapeutically effective amount of a pharmaceutical composition to a subject in need thereof.

According to a tenth aspect of the present invention, there is provided a method of treating irritable bowel syndrome subtype diarrhea (IBS-D).

Optionally, the method comprises administering a therapeutically effective amount of a pharmaceutical composition to a subject in need thereof.

According to an eleventh aspect of the present invention, there is provided a method of treating irritable microscopic colitis.

Optionally, the method comprises administering a therapeutically effective amount of a pharmaceutical composition to a subject in need thereof.

According to a twelfth aspect of the present invention, there is provided the use of a unit dose pharmaceutical composition for oral administration as described herein in the manufacture of a medicament for treating bile acid diarrhea, irritable bowel syndrome, optionally, irritable bowel syndrome subtype diarrhea (IBS-D), colitis, such as microscopic colitis, and/or bile acid malabsorption.

The present disclosure further provides a unit dose pharmaceutical composition for oral administration as described herein for use in a method of treating bile acid diarrhea, irritable bowel syndrome, optionally, irritable bowel syndrome subtype diarrhea (IBS-D), colitis, such as microscopic colitis, and/or bile acid malabsorption in a patient in need thereof, optionally, wherein the method comprises administering at least one of said unit dose pharmaceutical compositions for oral administration to said patient. Suitably, the method comprises administering one of said unit dose pharmaceutical compositions for oral administration to said patient, optionally, the method comprises administering more than one of said unit dose pharmaceutical compositions for oral administration to said patient. For example, the method may comprise administering one or more of said unit dose pharmaceutical compositions for oral administration to said patient.

The present disclosure also provides a method of treating bile acid diarrhea, irritable bowel syndrome, optionally, irritable bowel syndrome subtype diarrhea (IBS-D), colitis, such as microscopic colitis, and/or bile acid malabsorption in a patient in need thereof, comprising administering to said patient a unit dose pharmaceutical composition for oral administration as described herein, optionally, the method comprises administering one of said unit dose pharmaceutical compositions for oral administration to said patient, optionally, the method comprises administering more than one of said unit dose pharmaceutical compositions for oral administration to said patient. For example, the method may comprise administering more than one of said unit dose oral pharmaceutical compositions to said patient.

Disclosed herein is a **unit dose** pharmaceutical composition for oral administration comprising **a tablet core** and a gastro-resistant coating covering the tablet core,
wherein the tablet core comprises colesevelam or a pharmaceutically acceptable salt thereof, wherein the colesevelam or a pharmaceutically acceptable salt thereof is at least 66 wt% of the tablet core, and
wherein the colesevelam or a pharmaceutically acceptable salt thereof is present in the **tablet core** in an amount of from about 700 mg to 1400 mg;
optionally wherein the colesevelam or a pharmaceutically acceptable salt thereof is at least 70 wt%, or at least 71 wt%, or at least 75 wt%, or at least 76 wt%, or at least 80 wt%, or at least 81 wt% of the tablet core, optionally, at least 82 wt% of the tablet core;
optionally wherein the tablet core comprises colesevelam hydrochloride.

The tablet core may comprise a diluent or mixture of two or more diluents selected from the group comprising: lactose, sugars such as sucrose and fructose, dextrose, dextrates, dextrans, polyols such as sorbitol, mannitol and xylitol, dibasic calcium phosphate, tribasic calcium phosphate, calcium sulphate, sodium chloride, starch, such as pregelatinized starch, cellulose or derivatives thereof, such as methyl, ethyl, or hydroxyethyl cellulose; optionally wherein the diluent is selected from cellulose or derivatives thereof, lactose, calcium phosphate, starch, modified starch and mannitol; optionally wherein the diluent is microcrystalline cellulose, optionally the diluent is a porous microcrystalline cellulose having an average particle size of in the range of from 80 to 110 µm and an angle of repose of less than about 40°, preferably, wherein the porous microcrystalline cellulose has an average particle size of about 90 µm and an angle of repose of about 34;
optionally wherein the diluent is present in an amount of from 6 to 21 wt% of the tablet core, optionally 8 to 18 wt% of the tablet core, optionally the diluent is present in an amount of from 9 to 13 wt% of the tablet core.

The tablet core may comprise a binder, wherein the binder is selected from the group comprising: polyvinyl alcohol, natural gums, gelatin, polymethacrylates, cellulose and derivatives thereof (e.g., ethyl cellulose) and copovidone, or any combination thereof; optionally wherein the binder is selected from copovidone and polyvinyl alcohol;
optionally wherein the binder is present in an amount of from 1 to 7 wt%, of the tablet core, optionally wherein the binder is present in an amount of from 2 to 6 wt%, of the tablet core, optionally wherein the binder is present in an amount of from 3 to 5 wt% of the tablet core.

The tablet core may comprise a disintegrant selected from the group comprising: sodium carboxymethyl cellulose, cross-linked sodium carboxymethyl cellulose, bentonite, alginic acid or a derivative or salt thereof, and crospovidone, or any combination thereof, optionally wherein the disintegrant is selected from crospovidone and cross-linked sodium carboxymethyl cellulose; optionally wherein the disintegrant is present in an amount of from 0.01 to 2 wt% of the tablet core; optionally wherein the disintegrant is present in an amount of from 0.1 to 1 wt% of the tablet core.

The tablet core may comprises a glidant selected from the group comprising: talc, starch, magnesium oxide, silica, silicon dioxide, colloidal silicon dioxide, a silicate or a salt thereof such as magnesium or calcium silicate, or any combination thereof; optionally wherein the glidant is selected from silica, colloidal silicon dioxide, talc and magnesium oxide;
optionally wherein the glidant is present in an amount of from 0.1 to 2 wt% of the tablet core, optionally wherein the glidant is present in an amount of from 0.5 to 1.5 wt% of the tablet core, further optionally, wherein the glidant is present in an amount of from 0.7 to 1.2 wt% of the tablet core.

The tablet core may comprise a lubricant selected from the group comprising: stearic acid or a salt thereof such as magnesium, calcium or zinc stearate, polyethylene glycol, fumaric acid or a salt thereof, glyceryl behenate, glyceryl palmitostearate, wax, and sodium benzoate, or any combination thereof; optionally wherein the lubricant is selected from stearic acid or a salt thereof, fumaric acid or a salt thereof, and polyethylene glycol;
optionally wherein the lubricant is present in an amount of from 0.1 to 2 wt% of the tablet core, optionally wherein the lubricant is present in an amount of from 0.5 to 1.5 wt% of the tablet core, further optionally, wherein the lubricant is present in an amount of from 0.7 to 1.2 wt% of the tablet core.

The tablet core may comprise microcrystalline cellulose, copovidone, colloidal silicon dioxide, magnesium stearate, and optionally crospovidone;
optionally wherein the tablet core comprises:
microcrystalline cellulose in an amount of from 9 to 13 wt% of the tablet core,
copovidone in an amount of from 3 to 4 wt% of the tablet core,
colloidal silicon dioxide in an amount of from 0.5 to 1.5 wt% of the tablet core,
magnesium stearate in an amount of from 0.5 to 1.5 wt% of the tablet core, and optionally, crospovidone in an amount of from 0 to 1 wt% of the tablet core.

The unit dose pharmaceutical composition may further comprise a sub-coating between the tablet core and the gastro-resistant coating, optionally, wherein the sub-coating comprises hydroxypropyl cellulose;
optionally wherein said gastro-resistant coating comprises methacrylic acid/methyl methacrylate (1:1) copolymer;
optionally wherein said gastro-resistant coating comprises methacrylic acid/methyl methacrylate (1:2) copolymer;
optionally wherein said gastro-resistant coating comprises a mixture of methacrylic acid/methyl methacrylate (1:1) copolymer and of methacrylic acid/methyl methacrylate (1:2) copolymer, optionally, the weight ratio of methacrylic acid/methyl methacrylate (1:1) copolymer to methacrylic acid/methyl methacrylate (1:2) copolymer is in the range of from 0.5:1 to 3:1, suitably, in a weight range of from 1:1 to 2:1;
optionally wherein the gastro-resistant coating of the unit dose starts breaking or dissolving at or above a pH of 5, suitably at or above a pH of 6, preferably at or above a pH of 6.5, more preferably at a pH of 6.8
optionally wherein the gastro-resistant coating is formulated for delayed release, optionally, wherein the gastro-resistant coating is formulated for release in the ileum.

Suitably, the tablet core has a hardness of greater than 70 N, suitably greater than 90 N, preferably greater than 120 N as determined in accordance with European Pharmacopoeia 2.9.8 or with US Pharmacopoeia monograph <1217>.

Suitably, the tablet core has a friability of less than 1%, preferably less than 0.5%, more preferably of less than 0.1% as determined in accordance with US Pharmacopoeia monograph <1216> or with European Pharmacopoeia monograph 2.9.7.

Optionally, the tablet core has a length of less than about 25±0.5 mm, preferably less than about 24±0.5 mm, more preferably of less than 23 mm ± 0.5; optionally wherein the tablet core has a thickness of less than about 11.5 mm ± 0.5 mm, preferably of less than about 10.5 mm ± 0.5 mm, more preferably of less than about 9.5 mm ± 0.5 mm, more preferably of less than or equal to about 8.5 mm ± 0.5 mm; optionally wherein the tablet core is formed by direct compression.

Optionally, the pharmaceutical composition releases not more than about 50% of the amount of colesevelam, or a pharmaceutically acceptable salt thereof, when the pharmaceutical composition is placed in a USP dissolution apparatus ∥ complying with USP <711> for 1 hour, optionally wherein the pharmaceutical composition releases not less than about 80% of the amount of colesevelam, or a pharmaceutically acceptable salt thereof, when the pharmaceutical composition is placed in a USP dissolution apparatus II complying with USP <711> for 2 hours, wherein the dissolution apparatus comprises an aqueous phosphate buffer (optionally 1000 mL of said buffer) at pH 6.8 and at a temperature of 37 ± 0.5 °C, and wherein the aqueous solution is stirred at a rate of 100 rpm.

Suitably, the colesevelam or a pharmaceutically acceptable salt thereof is present in an amount of from 76 wt% to 90 wt% of the tablet core, optionally, from 80 wt% to 90 wt%, optionally from 81 wt% to 90 wt%, such as from 82 wt% to 90 wt% of the tablet core and wherein the colesevelam or a pharmaceutically acceptable salt thereof is present in the tablet core in an amount of from about 700 mg to 1400 mg, wherein the tablet core has a hardness in the range of from 70 N to 450 N, optionally, from 80 N to 400 N, further optionally, from 90 N to 370 N, such as from 100 N to 330 N as determined in accordance with European Pharmacopoeia 2.9.8 or with US Pharmacopoeia monograph <1217>; and wherein the tablet core has a friability of from 0.001% to 1%, optionally, from 0.01 to 0.5%, optionally from 0.01 to 0.1% as determined in accordance with US Pharmacopoeia monograph <1216> or with European Pharmacopoeia monograph 2.9.7.

The colesevelam or a pharmaceutically acceptable salt thereof may be present in an amount of from 76 wt% to 90 wt% of the tablet core, optionally, from 80 wt% to 90 wt%, optionally from 81 wt% to 90 wt%, such as from 82 wt% to 90 wt% of the tablet core and wherein the colesevelam or a pharmaceutically acceptable salt thereof is present in the tablet core in an amount of from about 700 mg to 1400 mg;
wherein the tablet core comprises a diluent, wherein the diluent comprises a porous microcrystalline cellulose having an average particle size of in the range of from 80 to 110 µm and an angle of repose of less than about 40°, preferably, wherein the porous microcrystalline cellulose has an average particle size of about 90 µm and an angle of repose of about 34°, wherein the diluent is present in an amount of from 8 to 18 wt% of the tablet core;
wherein the tablet core has a hardness in the range of from 70 N to 450 N, optionally, from 80 N to 400 N, further optionally, from 90 N to 370 N, such as from 100 N to 330 N as determined in accordance with European Pharmacopoeia 2.9.8 or with US Pharmacopoeia monograph <1217>; and wherein the tablet core has a friability of from 0.001% to 1%, optionally, from 0.01 to 0.5%, optionally from 0.01 to 0.1% as determined in accordance with US Pharmacopoeia monograph <1216> or with European Pharmacopoeia monograph 2.9.7.

Also disclosed is a method of manufacturing a unit dose pharmaceutical composition for oral administration comprising the steps of:
providing a tablet core comprising colesevelam or a pharmaceutically acceptable salt thereof,
wherein the colesevelam or a pharmaceutically acceptable salt thereof is at least 66 wt% of the tablet core, wherein the colesevelam or a pharmaceutically acceptable salt thereof is present in the **tablet core** in an amount of from 850 mg to 1200 mg; and
coating the tablet core with a gastro-resistant coating, and optionally a subcoating;
optionally wherein the method further comprises the steps of:
   (i) combining colesevelam and/or pharmaceutically acceptable salts thereof, with a diluent, a binder, a glidant, a lubricant and optionally a disintegrant to form a mixture;
   (ii) tableting the mixture of step (i) to form tablet cores; and
   (iii) coating the tablet cores formed in step (ii) with a gastro-resistant coating, and optionally a subcoating.

The method may comprise bulk manufacture of a plurality of unit dose pharmaceutical compositions for oral administration, wherein the plurality of unit doses has a uniformity of mass of less than ±3%, preferably less than ±2%, more preferably less than ±1.7%.

Also disclosed is a unit dose pharmaceutical composition for oral administration as described herein for use in a method of treating bile acid diarrhea, irritable bowel syndrome, optionally, irritable bowel syndrome subtype diarrhea (IBS-D), colitis, such as microscopic colitis, and/or bile acid malabsorption in a patient in need thereof, optionally, wherein the method comprises administering one of said unit dose pharmaceutical compositions for oral administration to said patient, optionally, wherein the method comprises administering more than one of said unit dose pharmaceutical compositions for oral administration to said patient.

### Brief Description of the Drawings

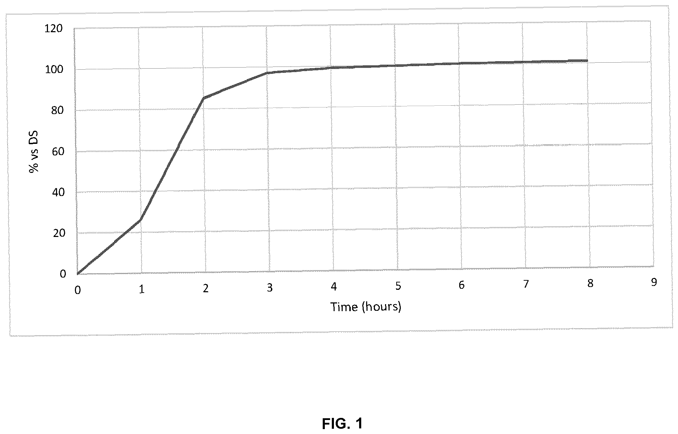

FIG. 1 shows the binding capacity of colesevelam HCl for taurodeoxycholate (DS) over time. The Y axis label "% vs DS", refers to the binding capacity of colesevelam hydrochloride versus the maximum binding capacity of the drug substance (colesevelam raw material). A unit dose pharmaceutical composition tablet according to the invention as described in Example 1 (Formulation C2) was added to 0.1 N HCl for 2 hours, and then the tablets (which remained intact during the course of the acid treatment and were drained of any residual acid) were added to phosphate buffer medium at pH 6.8 comprising a known amount of taurodeoxycholate. As the gastric resistant coating of the tablet dissolves (at pH 6.8) colesevelam HCl is released and swells and binds the taurodeoxycholate in the medium. The amount of unbound taurodeoxycholate is monitored over time, and the binding capacity of colesevelam is calculated by the consumption of the free non-bound taurodeoxycholate.

### Detailed Description

The present invention provides a unit dose pharmaceutical composition for oral administration comprising a tablet core and a gastro-resistant coating covering the tablet core,
wherein the tablet core comprises colesevelam or a pharmaceutically acceptable salt thereof, for example colesevelam hydrochloride,
wherein the colesevelam or a pharmaceutically acceptable salt thereof, e.g., colesevelam hydrochloride, is at least 66 wt% of the tablet core,
wherein the colesevelam or a pharmaceutically acceptable salt thereof, e.g., colesevelam hydrochloride, is present in the tablet core in an amount of from about 700 mg to about 1400 mg.

Obtaining tablets with a very high content of active substance is difficult, in particular due to the physical properties of colesevelam. Colesevelam is a cross linked polymer which is insoluble in aqueous and organic solvents and has poor flowability and compressibility properties.

Providing tablets with certain advanced characteristics, such as a targeted and/or delayed release profile, presents significant a challenge when employing a very low content of excipients in the tablet, which give rise to the desired release properties.

In order to overcome the tableting issues, and in the hope of achieving a higher drug loading of colesevelam, and in particular colesevelam hydrochloride, the inventors investigated a plethora of different excipient formulations including different amounts and combinations of various excipients, different drug loadings, as well as different manufacturing processes.

The inventors successfully identified a combination of excipients that enables the formation of high loading colesevelam tablets, comprising over 700 mg colesevelam hydrochloride, preferably over 850 mg colesevelam hydrochloride, at loadings as high as 76%, 82% and 85%.

The inventors were surprisingly able to produce tablets with the desired characteristics, such as excellent hardness and low friability, that have a high drug loading of colesevelam.

Optionally, the colesevelam or a pharmaceutically acceptable salt thereof, e.g., colesevelam hydrochloride, is present in the tablet core in an amount of from about 850 mg to about 1200 mg

Optionally, the unit dose pharmaceutical composition further comprises a sub-coating between the tablet core and the gastro-resistant coating.

Optionally, the sub-coating comprises hydroxypropyl cellulose.

As outlined above, due to the large doses of colesevelam required for treating bile acid malabsorption (about 3.75g per day), and the large number of tablets required to achieve such a dose, patient compliance issues often occur.

There is therefore a need for a pharmaceutical composition comprising a large amount of colesevelam per dosage unit, to improve patient compliance by reducing the number of tablets required to be administered.

Despite the need for such dosage forms, colesevelam, and in particular colesevelam hydrochloride, is a powder characterized by very poor flow properties and compressibility properties. As a result of these poor formulation characteristics, and in order to obtain tablets containing a sufficient amount of colesevelam, to date, colesevelam formulations have included a substantial quantity of excipients in the formulation, such as binders and/or fillers. As a result, attempts in the art to increase the amount of colesevelam in particular dosage forms, has also resulted in increased amounts of binders and/or fillers.

But because a suitable tablet size for oral administration is a requirement, the poor formulation properties of colesevelam (or salts thereof) cannot simply be circumvented by making larger tablets with higher doses of colesevelam and larger amounts of excipients such as binders and/or fillers. Such large tablets cannot be easily swallowed and are not suitable for administration. The requirement that tablet size remains suitable for oral administration consequently has limited the unitary content of the active substance per tablet (i.e., the drug loading of colesevelam) due to the large quantities of excipients required for tablet formulation. Thus, due to the poor processing properties of colesevelam (or pharmaceutically acceptable salts thereof), and the requirement for relatively large amounts of tablet excipients, and the need to employ tablets with dimensions within an acceptable range for oral administration to the patient, the amount of colesevelam dose per tablet has been limited to date to an upper limit of approximately 650 mg colesevelam per tablet. This gives rise to the need to administer several tablets per day, in order to achieve the desired pharmacological effect in treating bile acid diarrhea.

The present invention solves these problems by providing a high unit dose pharmaceutical composition for oral administration comprising a high dose of colesevelam, and in particular colesevelam hydrochloride, with a high weight percentage per unit dose.

The unit dose oral pharmaceutical composition (or one or more unit doses) of the present invention may be used to treat diseases or conditions such as bile acid diarrhea, irritable bowel syndrome, optionally, irritable bowel syndrome subtype diarrhea (IBS-D), colitis, such as microscopic colitis, and/or bile acid malabsorption in a patient in need thereof. The treatment may comprise administering more than one of said unit dose oral pharmaceutical compositions to said patient.

Given the chronicity of the aforementioned diseases/conditions, treatment with prior art or commercially available colesevelam tablets which comprise lower amounts and loadings of colesevelam requires the administration of a large number of tablets per day. This often leads to patient compliance issues. Advantageously, the unit dose oral pharmaceutical composition of the present invention allows for a reduction in the number of tablets and a reduction in the frequency of administration per day (e.g., two tablets twice per day, or one tablet twice per day), which leads to an improvement in patient compliance.

Provided herein is a **unit dose** pharmaceutical composition for oral administration comprising a **tablet core** and a gastro-resistant coating covering the tablet core,
wherein the tablet core comprises colesevelam or a pharmaceutically acceptable salt thereof, such as colesevelam hydrochloride,
wherein the colesevelam or a pharmaceutically acceptable salt thereof (e.g., colesevelam hydrochloride) is at least 66 wt% of the tablet core, and wherein the colesevelam or a pharmaceutically acceptable salt thereof (e.g., colesevelam hydrochloride) is present in the **tablet core** in an amount of from about 700 mg to about 1400 mg, such as from about 850 mg to about 1200 mg.

The pharmaceutical compositions of the invention can contain colesevelam or a pharmaceutically acceptable salt thereof is present in the tablet core in an amount of from about 850 mg to about 1200 mg. In particular embodiments, the pharmaceutical compositions of the invention can contain colesevelam hydrochloride in the tablet core in an amount of from about 850 mg to about 1200 mg

The pharmaceutical compositions of the invention can contain more than about 66% w/w, optionally more than about 70% w/w of colesevelam or a pharmaceutically acceptable salt thereof in the tablet core, such as, for example, more than about 71% w/w, more than about 72% w/w, more than about 73% w/w, more than about 74% w/w, more than about 75% w/w, more than about 76% w/w, more than about 77% w/w, more than about 78% w/w, more than about 79% w/w of colesevelam or a pharmaceutically acceptable salt thereof in the tablet core; preferably the pharmaceutical compositions of the invention can optionally contain more than about 80% w/w of colesevelam or a pharmaceutically acceptable salt thereof in the tablet core, such as more than about 81% w/w, more than about 82% w/w, more than about 83% w/w, more than about 84% w/w, more than about 85% w/w, more than about 86% w/w, more than about 87% w/w, more than about 88% w/w, more than about 89% w/w, more than about 90% w/w of colesevelam in the tablet core. Preferably, the pharmaceutical compositions of the invention can contain more than about 81% w/w of colesevelam or a pharmaceutically acceptable salt thereof in the tablet core. For avoidance of any doubt, the term core shall be interpreted as the inner part of the tablet, which may optionally be surrounded by a film coating.

Additionally, the pharmaceutical compositions of the invention can contain more than about 66% w/w, optionally more than about 70% w/w of colesevelam hydrochloride in the tablet core, such as, for example, more than about 71% w/w, more than about 72% w/w, more than about 73% w/w, more than about 74% w/w, more than about 75% w/w, more than about 76% w/w, more than about 77% w/w, more than about 78% w/w, more than about 79% w/w of colesevelam hydrochloride in the tablet core; preferably the pharmaceutical compositions of the invention can optionally contain more than about 80% w/w of colesevelam hydrochloride in the tablet core, such as more than about 81% w/w, more than about 82% w/w, more than about 83% w/w, more than about 84% w/w, more than about 85% w/w, more than about 86% w/w, more than about 87% w/w, more than about 88% w/w, more than about 89% w/w, more than about 90% w/w of colesevelam hydrochloride in the tablet core. Preferably, the pharmaceutical compositions of the invention can contain more than about 81% w/w of colesevelam hydrochloride in the tablet core. For avoidance of any doubt, the term core shall be interpreted as the inner part of the tablet, which may optionally be surrounded by a film coating.

For example, colesevelam and/or pharmaceutically acceptable salts thereof is present in the unit dose pharmaceutical composition for oral administration in an amount of 70 wt% to 90 wt% of the tablet core, or in an amount of 75 wt% to 90 wt% of the tablet core, or in an amount of 76 wt% to 90 wt% of the tablet core, suitably in an amount of 80 wt% to 90 wt% of the tablet core, more suitably 81 wt% to 90 wt% of the tablet core, optionally 82 wt% to 90 wt% of the tablet core. Optionally, colesevelam and/or pharmaceutically acceptable salts thereof is present in the unit dose pharmaceutical composition for oral administration in an amount of 70 wt% to 88 wt% of the tablet core, or in an amount of 75 wt% to 88 wt% of the tablet core, or in an amount of 76 wt% to 88 wt% of the tablet core, suitably in an amount of 80 wt% to 88 wt% of the tablet core, more suitably 81 wt% to 88 wt% of the tablet core, optionally 82 wt% to 88 wt% of the tablet core. Further optionally, colesevelam and/or pharmaceutically acceptable salts thereof is present in the unit dose pharmaceutical composition for oral administration in an amount of 70 wt% to 85 wt% of the tablet core, or in an amount of 75 wt% to 85 wt% of the tablet core, or in an amount of 76 wt% to 85 wt% of the tablet core, suitably in an amount of 80 wt% to 85 wt% of the tablet core, more suitably 81 wt% to 85 wt% of the tablet core, optionally 82 wt% to 85 wt% of the tablet core.

By way of further example, colesevelam hydrochloride is present in the unit dose pharmaceutical composition for oral administration in an amount of 70 wt% to 90 wt% of the tablet core, or in an amount of 75 wt% to 90 wt% of the tablet core, or in an amount of 76 wt% to 90 wt% of the tablet core, suitably in an amount of 80 wt% to 90 wt% of the tablet core, more suitably 81 wt% to 90 wt% of the tablet core, optionally 82 wt% to 90 wt% of the tablet core. Optionally, colesevelam hydrochloride is present in the unit dose pharmaceutical composition for oral administration in an amount of 70 wt% to 88 wt% of the tablet core, or in an amount of 75 wt% to 88 wt% of the tablet core, or in an amount of 76 wt% to 88 wt% of the tablet core, suitably in an amount of 80 wt% to 88 wt% of the tablet core, more suitably 81 wt% to 88 wt% of the tablet core, optionally 82 wt% to 88 wt% of the tablet core. Further optionally, colesevelam hydrochloride is present in the unit dose pharmaceutical composition for oral administration in an amount of 70 wt% to 85 wt% of the tablet core, or in an amount of 75 wt% to 85 wt% of the tablet core, or in an amount of 76 wt% to 85 wt% of the tablet core, suitably in an amount of 80 wt% to 85 wt% of the tablet core, more suitably 81 wt% to 85 wt% of the tablet core, optionally 82 wt% to 85 wt% of the tablet core.

Preferably, colesevelam and/or pharmaceutically acceptable salts thereof is present in the unit dose pharmaceutical composition for oral administration in an amount of from 75 wt% to 90 wt% of the tablet core, preferably in an amount of from 80 wt% to 90 wt% of the tablet core, more preferably in an amount of from 81 wt% to 90 wt% of the tablet core. For example, colesevelam and/or pharmaceutically acceptable salts thereof may be present in the unit dose pharmaceutical composition for oral administration in an amount of from 75 wt% to 90 wt% of the tablet core, optionally, in an amount of from 80 wt% to 88 wt% of the tablet core, optionally in an amount of from 81 wt% to 85 wt% of the tablet core, optionally 82 wt% to 85 wt% of the tablet core.

In another preferable embodiment, colesevelam hydrochloride is present in the unit dose pharmaceutical composition for oral administration in an amount of from 75 wt% to 90 wt% of the tablet core, preferably in an amount of from 80 wt% to 90 wt% of the tablet core, more preferably in an amount of from 81 wt% to 90 wt% of the tablet core. For example, colesevelam hydrochloride may be present in the unit dose pharmaceutical composition for oral administration in an amount of from 75 wt% to 90 wt% of the tablet core, optionally, in an amount of from 80 wt% to 88 wt% of the tablet core, optionally in an amount of from 81 wt% to 85 wt% of the tablet core, optionally 82 wt% to 85 wt% of the tablet core.

The weight percentages of colesevelam or a pharmaceutically acceptable salt(s) thereof, for example colesevelam hydrochloride, refers to the weight percent of anhydrous colesevelam or a pharmaceutically acceptable salt(s) thereof (e.g., anhydrous colesevelam hydrochloride). For example, "at least 66 wt% colesevelam and/or pharmaceutically acceptable salt thereof" implies "at least 66 wt% anhydrous colesevelam and/or pharmaceutically acceptable salt thereof". Likewise, "at least 66 wt% colesevelam hydrochloride" implies "at least 66 wt% anhydrous colesevelam hydrochloride". Hydrated forms of colesevelam may also be used, however, the amounts employed are adjusted to compensate for the amount of water present, i.e., when using hydrated forms of colesevelam, the amount of water/hydrate present in the hydrated colesevelam does not contribute to the weight percentage of colesevelam based on dry weight.

Suitably, hydrated forms of colesevelam or pharmaceutically acceptable salts thereof that may be used in the present invention comprise less than about 20 wt% water based on the total weight of the hydrated colesevelam or pharmaceutically acceptable salt thereof, preferably less than about 10 wt%, e.g., about 1 wt% , or 2% wt%, or 3 wt%, or 4 wt%, or 5 wt%, or 6 wt%, or 7 wt%, or 8 wt%, or 9 wt%, water based on the total weight of the hydrated colesevelam or pharmaceutically acceptable salt thereof or any fractions of the above integers. When using hydrated forms of colesevelam, the amount of water present in the hydrated colesevelam (shall be compensated for, thus in order to achieve 0.9 X g of colesevelam (based on dry weight colesevelam), X g of a hydrated colesevelam comprising 10% water would be required.

The pharmaceutical composition described herein has a high drug content, and in particular, a high drug load in each dosage form. This allows for a decrease in the number of units to be administered to a subject in need thereof, which in turn, improves patient compliance.

Suitably, the tablet core further comprises one or more of a diluent, binder, disintegrant, glidant or lubricant, preferably wherein the tablet core comprises a diluent, a binder, a glidant and a lubricant, and optionally a disintegrant.

Optionally, the tablet core comprises a diluent or mixture of two or more diluents selected from the group comprising: lactose, a sugar such as sucrose and fructose, dextrose, dextrates, dextrans, a polyol such as sorbitol, mannitol and xylitol, dibasic calcium phosphate, tribasic calcium phosphate, calcium sulphate, sodium chloride, starch (e.g., pregelatinized starch), and cellulose or a derivative thereof (e.g., methyl, ethyl or hydroxyethyl cellulose).

Preferably, the tablet core comprises a diluent or mixture of two or more diluents selected from the group comprising: cellulose or a derivative thereof, lactose, calcium phosphate, starch, modified starch, and mannitol.

The diluent is present in an amount of from 6 to 21 wt% of the tablet core, such as 6 wt%, 7wt%, 8 wt%, 9 wt%, 10 wt%, 11 wt%, 12 wt%, 13 wt%, 14 wt%, 15 wt%, 16 wt%, 17 wt%, 18 wt%, 19 wt%, 20 wt%, or 21 wt%, preferably from 8 to 18 wt% of the tablet core, more preferably, the diluent is present in an amount of from 9 to 13 wt% of the tablet core.

Preferably, the tablet core comprises a diluent, wherein the diluent is microcrystalline cellulose, optionally wherein the diluent is a porous microcrystalline cellulose having an average particle size of in the range of from 80 to 110 µm and an angle of repose of less than 40°, preferably, wherein the porous microcrystalline cellulose has an average particle size of about 90 µm and an angle of repose of 34°.

The binder may be selected from the group comprising: polyvinyl alcohol, natural gums, gelatin, a polymethacrylate, cellulose or a derivative thereof (e.g., ethyl cellulose) and copovidone, or any combination thereof. Preferably, the binder is copovidone or polyvinyl alcohol. More preferably, the binder is copovidone.

The tablet core may comprise a binder, wherein the binder is present in an amount of from 1 to 7 wt% of the tablet core, preferably, the binder is present in an amount of from 2 to 6 wt% of the tablet core, optionally, in an amount of from 3 to 5 wt% of the tablet core.

The tablet core comprises a disintegrant selected from the group comprising: sodium carboxymethyl cellulose, cross-linked sodium carboxymethyl cellulose, bentonite, alginic acid or a derivative or salt thereof, and crospovidone, or any combination thereof. Preferably, the disintegrant is crospovidone or cross-linked sodium carboxymethyl cellulose. More preferably, the disintegrant is crospovidone.

The tablet core may comprise a disintegrant wherein the disintegrant is present in an amount of from 0.01 to 2 wt% of the tablet core, preferably wherein the disintegrant is present in an amount of from 0.1 to 1 wt% of the tablet core.

The tablet core comprises a glidant selected from the group comprising: talc, starch, magnesium oxide, silica (silicon dioxide) and a silicate, such as magnesium or calcium silicate, or any combination thereof. Preferably, the glidant is silica, colloidal silicon dioxide, talc, or magnesium oxide. More preferably, the glidant is colloidal silicon dioxide.

The tablet core may comprise a glidant wherein the glidant is present in an amount of from 0.1 to 2 wt% of the tablet core, preferably wherein the glidant is present in an amount of from 0.5 to 1.5 wt% of the tablet core, optionally, the glidant is present in an amount of from 0.7 to 1.2 wt% of the tablet core.

The tablet core comprises a lubricant selected from the group comprising: stearic acid or a salt thereof (e.g., magnesium, calcium or zinc stearate), polyethylene glycol, fumaric acid or a salt thereof, glyceryl behenate, glyceryl palmitostearate, wax, and sodium benzoate, or any combination thereof. Preferably, the lubricant is stearic acid or a salt thereof, fumaric acid or a salt thereof, or polyethylene glycol.

The tablet core may comprise a lubricant and the lubricant is present in an amount of from 0.1 to 2 wt% of the tablet core, preferably wherein the lubricant is present in an amount of from 0.5 to 1.5 wt% of the tablet core, optionally, the lubricant is present in an amount of from 0.7 to 1.2 wt% of the tablet core.

Preferably, the tablet core comprises microcrystalline cellulose, copovidone, colloidal silicon dioxide, magnesium stearate, and optionally crospovidone.

More preferably, the tablet core comprises:
microcrystalline cellulose in an amount of from 9 to 13 wt% of the tablet core,
copovidone in an amount of from 3 to 4 wt% of the tablet core,
colloidal silicon dioxide in an amount of from 0.5 to 1.5 wt% of the tablet core,
magnesium stearate in an amount of from 0.5 to 1.5 wt% of the tablet core, and optionally, crospovidone in an amount of from 0 to 1 wt% of the tablet core.

In addition to the patient compliance issues discussed above, the treatment of bile acid diarrhea with currently available bile acid sequestrants has other substantial drawbacks which limit the use of such treatment in current clinical practice. These limitations are briefly discussed hereinafter.

The currently available formulations of bile acid sequestrants are characterized by immediate release in the stomach. The bile acid sequestrants therefore exert their pharmacological action starting in the duodenum: once the bile acids are released by the gallbladder in the duodenum, they are bound by the sequestrants and cannot exert their physiological digestive role of solubilization and absorption-enhancement of lipids and liposoluble vitamins. This leads to nutritional disorders and deficiencies of liposoluble vitamins.

In one aspect, the invention provides pharmaceutical compositions containing colesevelam or a pharmaceutically acceptable salt thereof, such as colesevelam hydrochloride, with modified release of the active substance. In some embodiments, the pharmaceutical compositions of the invention have delayed release of the colesevelam or a pharmaceutically acceptable salt thereof, e.g., delayed release of colesevelam hydrochloride. In other embodiments, the pharmaceutical compositions of the invention have delayed and extended release of the colesevelam or a pharmaceutically acceptable salt thereof, e.g., delayed and extended release of colesevelam hydrochloride.

Optionally, the active substance is released from the tablet core at pH of at least 5, such as 5.1, or 5.2, or 5.3, or 5.4, or 5.5, or 5.6, or 5.7, or 5.8, or 5.9, or 6, or 6.1, or 6.2, or 6.3, or 6.4, or 6.5, or 6.6, or 6.7, or 6.8, or 6.9, or 7, or 7.1, or 7.2, or 7.3, or 7.4, or 7.5. Preferably, the active substance is released from the tablet core at pH in the range of about 6.5 to about 7.2, more preferably in the range between 6.8 to about 7. According to a preferred aspect, the active substance is released from the tablet core at pH of 6.8. According to another preferred aspect, the active substance is released from the tablet core at pH of 7. According to a further preferred aspect, the active substance is released from the tablet core at pH of 7.2.

The gastro-resistant coating may comprise methacrylic acid/methyl methacrylate (1:1) copolymer, or methacrylic acid/methyl methacrylate (1:2) copolymer, or a mixture of methacrylic acid/methyl methacrylate (1:1) copolymer and of methacrylic acid/methyl methacrylate (1:2) copolymer.

Optionally, the gastro-resistant coating comprises methacrylic acid/methyl methacrylate copolymers (EUDRAGIT^{®} (S) and/or EUDRAGIT^{®} (L)); said gastro-resistant coating may optionally further comprise cellulose derivatives, such as cellulose aceto-phthalate, ethyl cellulose.

Optionally, the coating comprises a gastro-resistant coating, as described above, and further comprises a non-gastro-resistant coating comprising a cellulose derivative, such as cellulose aceto-phthalate, and ethyl cellulose. Optionally, such non-gastro-resistant coating can be outer to the gastro-resistant coating (and further from the tablet core). Preferably, such non-gastro-resistant coating can optionally be inner to the gastro-resistant coating (and closer to the tablet core).

The weight ratio of methacrylic acid/methyl methacrylate (1:1) copolymer to methacrylic acid/methyl methacrylate (1:2) copolymer may be in the range of from 0.5:1 to 3:1, suitably, in a weight range of from 1:1 to 2:1.

Suitably, the pharmaceutical composition described herein is formulated for targeted and/or delayed release. Suitably, release starts in the ileum and continues into the initial portion of the colon. The composition is able to transit without disintegration through the stomach, the duodenum and the jejunum and can start its drug release in the ileum and if needed, continues in the initial portion of colon. The delayed release of the colesevelam from the tablet core prevents drug/vitamin interactions from taking place in the upper intestinal tracts.

Optionally, the pharmaceutical composition is formulated for release starting in the ileum and throughout the ascending colon.

Optionally, the pharmaceutical composition is formulated for release starting in the ileum and throughout the ascending colon and transverse colon.

Optionally, the pharmaceutical composition is formulated for release starting in the ileum and throughout the ascending colon, transverse colon and descending colon.

Optionally, the pharmaceutical composition is formulated for release starting in the ileum and throughout the ascending colon, transverse colon, descending colon and sigmoid colon.

Optionally, the pharmaceutical composition is formulated for release starting in the ileum and throughout the ascending colon, transverse colon, descending colon, sigmoid colon and the rectum.

The delayed release characteristics of the pharmaceutical composition described herein can assure that the excess of bile acids could be inactivated by binding to the sequestrant molecules, avoiding the irritating contact with the colonic mucosa that typically generates the diarrhea.

This site specific release of the active substance from the compositions described herein is achieved via delayed release assured by the film-coating with a gastro-resistant polymer or mixture of polymers, and an immediate or sustained release from the tablet core once the film coating has dissolved. The binding of the excess of bile acid before the entrance into the ascending colon, prevents excess bile acids contacting the colonic mucosa, where the bile acids exert their pro-secretory effects, increase the motility and promote diarrhea.

The pharmaceutical composition of the invention achieves site specific release of the active substance as the unit dosage form transits intact through the gastrointestinal tract until the gastro-resistant coating is degraded or dissolved at pH of at least 5, such as when the pH is approximately between 6.5-7.5, preferably between 6.8 to 7.2, which is typically observed in the ileum. Once the gastro-resistant coating is degraded or dissolved, colesevelam or a pharmaceutically acceptable salt thereof starts to (and optionally continues to) leak (e.g., elute) from the tablet core during the transit time of the drug dosage unit in the ileum and its entrance into the ascending colon. The colesevelam continues to exert its bile acid binding capacity in the colon.

This late availability of the sequestrant leaked by the dosage form in the two last intestinal segments ensures that no drug or vitamin interactions take place in the upper intestinal districts due to the colonic transit time. At the same time, the delayed release characteristic of the formulation described herein ensures that the excess of bile acids (that need to be sequestered to treat the bile acid diarrhea) is inactivated by binding to the colesevelam, avoiding the irritating contact with the colonic mucosa that typically generates the diarrhea.

To achieve site specific release, the composition may be structured as a unit dose tablet formed by a direct compression method.

The pharmaceutical compositions of the invention are formulated in form of tablets or capsules. Preferably, the pharmaceutical compositions of the invention are formulated as tablets.

The tablet core has a length of less than about 25 ± 0.5 mm, preferably less than about 24 ± 0.5 mm, more preferably of less than 23 mm ± 0.5 mm.

The tablet core has a thickness of less than about 11.5 mm ± 0.5 mm, preferably of less than about 10.5 mm ± 0.5 mm, more preferably of less than about 9.5 mm ± 0.5 mm, more preferably of less than or equal to about 8.5 mm ± 0.5 mm.

Providing tablets having a tablet core with length and thickness according to the formulations of the present invention improves patient compliance.

The tablets according to the present invention contain colesevelam or a pharmaceutically acceptable salt thereof is present in the **tablet core** in an amount of from about 700 mg to about 1400 mg, such as about: 700 mg, 725 mg, 750 mg, 775 mg, 800 mg, 825 mg, 850 mg, 875 mg, 900 mg, 925 mg, 950 mg, 975 mg, 1000 mg, 1025 mg, 1050 mg, 1075 mg, 1100 mg, 1125 mg, 1150 mg, 1175 mg or 1200 mg. For example, the tablet cores can contain colesevelam hydrochloride in the **tablet core** in an amount of from about 700 mg to about 1400 mg, such as about: 700 mg, 725 mg, 750 mg, 775 mg, 800 mg, 825 mg, 850 mg, 875 mg, 900 mg, 925 mg, 950 mg, 975 mg, 1000 mg, 1025 mg, 1050 mg, 1075 mg, 1100 mg, 1125 mg, 1150 mg, 1175 mg or 1200 mg.

Preferably, the tablet core comprises about 900 mg of colesevelam, or pharmaceutically acceptable salts thereof, such as colesevelam hydrochloride.

Preferably, the tablets comprise a tablet core and an external film-coating i.e., a gastro-resistant coating covering the tablet core.

Optionally, the tablets comprise a sub-coating between the tablet core and the gastro-resistant coating, optionally, wherein the sub-coating comprises hydroxypropyl cellulose.

The tablet core has a hardness of greater than about 70 N, optionally, greater than about 80 N, optionally, greater than about 90 N, when measured in accordance with US Pharmacopeia monograph <1217> or with European Pharmacopoeia monograph 2.9.8.

The tablet core may have a hardness in the range of from 70 N to 450 N, such as from 80 N to 400 N, optionally from 90 N to 370 N, such as from 100 N to 330 N when measured in accordance with US Pharmacopeia monograph <1217> or with European Pharmacopoeia monograph 2.9.8.

For example, the tablet core may have a hardness in the range of from 70 N to 450 N, optionally, from 80 N to 450 N, optionally, from 90 N to 450 N, optionally from 100 N to 450 N when measured in accordance with US Pharmacopeia monograph <1217> or with European Pharmacopoeia monograph 2.9.8. Optionally, the tablet core may have a hardness in the range of from 70 N to 400 N, optionally, from 80 N to 400 N, optionally, from 90 N to 400 N, optionally from 100 N to 400 N, when measured in accordance with US Pharmacopeia monograph <1217> or with European Pharmacopoeia monograph 2.9.8. Further optionally, the tablet core may have a hardness in the range of from 70 N to 350 N, optionally, from 80 N to 350 N, optionally, from 90 N to 350 N, optionally from 100 N to 350 N when measured in accordance with US Pharmacopeia monograph <1217> or with European Pharmacopoeia monograph 2.9.8. Further optionally, the tablet core may have a hardness in the range of from 70 N to 330 N, optionally, from 80 N to 330 N, optionally, from 90 N to 330 N, optionally from 100 N to 330 N when measured in accordance with US Pharmacopeia monograph <1217> or with European Pharmacopoeia monograph 2.9.8.

Preferably, the pharmaceutical compositions of the invention are in the form of tablets comprising a tablet core characterized by a friability equal to or less than 1.5%, or 1.2%, preferably equal to or less than 1%, more preferably equal to or less than 0.8%, much more preferably equal to or less than 0.5% as determined in accordance with US Pharmacopoeia monograph <1216> or with European Pharmacopoeia monograph 2.9.7.

Preferably, the pharmaceutical compositions of the invention are in the form of tablets, comprising colesevelam or a pharmaceutically acceptable salt thereof (e.g., colesevelam hydrochloride) as the drug substance and at least one pharmaceutically acceptable excipient, characterized in that:
- the tablet comprises a tablet core and a suitable gastro-resistant or enteric film-coating;
- the tablet core has a content of colesevelam or a pharmaceutically acceptable salt thereof (such as colesevelam hydrochloride) above about 66% w/w;
- the tablet core has a hardness of more than about 120 N;
- the tablet core has a friability equal to or less than 0.5%;
- the tablets have a modified, or delayed, or delayed/extended, or extended-release profile; and
- wherein said tablets release the active substance at pH above about 5.

The gastro-resistant coating (or enteric film-coating) dissolves at pH above 5, such as 5.1, or 5.2, or 5.3, or 5.4, or 5.5, or 5.6, or 5.7, or 5.8, or 5.9, or 6, or 6.1, or 6.2, or 6.3, or 6.4, or 6.5, or 6.6, or 6.7, or 6.8, or 6.9, or 7, or 7.1, or 7.2, or 7.3, or 7.4, or 7.5. Preferably, the gastro-resistant coating (or enteric film-coating) dissolves at a pH in the range of about 6.5 to about 7.2, more preferably in the range between 6.8 to about 7. According to a preferred aspect, the gastro-resistant coating (or enteric film-coating) dissolves at pH of 6.8. According to another preferred aspect, the gastro-resistant coating (or enteric film-coating) dissolves at pH of 7. According to a further preferred aspect, the gastro-resistant coating (or enteric film-coating) dissolves at pH of 7.2. More preferably, the gastro-resistant coating (or enteric film-coating) dissolves at pH of 6.8.

Optionally, the pharmaceutical compositions of the present invention can contain up to about 1,200 mg of colesevelam or a pharmaceutically acceptable salt thereof. Optionally, the pharmaceutical compositions of the present invention can contain up to about 1,200 mg of colesevelam hydrochloride.

Optionally, the pharmaceutical compositions of the present invention can contain up to about 1,100 mg of colesevelam or a pharmaceutically acceptable salt thereof. Optionally, the pharmaceutical compositions of the present invention can contain up to about 1,100 mg of colesevelam hydrochloride.

Optionally, the pharmaceutical compositions of the present invention can contain up to about 1,000 mg of colesevelam or a pharmaceutically acceptable salt thereof. Optionally, the pharmaceutical compositions of the present invention can contain up to about 1,000 mg of colesevelam hydrochloride.

Suitably, the pharmaceutical compositions of the present invention can contain about 900 mg of colesevelam or a pharmaceutically acceptable salt thereof. Optionally, the pharmaceutical compositions of the present invention can contain about 900 mg of colesevelam hydrochloride.

Suitably, the pharmaceutical compositions of the present invention can contain at least about 800 mg of colesevelam or a pharmaceutically acceptable salt thereof. Optionally, the pharmaceutical compositions of the present invention can contain at least about 800 mg of colesevelam hydrochloride.

Suitably, the pharmaceutical compositions of the present invention can contain at least 700 mg of colesevelam or a pharmaceutically acceptable salt thereof. Optionally, the pharmaceutical compositions of the present invention can contain at least about 700 mg of colesevelam hydrochloride.

The method of manufacturing a unit dose pharmaceutical composition for oral administration comprises the steps of:
providing a tablet core comprising colesevelam or a pharmaceutically acceptable salt thereof,
wherein the colesevelam or a pharmaceutically acceptable salt thereof is at least 66 wt% of the tablet core, preferably at least 70 wt%, more preferably at least 76 wt%,
wherein the colesevelam or a pharmaceutically acceptable salt thereof is present in the **tablet core** in an amount of from about 700 to about 1400, such as from about 850 mg to about 1200 mg; and
coating the tablet core with a gastro-resistant coating, and optionally a sub-coating between the tablet core and the gastro-resistant coating.

Suitably, the present invention provides a unit dose pharmaceutical composition for oral administration comprising a tablet core and a gastro-resistant coating covering the tablet core, wherein the tablet core comprises colesevelam or a pharmaceutically acceptable salt thereof,
wherein the colesevelam or a pharmaceutically acceptable salt thereof is present in an amount of from 76 wt% to 90 wt% of the tablet core, optionally, from 80 wt% to 90 wt%, optionally from 81 wt% to 90 wt%, such as from 82 wt% to 90 wt% of the tablet core,
wherein the colesevelam or a pharmaceutically acceptable salt thereof is present in the tablet core in an amount of from about 700 mg to 1400 mg.

Suitably, the present invention also provides a unit dose pharmaceutical composition for oral administration comprising a tablet core and a gastro-resistant coating covering the tablet core, wherein the tablet core comprises colesevelam hydrochloride,
wherein the colesevelam hydrochloride is present in an amount of from 76 wt% to 90 wt% of the tablet core, optionally, from 80 wt% to 90 wt%, optionally from 81 wt% to 90 wt%, such as from 82 wt% to 90 wt% of the tablet core and
wherein the colesevelam hydrochloride is present in the tablet core in an amount of from about 700 mg to 1400 mg.

For example, disclosed herein is a unit dose pharmaceutical composition for oral administration comprising a tablet core and a gastro-resistant coating covering the tablet core, wherein the tablet core comprises colesevelam or a pharmaceutically acceptable salt thereof,
wherein the colesevelam or a pharmaceutically acceptable salt thereof is present in an amount of from 76 wt% to 90 wt% of the tablet core, optionally, from 80 wt% to 90 wt%, optionally from 81 wt% to 90 wt%, such as from 82 wt% to 90 wt% of the tablet core,
wherein the colesevelam or a pharmaceutically acceptable salt thereof is present in the tablet core in an amount of from about 700 mg to 1400 mg,
wherein the tablet core has a hardness in the range of from 70 N to 450 N, optionally, from 80 N to 400 N, further optionally, from 90 N to 370 N, such as from 100 N to 330 N as determined in accordance with European Pharmacopoeia 2.9.8 or with US Pharmacopoeia monograph <1217>; and wherein the tablet core has a friability of from 0.001% to 1%, optionally, from 0.01 to 0.5%, optionally from 0.01 to 0.1% as determined in accordance with US Pharmacopoeia monograph <1216> or with European Pharmacopoeia monograph 2.9.7.

For example, disclosed herein is a unit dose pharmaceutical composition for oral administration comprising a tablet core and a gastro-resistant coating covering the tablet core, wherein the tablet core comprises colesevelam hydrochloride,
wherein the colesevelam hydrochloride is present in an amount of from 76 wt% to 90 wt% of the tablet core, optionally, from 80 wt% to 90 wt%, optionally from 81 wt% to 90 wt%, such as from 82 wt% to 90 wt% of the tablet core,
wherein the colesevelam hydrochloride is present in the tablet core in an amount of from about 700 mg to 1400 mg,
wherein the tablet core has a hardness in the range of from 70 N to 450 N, optionally, from 80 N to 400 N, further optionally, from 90 N to 370 N, such as from 100 N to 330 N as determined in accordance with European Pharmacopoeia 2.9.8 or with US Pharmacopoeia monograph <1217>; and wherein the tablet core has a friability of from 0.001% to 1%, optionally, from 0.01 to 0.5%, optionally from 0.01 to 0.1% as determined in accordance with US Pharmacopoeia monograph <1216> or with European Pharmacopoeia monograph 2.9.7.

Suitably, disclosed herein is a unit dose pharmaceutical composition for oral administration comprising a tablet core and a gastro-resistant coating covering the tablet core, wherein the tablet core comprises colesevelam or a pharmaceutically acceptable salt thereof,
wherein the colesevelam or a pharmaceutically acceptable salt thereof is present in an amount of from 76 wt% to 90 wt% of the tablet core, optionally, from 80 wt% to 90 wt%, optionally from 81 wt% to 90 wt%, such as from 82 wt% to 90 wt% of the tablet core,
wherein the colesevelam or a pharmaceutically acceptable salt thereof is present in the tablet core in an amount of from about 700 mg to 1400 mg,
wherein the tablet core has a hardness in the range of from 100 N to 330 N as determined in accordance with European Pharmacopoeia 2.9.8 or with US Pharmacopoeia monograph <1217>; and wherein the tablet core has a friability of from 0.01 to 0.1% as determined in accordance with US Pharmacopoeia monograph <1216> or with European Pharmacopoeia monograph 2.9.7.

Suitably, disclosed herein is a unit dose pharmaceutical composition for oral administration comprising a tablet core and a gastro-resistant coating covering the tablet core, wherein the tablet core comprises colesevelam hydrochloride,
wherein the colesevelam hydrochloride is present in an amount of from 76 wt% to 90 wt% of the tablet core, optionally, from 80 wt% to 90 wt%, optionally from 81 wt% to 90 wt%, such as from 82 wt% to 90 wt% of the tablet core,
wherein the colesevelam hydrochloride is present in the tablet core in an amount of from about 700 mg to 1400 mg,
wherein the tablet core has a hardness in the range of from 100 N to 330 N as determined in accordance with European Pharmacopoeia 2.9.8 or with US Pharmacopoeia monograph <1217>; and wherein the tablet core has a friability of from 0.01 to 0.1% as determined in accordance with US Pharmacopoeia monograph <1216> or with European Pharmacopoeia monograph 2.9.7.

Suitably, the present invention provides a unit dose pharmaceutical composition for oral administration comprising a tablet core and a gastro-resistant coating covering the tablet core, wherein the tablet core comprises colesevelam or a pharmaceutically acceptable salt thereof,
wherein the colesevelam or a pharmaceutically acceptable salt thereof is present in an amount of from 76 wt% to 90 wt% of the tablet core, optionally, from 80 wt% to 90 wt%, optionally from 81 wt% to 90 wt%, such as from 82 wt% to 90 wt% of the tablet core,
wherein the colesevelam or a pharmaceutically acceptable salt thereof is present in the tablet core in an amount of from about 700 mg to 1400 mg;
wherein the tablet core comprises a diluent, optionally the diluent is microcrystalline cellulose, optionally the diluent is a porous microcrystalline cellulose having an average particle size of in the range of from 80 to 110 µm and an angle of repose of less than about 40°, preferably, wherein the porous microcrystalline cellulose has an average particle size of about 90 µm and an angle of repose of about 34°, and wherein the diluent is present in an amount of from 8 to 18 wt% of the tablet core.

Suitably, the present invention provides a unit dose pharmaceutical composition for oral administration comprising a tablet core and a gastro-resistant coating covering the tablet core, wherein the tablet core comprises colesevelam hydrochloride,
wherein the colesevelam hydrochloride is present in an amount of from 76 wt% to 90 wt% of the tablet core, optionally, from 80 wt% to 90 wt%, optionally from 81 wt% to 90 wt%, such as from 82 wt% to 90 wt% of the tablet core,
wherein the colesevelam hydrochloride is present in the tablet core in an amount of from about 700 mg to 1400 mg;
wherein the tablet core comprises a diluent, optionally the diluent is microcrystalline cellulose, optionally the diluent is a porous microcrystalline cellulose having an average particle size of in the range of from 80 to 110 µm and an angle of repose of less than about 40°, preferably, wherein the porous microcrystalline cellulose has an average particle size of about 90 µm and an angle of repose of about 34°, and wherein the diluent is present in an amount of from 8 to 18 wt% of the tablet core.

For example, disclosed herein is a unit dose pharmaceutical composition for oral administration comprising a tablet core and a gastro-resistant coating covering the tablet core, wherein the tablet core comprises colesevelam or a pharmaceutically acceptable salt thereof,
wherein the colesevelam or a pharmaceutically acceptable salt thereof is present in an amount of from 76 wt% to 90 wt% of the tablet core, optionally, from 80 wt% to 90 wt%, optionally from 81 wt% to 90 wt%, such as from 82 wt% to 90 wt% of the tablet core,
wherein the colesevelam or a pharmaceutically acceptable salt thereof is present in the tablet core in an amount of from about 700 mg to 1400 mg;
wherein the tablet core comprises a diluent, optionally the diluent is microcrystalline cellulose, optionally the diluent is a porous microcrystalline cellulose having an average particle size of in the range of from 80 to 110 µm and an angle of repose of less than about 40°, preferably, wherein the porous microcrystalline cellulose has an average particle size of about 90 µm and an angle of repose of about 34°, wherein the diluent is present in an amount of from 8 to 18 wt% of the tablet core;
wherein the tablet core has a hardness in the range of from 70 N to 450 N, optionally, from 80 N to 400 N, further optionally, from 90 N to 370 N, such as from 100 N to 330 N as determined in accordance with European Pharmacopoeia 2.9.8 or with US Pharmacopoeia monograph <1217>; and wherein the tablet core has a friability of from 0.001% to 1%, optionally, from 0.01 to 0.5%, optionally from 0.01 to 0.1% as determined in accordance with US Pharmacopoeia monograph <1216> or with European Pharmacopoeia monograph 2.9.7.

By way of further example, disclosed herein is a unit dose pharmaceutical composition for oral administration comprising a tablet core and a gastro-resistant coating covering the tablet core, wherein the tablet core comprises colesevelam hydrochloride,
wherein the colesevelam hydrochloride is present in an amount of from 76 wt% to 90 wt% of the tablet core, optionally, from 80 wt% to 90 wt%, optionally from 81 wt% to 90 wt%, such as from 82 wt% to 90 wt% of the tablet core,
wherein the colesevelam hydrochloride is present in the tablet core in an amount of from about 700 mg to 1400 mg;
wherein the tablet core comprises a diluent, optionally the diluent is microcrystalline cellulose, optionally the diluent is a porous microcrystalline cellulose having an average particle size of in the range of from 80 to 110 µm and an angle of repose of less than about 40°, preferably, wherein the porous microcrystalline cellulose has an average particle size of about 90 µm and an angle of repose of about 34°, wherein the diluent is present in an amount of from 8 to 18 wt% of the tablet core;
wherein the tablet core has a hardness in the range of from 70 N to 450 N, optionally, from 80 N to 400 N, further optionally, from 90 N to 370 N, such as from 100 N to 330 N as determined in accordance with European Pharmacopoeia 2.9.8 or with US Pharmacopoeia monograph <1217>; and wherein the tablet core has a friability of from 0.001% to 1%, optionally, from 0.01 to 0.5%, optionally from 0.01 to 0.1% as determined in accordance with US Pharmacopoeia monograph <1216> or with European Pharmacopoeia monograph 2.9.7.

Suitably, disclosed herein is a unit dose pharmaceutical composition for oral administration comprising a tablet core and a gastro-resistant coating covering the tablet core, wherein the tablet core comprises colesevelam or a pharmaceutically acceptable salt thereof,
wherein the colesevelam or a pharmaceutically acceptable salt thereof is present in an amount of from 76 wt% to 90 wt% of the tablet core, optionally, from 80 wt% to 90 wt%, optionally from 81 wt% to 90 wt%, such as from 82 wt% to 90 wt% of the tablet core,
wherein the colesevelam or a pharmaceutically acceptable salt thereof is present in the **tablet core** in an amount of from about 700 mg to 1400 mg;
wherein the tablet core comprises a diluent, optionally the diluent is microcrystalline cellulose, optionally the diluent is a porous microcrystalline cellulose having an average particle size of in the range of from 80 to 110 µm and an angle of repose of less than about 40°, preferably, wherein the porous microcrystalline cellulose has an average particle size of about 90 µm and an angle of repose of about 34°, wherein the diluent is present in an amount of from 8 to 18 wt% of the tablet core;
wherein the tablet core has a hardness in the range of from 100 N to 330 N as determined in accordance with European Pharmacopoeia 2.9.8 or with US Pharmacopoeia monograph <1217>; and wherein the tablet core has a friability of 0.01 to 0.1% as determined in accordance with US Pharmacopoeia monograph <1216> or with European Pharmacopoeia monograph 2.9.7.

Suitably, disclosed herein is a unit dose pharmaceutical composition for oral administration comprising a tablet core and a gastro-resistant coating covering the tablet core, wherein the tablet core comprises colesevelam hydrochloride,
wherein the colesevelam hydrochloride is present in an amount of from 76 wt% to 90 wt% of the tablet core, optionally, from 80 wt% to 90 wt%, optionally from 81 wt% to 90 wt%, such as from 82 wt% to 90 wt% of the tablet core,
wherein the colesevelam hydrochloride is present in the tablet core in an amount of from about 700 mg to 1400 mg;
wherein the tablet core comprises a diluent, optionally the diluent is microcrystalline cellulose, optionally the diluent is a porous microcrystalline cellulose having an average particle size of in the range of from 80 to 110 µm and an angle of repose of less than about 40°, preferably, wherein the porous microcrystalline cellulose has an average particle size of about 90 µm and an angle of repose of about 34°, wherein the diluent is present in an amount of from 8 to 18 wt% of the tablet core;
wherein the tablet core has a hardness in the range of from 100 N to 330 N as determined in accordance with European Pharmacopoeia 2.9.8 or with US Pharmacopoeia monograph <1217>; and wherein the tablet core has a friability of 0.01 to 0.1% as determined in accordance with US Pharmacopoeia monograph <1216> or with European Pharmacopoeia monograph 2.9.7.

Suitably, the present invention provides a unit dose pharmaceutical composition for oral administration comprising a tablet core and a gastro-resistant coating covering the tablet core, wherein the tablet core comprises colesevelam or a pharmaceutically acceptable salt thereof,
wherein the colesevelam or a pharmaceutically acceptable salt thereof is present in an amount of from 76 wt% to 88 wt% of the tablet core, optionally, from 80 wt% to 88 wt%, optionally from 81 wt% to 88 wt%, such as from 82 wt% to 88 wt% of the tablet core,
wherein the colesevelam or a pharmaceutically acceptable salt thereof is present in the **tablet core** in an amount of from about 700 mg to 1400 mg;
wherein the tablet core comprises a diluent, optionally the diluent is microcrystalline cellulose, optionally the diluent is a porous microcrystalline cellulose having an average particle size of in the range of from 80 to 110 µm and an angle of repose of less than about 40°, preferably, wherein the porous microcrystalline cellulose has an average particle size of about 90 µm and an angle of repose of about 34°, wherein the diluent is present in an amount of from 8 to 18 wt% of the tablet core;
wherein the tablet core comprises a binder present in an amount of from 2 to 6 wt% of the tablet core, optionally, wherein the binder is copovidone.

Additionally, and also suitably, the present invention provides a unit dose pharmaceutical composition for oral administration comprising a tablet core and a gastro-resistant coating covering the tablet core, wherein the tablet core comprises colesevelam hydrochloride,
wherein the colesevelam hydrochloride is present in an amount of from 76 wt% to 88 wt% of the tablet core, optionally, from 80 wt% to 88 wt%, optionally from 81 wt% to 88 wt%, such as from 82 wt% to 88 wt% of the tablet core,
wherein the colesevelam hydrochloride is present in the tablet core in an amount of from about 700 mg to 1400 mg;
wherein the tablet core comprises a diluent, optionally the diluent is microcrystalline cellulose, optionally the diluent is a porous microcrystalline cellulose having an average particle size of in the range of from 80 to 110 µm and an angle of repose of less than about 40°, preferably, wherein the porous microcrystalline cellulose has an average particle size of about 90 µm and an angle of repose of about 34°, wherein the diluent is present in an amount of from 8 to 18 wt% of the tablet core;
wherein the tablet core comprises a binder present in an amount of from 2 to 6 wt% of the tablet core, optionally, wherein the binder is copovidone.

For example, disclosed herein is a unit dose pharmaceutical composition for oral administration comprising a tablet core and a gastro-resistant coating covering the tablet core, wherein the tablet core comprises colesevelam or a pharmaceutically acceptable salt thereof,
wherein the colesevelam or a pharmaceutically acceptable salt thereof is present in an amount of from 76 wt% to 88 wt% of the tablet core, optionally, from 80 wt% to 88 wt%, optionally from 81 wt% to 88 wt%, such as from 82 wt% to 88 wt% of the tablet core,
wherein the colesevelam or a pharmaceutically acceptable salt thereof is present in the **tablet core** in an amount of from about 700 mg to 1400 mg;
wherein the tablet core comprises a diluent, optionally the diluent is microcrystalline cellulose, optionally the diluent is a porous microcrystalline cellulose having an average particle size of in the range of from 80 to 110 µm and an angle of repose of less than about 40°, preferably, wherein the porous microcrystalline cellulose has an average particle size of about 90 µm and an angle of repose of about 34°, wherein the diluent is present in an amount of from 8 to 18 wt% of the tablet core;
wherein the tablet core comprises a binder present in an amount of from 2 to 6 wt% of the tablet core, optionally, wherein the binder is copovidone.
wherein the tablet core has a hardness in the range of from 70 N to 450 N, optionally, from 80 N to 400 N, further optionally, from 90 N to 370 N, such as from 100 N to 330 N as determined in accordance with European Pharmacopoeia 2.9.8 or with US Pharmacopoeia monograph <1217>; and wherein the tablet core has a friability of from 0.001% to 1%, optionally, from 0.01 to 0.5%, optionally from 0.01 to 0.1% as determined in accordance with US Pharmacopoeia monograph <1216> or with European Pharmacopoeia monograph 2.9.7.

By way of further example, disclosed herein is a unit dose pharmaceutical composition for oral administration comprising a tablet core and a gastro-resistant coating covering the tablet core, wherein the tablet core comprises colesevelam hydrochloride,
wherein the colesevelam hydrochloride is present in an amount of from 76 wt% to 88 wt% of the tablet core, optionally, from 80 wt% to 88 wt%, optionally from 81 wt% to 88 wt%, such as from 82 wt% to 88 wt% of the tablet core,
wherein the colesevelam hydrochloride is present in the **tablet core** in an amount of from about 700 mg to 1400 mg;
wherein the tablet core comprises a diluent, optionally the diluent is microcrystalline cellulose, optionally the diluent is a porous microcrystalline cellulose having an average particle size of in the range of from 80 to 110 µm and an angle of repose of less than about 40°, preferably, wherein the porous microcrystalline cellulose has an average particle size of about 90 µm and an angle of repose of about 34°, wherein the diluent is present in an amount of from 8 to 18 wt% of the tablet core;
wherein the tablet core comprises a binder present in an amount of from 2 to 6 wt% of the tablet core, optionally, wherein the binder is copovidone.
wherein the tablet core has a hardness in the range of from 70 N to 450 N, optionally, from 80 N to 400 N, further optionally, from 90 N to 370 N, such as from 100 N to 330 N as determined in accordance with European Pharmacopoeia 2.9.8 or with US Pharmacopoeia monograph <1217>; and wherein the tablet core has a friability of from 0.001% to 1%, optionally, from 0.01 to 0.5%, optionally from 0.01 to 0.1% as determined in accordance with US Pharmacopoeia monograph <1216> or with European Pharmacopoeia monograph 2.9.7.

Suitably, disclosed herein is a unit dose pharmaceutical composition for oral administration comprising a tablet core and a gastro-resistant coating covering the tablet core, wherein the tablet core comprises colesevelam or a pharmaceutically acceptable salt thereof,
wherein the colesevelam or a pharmaceutically acceptable salt thereof is present in an amount of from 76 wt% to 88 wt% of the tablet core, optionally, from 80 wt% to 88 wt%, optionally from 81 wt% to 88 wt%, such as from 82 wt% to 88 wt% of the tablet core,
wherein the colesevelam or a pharmaceutically acceptable salt thereof is present in the **tablet core** in an amount of from about 700 mg to 1400 mg;
wherein the tablet core comprises a diluent, optionally the diluent is microcrystalline cellulose, optionally the diluent is a porous microcrystalline cellulose having an average particle size of in the range of from 80 to 110 µm and an angle of repose of less than about 40°, preferably, wherein the porous microcrystalline cellulose has an average particle size of about 90 µm and an angle of repose of about 34°, wherein the diluent is present in an amount of from 8 to 18 wt% of the tablet core;
wherein the tablet core comprises a binder present in an amount of from 2 to 6 wt% of the tablet core, optionally, wherein the binder is copovidone.
wherein the tablet core has a hardness in the range of from 100 N to 330 N as determined in accordance with European Pharmacopoeia 2.9.8 or with US Pharmacopoeia monograph <1217>; and wherein the tablet core has a friability of 0.01 to 0.1% as determined in accordance with US Pharmacopoeia monograph <1216> or with European Pharmacopoeia monograph 2.9.7.

Suitably, disclosed herein is a unit dose pharmaceutical composition for oral administration comprising a tablet core and a gastro-resistant coating covering the tablet core, wherein the tablet core comprises colesevelam hydrochloride,
wherein the colesevelam hydrochloride is present in an amount of from 76 wt% to 88 wt% of the tablet core, optionally, from 80 wt% to 88 wt%, optionally from 81 wt% to 88 wt%, such as from 82 wt% to 88 wt% of the tablet core,
wherein the colesevelam hydrochloride is present in the tablet core in an amount of from about 700 mg to 1400 mg;
wherein the tablet core comprises a diluent, optionally the diluent is microcrystalline cellulose, optionally the diluent is a porous microcrystalline cellulose having an average particle size of in the range of from 80 to 110 µm and an angle of repose of less than about 40°, preferably, wherein the porous microcrystalline cellulose has an average particle size of about 90 µm and an angle of repose of about 34°, wherein the diluent is present in an amount of from 8 to 18 wt% of the tablet core;
wherein the tablet core comprises a binder present in an amount of from 2 to 6 wt% of the tablet core, optionally, wherein the binder is copovidone.
wherein the tablet core has a hardness in the range of from 100 N to 330 N as determined in accordance with European Pharmacopoeia 2.9.8 or with US Pharmacopoeia monograph <1217>; and wherein the tablet core has a friability of 0.01 to 0.1% as determined in accordance with US Pharmacopoeia monograph <1216> or with European Pharmacopoeia monograph 2.9.7.

The present invention further provides a unit dose pharmaceutical composition for oral administration comprising a tablet core and a gastro-resistant coating covering the tablet core, wherein the tablet core comprises colesevelam or a pharmaceutically acceptable salt thereof,
wherein the colesevelam or a pharmaceutically acceptable salt thereof is present in an amount of from 76 wt% to 88 wt% of the tablet core, optionally, from 80 wt% to 88 wt%, optionally from 81 wt% to 88 wt%, such as from 82 wt% to 88 wt% of the tablet core,
wherein the colesevelam or a pharmaceutically acceptable salt thereof is present in the **tablet core** in an amount of from about 700 mg to 1400 mg;
wherein the tablet core comprises a diluent, optionally the diluent is microcrystalline cellulose, optionally the diluent is a porous microcrystalline cellulose having an average particle size of in the range of from 80 to 110 µm and an angle of repose of less than about 40°, preferably, wherein the porous microcrystalline cellulose has an average particle size of about 90 µm and an angle of repose of about 34°, wherein the diluent is present in an amount of from 8 to 18 wt% of the tablet core;
wherein the tablet core comprises a binder, present in an amount of from 2 to 6 wt% of the tablet core, optionally, wherein the binder is copovidone;
wherein the tablet core comprises a glidant, present in an amount of from 0.5 to 1.5 wt% of the tablet core, optionally, wherein the glidant is a silica, such as colloidal silicon dioxide.

The present invention still further provides a unit dose pharmaceutical composition for oral administration comprising a tablet core and a gastro-resistant coating covering the tablet core, wherein the tablet core comprises colesevelam hydrochloride,
wherein the colesevelam hydrochloride is present in an amount of from 76 wt% to 88 wt% of the tablet core, optionally, from 80 wt% to 88 wt%, optionally from 81 wt% to 88 wt%, such as from 82 wt% to 88 wt% of the tablet core,
wherein the colesevelam hydrochloride is present in the tablet core in an amount of from about 700 mg to 1400 mg;
wherein the tablet core comprises a diluent, optionally the diluent is microcrystalline cellulose, optionally the diluent is a porous microcrystalline cellulose having an average particle size of in the range of from 80 to 110 µm and an angle of repose of less than about 40°, preferably, wherein the porous microcrystalline cellulose has an average particle size of about 90 µm and an angle of repose of about 34°, wherein the diluent is present in an amount of from 8 to 18 wt% of the tablet core;
wherein the tablet core comprises a binder, present in an amount of from 2 to 6 wt% of the tablet core, optionally, wherein the binder is copovidone;
wherein the tablet core comprises a glidant, present in an amount of from 0.5 to 1.5 wt% of the tablet core, optionally, wherein the glidant is a silica, such as colloidal silicon dioxide.

For example, disclosed herein is a unit dose pharmaceutical composition for oral administration comprising a tablet core and a gastro-resistant coating covering the tablet core, wherein the tablet core comprises colesevelam or a pharmaceutically acceptable salt thereof,
wherein the colesevelam or a pharmaceutically acceptable salt thereof is present in an amount of from 76 wt% to 88 wt% of the tablet core, optionally, from 80 wt% to 88 wt%, optionally from 81 wt% to 88 wt%, such as from 82 wt% to 88 wt% of the tablet core,
wherein the colesevelam or a pharmaceutically acceptable salt thereof is present in the **tablet core** in an amount of from about 700 mg to 1400 mg;
wherein the tablet core comprises a diluent, optionally the diluent is microcrystalline cellulose, optionally the diluent is a porous microcrystalline cellulose having an average particle size of in the range of from 80 to 110 µm and an angle of repose of less than about 40°, preferably, wherein the porous microcrystalline cellulose has an average particle size of about 90 µm and an angle of repose of about 34°, wherein the diluent is present in an amount of from 8 to 18 wt% of the tablet core;
wherein the tablet core comprises a binder present in an amount of from 2 to 6 wt% of the tablet core, optionally, wherein the binder is copovidone;
wherein the tablet core comprises a glidant, present in an amount of from 0.5 to 1.5 wt% of the tablet core, optionally, wherein the glidant is a silica, such as colloidal silicon dioxide;
wherein the tablet core has a hardness in the range of from 70 N to 450 N, optionally, from 80 N to 400 N, further optionally, from 90 N to 370 N, such as from 100 N to 330 N as determined in accordance with European Pharmacopoeia 2.9.8 or with US Pharmacopoeia monograph <1217>; and wherein the tablet core has a friability of from 0.001% to 1%, optionally, from 0.01 to 0.5%, optionally from 0.01 to 0.1% as determined in accordance with US Pharmacopoeia monograph <1216> or with European Pharmacopoeia monograph 2.9.7.

By way of further example, disclosed herein is a unit dose pharmaceutical composition for oral administration comprising a tablet core and a gastro-resistant coating covering the tablet core, wherein the tablet core comprises colesevelam hydrochloride,
wherein the colesevelam hydrochloride is present in an amount of from 76 wt% to 88 wt% of the tablet core, optionally, from 80 wt% to 88 wt%, optionally from 81 wt% to 88 wt%, such as from 82 wt% to 88 wt% of the tablet core,
wherein the colesevelam hydrochloride is present in the **tablet core** in an amount of from about 700 mg to 1400 mg;
wherein the tablet core comprises a diluent, optionally the diluent is microcrystalline cellulose, optionally the diluent is a porous microcrystalline cellulose having an average particle size of in the range of from 80 to 110 µm and an angle of repose of less than about 40°, preferably, wherein the porous microcrystalline cellulose has an average particle size of about 90 µm and an angle of repose of about 34°, wherein the diluent is present in an amount of from 8 to 18 wt% of the tablet core;
wherein the tablet core comprises a binder present in an amount of from 2 to 6 wt% of the tablet core, optionally, wherein the binder is copovidone;
wherein the tablet core comprises a glidant, present in an amount of from 0.5 to 1.5 wt% of the tablet core, optionally, wherein the glidant is a silica, such as colloidal silicon dioxide;
wherein the tablet core has a hardness in the range of from 70 N to 450 N, optionally, from 80 N to 400 N, further optionally, from 90 N to 370 N, such as from 100 N to 330 N as determined in accordance with European Pharmacopoeia 2.9.8 or with US Pharmacopoeia monograph <1217>; and wherein the tablet core has a friability of from 0.001% to 1%, optionally, from 0.01 to 0.5%, optionally from 0.01 to 0.1% as determined in accordance with US Pharmacopoeia monograph <1216> or with European Pharmacopoeia monograph 2.9.7.

Suitably, disclosed herein is a unit dose pharmaceutical composition for oral administration comprising a tablet core and a gastro-resistant coating covering the tablet core, wherein the tablet core comprises colesevelam or a pharmaceutically acceptable salt thereof,
wherein the colesevelam or a pharmaceutically acceptable salt thereof is present in an amount of from 76 wt% to 88 wt% of the tablet core, optionally, from 80 wt% to 88 wt%, optionally from 81 wt% to 88 wt%, such as from 82 wt% to 88 wt% of the tablet core,
wherein the colesevelam or a pharmaceutically acceptable salt thereof is present in the **tablet core** in an amount of from about 700 mg to 1400 mg;
wherein the tablet core comprises a diluent, optionally the diluent is microcrystalline cellulose, optionally the diluent is a porous microcrystalline cellulose having an average particle size of in the range of from 80 to 110 µm and an angle of repose of less than about 40°, preferably, wherein the porous microcrystalline cellulose has an average particle size of about 90 µm and an angle of repose of about 34°, wherein the diluent is present in an amount of from 8 to 18 wt% of the tablet core;
wherein the tablet core comprises a binder present in an amount of from 2 to 6 wt% of the tablet core, optionally, wherein the binder is copovidone;
wherein the tablet core comprises a glidant, present in an amount of from 0.5 to 1.5 wt% of the tablet core, optionally, wherein the glidant is a silica, such as colloidal silicon dioxide
wherein the tablet core has a hardness in the range of from 100 N to 330 N as determined in accordance with European Pharmacopoeia 2.9.8 or with US Pharmacopoeia monograph <1217>; and wherein the tablet core has a friability of 0.01 to 0.1% as determined in accordance with US Pharmacopoeia monograph <1216> or with European Pharmacopoeia monograph 2.9.7.

Still further, disclosed herein is a unit dose pharmaceutical composition for oral administration comprising a tablet core and a gastro-resistant coating covering the tablet core, wherein the tablet core comprises colesevelam hydrochloride,
wherein the colesevelam hydrochloride is present in an amount of from 76 wt% to 88 wt% of the tablet core, optionally, from 80 wt% to 88 wt%, optionally from 81 wt% to 88 wt%, such as from 82 wt% to 88 wt% of the tablet core,
wherein the colesevelam hydrochloride is present in the tablet core in an amount of from about 700 mg to 1400 mg;
wherein the tablet core comprises a diluent, optionally the diluent is microcrystalline cellulose, optionally the diluent is a porous microcrystalline cellulose having an average particle size of in the range of from 80 to 110 µm and an angle of repose of less than about 40°, preferably, wherein the porous microcrystalline cellulose has an average particle size of about 90 µm and an angle of repose of about 34°, wherein the diluent is present in an amount of from 8 to 18 wt% of the tablet core;
wherein the tablet core comprises a binder present in an amount of from 2 to 6 wt% of the tablet core, optionally, wherein the binder is copovidone;
wherein the tablet core comprises a glidant, present in an amount of from 0.5 to 1.5 wt% of the tablet core, optionally, wherein the glidant is a silica, such as colloidal silicon dioxide
wherein the tablet core has a hardness in the range of from 100 N to 330 N as determined in accordance with European Pharmacopoeia 2.9.8 or with US Pharmacopoeia monograph <1217>; and wherein the tablet core has a friability of 0.01 to 0.1% as determined in accordance with US Pharmacopoeia monograph <1216> or with European Pharmacopoeia monograph 2.9.7.

The present invention further provides a unit dose pharmaceutical composition for oral administration comprising a tablet core and a gastro-resistant coating covering the tablet core, wherein the tablet core comprises colesevelam or a pharmaceutically acceptable salt thereof,
wherein the colesevelam or a pharmaceutically acceptable salt thereof is present in an amount of from 76 wt% to 88 wt% of the tablet core, optionally, from 80 wt% to 88 wt%, optionally from 81 wt% to 88 wt%, such as from 82 wt% to 88 wt% of the tablet core,
wherein the colesevelam or a pharmaceutically acceptable salt thereof is present in the **tablet core** in an amount of from about 700 mg to 1400 mg;
wherein the tablet core comprises a diluent, optionally the diluent is microcrystalline cellulose, optionally the diluent is a porous microcrystalline cellulose having an average particle size of in the range of from 80 to 110 µm and an angle of repose of less than about 40°, preferably, wherein the porous microcrystalline cellulose has an average particle size of about 90 µm and an angle of repose of about 34°, wherein the diluent is present in an amount of from 8 to 18 wt% of the tablet core;
wherein the tablet core comprises a binder, present in an amount of from 2 to 6 wt% of the tablet core, optionally, wherein the binder is copovidone;
wherein the tablet core comprises a glidant, present in an amount of from 0.5 to 1.5 wt% of the tablet core, optionally, wherein the glidant is a silica, such as colloidal silicon dioxide; and
wherein the tablet core comprises a lubricant in an amount of from 0.5 to 1.5 wt% of the tablet core, optionally, wherein the lubricant is magnesium stearate.

The present invention still further provides a unit dose pharmaceutical composition for oral administration comprising a tablet core and a gastro-resistant coating covering the tablet core, wherein the tablet core comprises colesevelam hydrochloride,
wherein the colesevelam hydrochloride is present in an amount of from 76 wt% to 88 wt% of the tablet core, optionally, from 80 wt% to 88 wt%, optionally from 81 wt% to 88 wt%, such as from 82 wt% to 88 wt% of the tablet core,
wherein the colesevelam hydrochloride is present in the tablet core in an amount of from about 700 mg to 1400 mg;
wherein the tablet core comprises a diluent, optionally the diluent is microcrystalline cellulose, optionally the diluent is a porous microcrystalline cellulose having an average particle size of in the range of from 80 to 110 µm and an angle of repose of less than about 40°, preferably, wherein the porous microcrystalline cellulose has an average particle size of about 90 µm and an angle of repose of about 34°, wherein the diluent is present in an amount of from 8 to 18 wt% of the tablet core;
wherein the tablet core comprises a binder, present in an amount of from 2 to 6 wt% of the tablet core, optionally, wherein the binder is copovidone;
wherein the tablet core comprises a glidant, present in an amount of from 0.5 to 1.5 wt% of the tablet core, optionally, wherein the glidant is a silica, such as colloidal silicon dioxide; and
wherein the tablet core comprises a lubricant in an amount of from 0.5 to 1.5 wt% of the tablet core, optionally, wherein the lubricant is magnesium stearate.

For example, disclosed herein is a unit dose pharmaceutical composition for oral administration comprising a tablet core and a gastro-resistant coating covering the tablet core, wherein the tablet core comprises colesevelam or a pharmaceutically acceptable salt thereof,
wherein the colesevelam or a pharmaceutically acceptable salt thereof is present in an amount of from 76 wt% to 88 wt% of the tablet core, optionally, from 80 wt% to 88 wt%, optionally from 81 wt% to 88 wt%, such as from 82 wt% to 88 wt% of the tablet core,
wherein the colesevelam or a pharmaceutically acceptable salt thereof is present in the **tablet core** in an amount of from about 700 mg to 1400 mg;
wherein the tablet core comprises a diluent, optionally the diluent is microcrystalline cellulose, optionally the diluent is a porous microcrystalline cellulose having an average particle size of in the range of from 80 to 110 µm and an angle of repose of less than about 40°, preferably, wherein the porous microcrystalline cellulose has an average particle size of about 90 µm and an angle of repose of about 34°, wherein the diluent is present in an amount of from 8 to 18 wt% of the tablet core;
wherein the tablet core comprises a binder present in an amount of from 2 to 6 wt% of the tablet core, optionally, wherein the binder is copovidone;
wherein the tablet core comprises a glidant, present in an amount of from 0.5 to 1.5 wt% of the tablet core, optionally, wherein the glidant is a silica, such as colloidal silicon dioxide;
wherein the tablet core has a hardness in the range of from 70 N to 450 N, optionally, from 80 N to 400 N, further optionally, from 90 N to 370 N, such as from 100 N to 330 N as determined in accordance with European Pharmacopoeia 2.9.8 or with US Pharmacopoeia monograph <1217>; and wherein the tablet core has a friability of from 0.001% to 1%, optionally, from 0.01 to 0.5%, optionally from 0.01 to 0.1% as determined in accordance with US Pharmacopoeia monograph <1216> or with European Pharmacopoeia monograph 2.9.7.

By way of further example, disclosed herein is a unit dose pharmaceutical composition for oral administration comprising a tablet core and a gastro-resistant coating covering the tablet core, wherein the tablet core comprises colesevelam hydrochloride,
wherein the colesevelam hydrochloride is present in an amount of from 76 wt% to 88 wt% of the tablet core, optionally, from 80 wt% to 88 wt%, optionally from 81 wt% to 88 wt%, such as from 82 wt% to 88 wt% of the tablet core,
wherein the colesevelam hydrochloride is present in the tablet core in an amount of from about 700 mg to 1400 mg;
wherein the tablet core comprises a diluent, optionally the diluent is microcrystalline cellulose, optionally the diluent is a porous microcrystalline cellulose having an average particle size of in the range of from 80 to 110 µm and an angle of repose of less than about 40°, preferably, wherein the porous microcrystalline cellulose has an average particle size of about 90 µm and an angle of repose of about 34°, wherein the diluent is present in an amount of from 8 to 18 wt% of the tablet core;
wherein the tablet core comprises a binder present in an amount of from 2 to 6 wt% of the tablet core, optionally, wherein the binder is copovidone;
wherein the tablet core comprises a glidant, present in an amount of from 0.5 to 1.5 wt% of the tablet core, optionally, wherein the glidant is a silica, such as colloidal silicon dioxide;
wherein the tablet core has a hardness in the range of from 70 N to 450 N, optionally, from 80 N to 400 N, further optionally, from 90 N to 370 N, such as from 100 N to 330 N as determined in accordance with European Pharmacopoeia 2.9.8 or with US Pharmacopoeia monograph <1217>; and wherein the tablet core has a friability of from 0.001% to 1%, optionally, from 0.01 to 0.5%, optionally from 0.01 to 0.1% as determined in accordance with US Pharmacopoeia monograph <1216> or with European Pharmacopoeia monograph 2.9.7.

Suitably, disclosed herein is a unit dose pharmaceutical composition for oral administration comprising a tablet core and a gastro-resistant coating covering the tablet core, wherein the tablet core comprises colesevelam or a pharmaceutically acceptable salt thereof,
wherein the colesevelam or a pharmaceutically acceptable salt thereof is present in an amount of from 76 wt% to 88 wt% of the tablet core, optionally, from 80 wt% to 88 wt%, optionally from 81 wt% to 88 wt%, such as from 82 wt% to 88 wt% of the tablet core,
wherein the colesevelam or a pharmaceutically acceptable salt thereof is present in the **tablet core** in an amount of from about 700 mg to 1400 mg;
wherein the tablet core comprises a diluent, optionally the diluent is microcrystalline cellulose, optionally the diluent is a porous microcrystalline cellulose having an average particle size of in the range of from 80 to 110 µm and an angle of repose of less than about 40°, preferably, wherein the porous microcrystalline cellulose has an average particle size of about 90 µm and an angle of repose of about 34°, wherein the diluent is present in an amount of from 8 to 18 wt% of the tablet core;
wherein the tablet core comprises a binder present in an amount of from 2 to 6 wt% of the tablet core, optionally, wherein the binder is copovidone;
wherein the tablet core comprises a glidant, present in an amount of from 0.5 to 1.5 wt% of the tablet core, optionally, wherein the glidant is a silica, such as colloidal silicon dioxide
wherein the tablet core has a hardness in the range of from 100 N to 330 N as determined in accordance with European Pharmacopoeia 2.9.8 or with US Pharmacopoeia monograph <1217>; and wherein the tablet core has a friability of 0.01 to 0.1% as determined in accordance with US Pharmacopoeia monograph <1216> or with European Pharmacopoeia monograph 2.9.7.

Still further suitably disclosed herein is a unit dose pharmaceutical composition for oral administration comprising a tablet core and a gastro-resistant coating covering the tablet core, wherein the tablet core comprises colesevelam hydrochloride,
wherein the colesevelam hydrochloride is present in an amount of from 76 wt% to 88 wt% of the tablet core, optionally, from 80 wt% to 88 wt%, optionally from 81 wt% to 88 wt%, such as from 82 wt% to 88 wt% of the tablet core,
wherein the colesevelam hydrochloride is present in the tablet core in an amount of from about 700 mg to 1400 mg;
wherein the tablet core comprises a diluent, optionally the diluent is microcrystalline cellulose, optionally the diluent is a porous microcrystalline cellulose having an average particle size of in the range of from 80 to 110 µm and an angle of repose of less than about 40°, preferably, wherein the porous microcrystalline cellulose has an average particle size of about 90 µm and an angle of repose of about 34°, wherein the diluent is present in an amount of from 8 to 18 wt% of the tablet core;
wherein the tablet core comprises a binder present in an amount of from 2 to 6 wt% of the tablet core, optionally, wherein the binder is copovidone;
wherein the tablet core comprises a glidant, present in an amount of from 0.5 to 1.5 wt% of the tablet core, optionally, wherein the glidant is a silica, such as colloidal silicon dioxide
wherein the tablet core has a hardness in the range of from 100 N to 330 N as determined in accordance with European Pharmacopoeia 2.9.8 or with US Pharmacopoeia monograph <1217>; and wherein the tablet core has a friability of 0.01 to 0.1% as determined in accordance with US Pharmacopoeia monograph <1216> or with European Pharmacopoeia monograph 2.9.7.

The method of manufacture may further comprise the steps of:
(i) combining colesevelam and/or pharmaceutically acceptable salts thereof, with a diluent, a binder, a glidant, a lubricant and optionally a disintegrant to form a mixture;
(ii) tableting the mixture of step (i) to form tablet cores; and
(iii) coating the tablet cores formed in step (ii) with a gastro-resistant coating, and optionally a sub-coating.

The method of manufacture may further comprise bulk manufacture of a plurality of unit dose pharmaceutical compositions for oral administration, wherein the plurality of unit doses has a uniformity of mass of less than ±3%, preferably less than ±2%, more preferably less than ±1.7%. Uniformity of mass is measured according to European Pharmacopoeia 10.0, section 2.9.5.

There is also provided a method of manufacturing a unit dose pharmaceutical composition for oral administration comprising coating a tablet core with a gastro-resistant coating, and optionally a subcoating, wherein the colesevelam or a pharmaceutically acceptable salt thereof is at least 66 wt% of the tablet core, wherein the colesevelam or a pharmaceutically acceptable salt thereof is present in the **tablet core** in an amount of from about 700 mg to about 1400 mg.

The obtained tablets are tested in a dissolution test USP <711> apparatus II, 0.1N HCl for 2 hours and then 8 hours at pH 6.8 (phosphate buffer). To the phosphate buffer (optionally 1000 mL of said buffer) having pH of 6.8, a fixed amount of sodium tauro-deoxycholate is added. Coated tablets remain intact for 2 hours in 0.1 N HCl; thereafter, the tablets are withdrawn from the above medium and, after draining, are placed in the 6.8 medium. The coating starts to dissolve, the colesevelam starts to swell and binds the tauro-deoxycholate in the medium.

The dissolution of Colesevelam over time is calculated by the difference on the residual amount of free, non-bound tauro-deoxycholate in the medium

Suitably, the oral pharmaceutical compositions disclosed herein releases not more than about 50% of the amount of colesevelam, or a pharmaceutically acceptable salt thereof, when the pharmaceutical composition is placed in a United States Pharmacopeia (USP) dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 2 hours, wherein the dissolution apparatus comprises an aqueous phosphate buffer (optionally 1000 mL of said buffer) at pH 6.8 and at a temperature of 37 ± 0.5 °C, and wherein the aqueous solution is stirred at a rate of 100 rpm.

Suitably, any of the oral pharmaceutical compositions disclosed herein, may release not more than about 45% of the amount of colesevelam, or a pharmaceutically acceptable salt thereof, when the pharmaceutical composition is placed in a USP dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 2 hours, wherein the dissolution apparatus comprises an aqueous phosphate buffer (optionally 1000 mL of said buffer) at pH 6.8 and at a temperature of 37 ± 0.5 °C, and wherein the aqueous solution is stirred at a rate of 100 rpm.

Any of the oral pharmaceutical compositions disclosed herein may release not more than about 40% of the amount of colesevelam, or a pharmaceutically acceptable salt thereof, when the pharmaceutical composition is placed in a USP dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 2 hours, wherein the dissolution apparatus comprises an aqueous phosphate buffer (optionally 1000 mL of said buffer) at pH 6.8 and at a temperature of 37 ± 0.5 °C, and wherein the aqueous solution is stirred at a rate of 100 rpm.

Suitably, the oral pharmaceutical compositions disclosed herein releases not more than about 35% of the amount of colesevelam, or a pharmaceutically acceptable salt thereof, when the pharmaceutical composition is placed in a United States Pharmacopeia (USP) dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 2 hours, wherein the dissolution apparatus comprises an aqueous phosphate buffer (optionally 1000 mL of said buffer) at pH 6.8 and at a temperature of 37 ± 0.5 °C, and wherein the aqueous solution is stirred at a rate of 100 rpm.

Suitably, the oral pharmaceutical compositions disclosed herein releases not more than about 30% of the amount of colesevelam, or a pharmaceutically acceptable salt thereof, when the pharmaceutical composition is placed in a United States Pharmacopeia (USP) dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 2 hours, wherein the dissolution apparatus comprises an aqueous phosphate buffer (optionally 1000 mL of said buffer) at pH 6.8 and at a temperature of 37 ± 0.5 °C, and wherein the aqueous solution is stirred at a rate of 100 rpm.

Suitably, any of the oral pharmaceutical compositions disclosed herein, may release not more than about 25% of the amount of colesevelam, or a pharmaceutically acceptable salt thereof, when the pharmaceutical composition is placed in a USP dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 2 hours, wherein the dissolution apparatus comprises an aqueous phosphate buffer (optionally 1000 mL of said buffer) at pH 6.8 and at a temperature of 37 ± 0.5 °C, and wherein the aqueous solution is stirred at a rate of 100 rpm.

Any of the oral pharmaceutical compositions disclosed herein may release not more than about 20% of the amount of colesevelam, or a pharmaceutically acceptable salt thereof, when the pharmaceutical composition is placed in a USP dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 2 hours, wherein the dissolution apparatus comprises an aqueous phosphate buffer (optionally 1000 mL of said buffer) at pH 6.8 and at a temperature of 37 ± 0.5 °C, and wherein the aqueous solution is stirred at a rate of 100 rpm.

Suitably, the oral pharmaceutical compositions disclosed herein releases not more than about 50% of the amount of colesevelam, or a pharmaceutically acceptable salt thereof, when the pharmaceutical composition is placed in a United States Pharmacopeia (USP) dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 3 hours, wherein the dissolution apparatus comprises an aqueous phosphate buffer (optionally 1000 mL of said buffer) at pH 6.8 and at a temperature of 37 ± 0.5 °C, and wherein the aqueous solution is stirred at a rate of 100 rpm.

Suitably, any of the oral pharmaceutical compositions disclosed herein, may release not more than about 45% of the amount of colesevelam, or a pharmaceutically acceptable salt thereof, when the pharmaceutical composition is placed in a USP dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 3 hours, wherein the dissolution apparatus comprises an aqueous phosphate buffer (optionally 1000 mL of said buffer) at pH 6.8 and at a temperature of 37 ± 0.5 °C, and wherein the aqueous solution is stirred at a rate of 100 rpm.

Any of the oral pharmaceutical compositions disclosed herein may release not more than about 40% of the amount of colesevelam, or a pharmaceutically acceptable salt thereof, when the pharmaceutical composition is placed in a USP dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 3 hours, wherein the dissolution apparatus comprises an aqueous phosphate buffer (optionally 1000 mL of said buffer) at pH 6.8 and at a temperature of 37 ± 0.5 °C, and wherein the aqueous solution is stirred at a rate of 100 rpm.

Suitably, the oral pharmaceutical compositions disclosed herein releases not more than about 35% of the amount of colesevelam, or a pharmaceutically acceptable salt thereof, when the pharmaceutical composition is placed in a United States Pharmacopeia (USP) dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 3 hours, wherein the dissolution apparatus comprises an aqueous phosphate buffer (optionally 1000 mL of said buffer) at pH 6.8 and at a temperature of 37 ± 0.5 °C, and wherein the aqueous solution is stirred at a rate of 100 rpm.

Suitably, the oral pharmaceutical compositions disclosed herein releases not more than about 30% of the amount of colesevelam, or a pharmaceutically acceptable salt thereof, when the pharmaceutical composition is placed in a United States Pharmacopeia (USP) dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 3 hours, wherein the dissolution apparatus comprises an aqueous phosphate buffer (optionally 1000 mL of said buffer) at pH 6.8 and at a temperature of 37 ± 0.5 °C, and wherein the aqueous solution is stirred at a rate of 100 rpm.

Suitably, any of the oral pharmaceutical compositions disclosed herein, may release not more than about 25% of the amount of colesevelam, or a pharmaceutically acceptable salt thereof, when the pharmaceutical composition is placed in a USP dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 3 hours, wherein the dissolution apparatus comprises an aqueous phosphate buffer (optionally 1000 mL of said buffer) at pH 6.8 and at a temperature of 37 ± 0.5 °C, and wherein the aqueous solution is stirred at a rate of 100 rpm.

Any of the oral pharmaceutical compositions disclosed herein may release not more than about 20% of the amount of colesevelam, or a pharmaceutically acceptable salt thereof, when the pharmaceutical composition is placed in a USP dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 3 hours, wherein the dissolution apparatus comprises an aqueous phosphate buffer (optionally 1000 mL of said buffer) at pH 6.8 and at a temperature of 37 ± 0.5 °C, and wherein the aqueous solution is stirred at a rate of 100 rpm.

Suitably, the oral pharmaceutical compositions disclosed herein releases not more than about 50% of the amount of colesevelam, or a pharmaceutically acceptable salt thereof, when the pharmaceutical composition is placed in a United States Pharmacopeia (USP) dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 4 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Suitably, the oral pharmaceutical compositions disclosed herein releases not more than about 45% of the amount of colesevelam, or a pharmaceutically acceptable salt thereof, when the pharmaceutical composition is placed in a United States Pharmacopeia (USP) dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 4 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Suitably, the oral pharmaceutical compositions disclosed herein releases not more than about 40% of the amount of colesevelam, or a pharmaceutically acceptable salt thereof, when the pharmaceutical composition is placed in a United States Pharmacopeia (USP) dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 4 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Suitably, the oral pharmaceutical compositions disclosed herein releases not more than about 35% of the amount of colesevelam, or a pharmaceutically acceptable salt thereof, when the pharmaceutical composition is placed in a United States Pharmacopeia (USP) dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 4 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Suitably, the oral pharmaceutical compositions disclosed herein releases not more than about 30% of the amount of colesevelam, or a pharmaceutically acceptable salt thereof, when the pharmaceutical composition is placed in a United States Pharmacopeia (USP) dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 4 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Suitably, any of the oral pharmaceutical compositions disclosed herein, may release not more than about 25% of the amount of colesevelam, or a pharmaceutically acceptable salt thereof, when the pharmaceutical composition is placed in a USP dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 4 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Suitably, any of the oral pharmaceutical compositions disclosed herein, may release not more than about 20% of the amount of colesevelam, or a pharmaceutically acceptable salt thereof, when the pharmaceutical composition is placed in a USP dissolution apparatus II complying with USP <711> for 1 hour, and wherein the pharmaceutical composition releases not less than about 80% of the amount of colesevelam, or a pharmaceutically acceptable salt thereof, when the pharmaceutical composition is placed in a USP dissolution apparatus II complying with USP <711> for 4 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.

Optionally, the pharmaceutical composition releases not more than about 50% of the amount of colesevelam, or a pharmaceutically acceptable salt thereof, when the pharmaceutical composition is placed in a USP dissolution apparatus II complying with USP <711> for 1 hour, wherein the dissolution apparatus comprises an aqueous phosphate buffer (optionally 1000 mL of said buffer) at pH 6.8 and at a temperature of 37 ± 0.5 °C, and wherein the aqueous solution is stirred at a rate of 100 rpm.

Optionally, the pharmaceutical composition releases not more than about 45% of the amount of colesevelam, or a pharmaceutically acceptable salt thereof, when the pharmaceutical composition is placed in a USP dissolution apparatus II complying with USP <711> for 1 hour, wherein the dissolution apparatus comprises an aqueous phosphate buffer (optionally 1000 mL of said buffer) at pH 6.8 and at a temperature of 37 ± 0.5 °C, and wherein the aqueous solution is stirred at a rate of 100 rpm.

Optionally, the pharmaceutical composition releases not more than about 40% of the amount of colesevelam, or a pharmaceutically acceptable salt thereof, when the pharmaceutical composition is placed in a USP dissolution apparatus II complying with USP <711> for 1 hour, wherein the dissolution apparatus comprises an aqueous phosphate buffer (optionally 1000 mL of said buffer) at pH 6.8 and at a temperature of 37 ± 0.5 °C, and wherein the aqueous solution is stirred at a rate of 100 rpm.

Optionally, the pharmaceutical composition releases not more than about 35% of the amount of colesevelam, or a pharmaceutically acceptable salt thereof, when the pharmaceutical composition is placed in a USP dissolution apparatus II complying with USP <711> for 1 hour, wherein the dissolution apparatus comprises an aqueous phosphate buffer (optionally 1000 mL of said buffer) at pH 6.8 and at a temperature of 37 ± 0.5 °C, and wherein the aqueous solution is stirred at a rate of 100 rpm.

Optionally, the pharmaceutical composition releases not more than about 30% of the amount of colesevelam, or a pharmaceutically acceptable salt thereof, when the pharmaceutical composition is placed in a USP dissolution apparatus II complying with USP <711> for 1 hour, wherein the dissolution apparatus comprises an aqueous phosphate buffer (optionally 1000 mL of said buffer) at pH 6.8 and at a temperature of 37 ± 0.5 °C, and wherein the aqueous solution is stirred at a rate of 100 rpm.

Optionally, the pharmaceutical composition releases not more than about 25% of the amount of colesevelam, or a pharmaceutically acceptable salt thereof, when the pharmaceutical composition is placed in a USP dissolution apparatus II complying with USP <711> for 1 hour, wherein the dissolution apparatus comprises an aqueous phosphate buffer (optionally 1000 mL of said buffer) at pH 6.8 and at a temperature of 37 ± 0.5 °C, and wherein the aqueous solution is stirred at a rate of 100 rpm.

Optionally, the pharmaceutical composition releases not more than about 20% of the amount of colesevelam, or a pharmaceutically acceptable salt thereof, when the pharmaceutical composition is placed in a USP dissolution apparatus II complying with USP <711> for 1 hour, wherein the dissolution apparatus comprises an aqueous phosphate buffer (optionally 1000 mL of said buffer) at pH 6.8 and at a temperature of 37 ± 0.5 °C, and wherein the aqueous solution is stirred at a rate of 100 rpm.

Optionally, the pharmaceutical composition releases not less than about 80% of the amount of colesevelam, or a pharmaceutically acceptable salt thereof, when the pharmaceutical composition is placed in a USP dissolution apparatus II complying with USP <711> for 2 hours, wherein the dissolution apparatus comprises an aqueous phosphate buffer (optionally 1000 mL of said buffer) at pH 6.8 and at a temperature of 37 ± 0.5 °C, and wherein the aqueous solution is stirred at a rate of 100 rpm.

Optionally, the pharmaceutical composition releases not less than about 80% of the amount of colesevelam, or a pharmaceutically acceptable salt thereof, when the pharmaceutical composition is placed in a USP dissolution apparatus II complying with USP <711> for 3 hours, wherein the dissolution apparatus comprises an aqueous phosphate buffer (optionally 1000 mL of said buffer) at pH 6.8 and at a temperature of 37 ± 0.5 °C, and wherein the aqueous solution is stirred at a rate of 100 rpm.

Optionally, the pharmaceutical composition releases not less than about 80% of the amount of colesevelam, or a pharmaceutically acceptable salt thereof, when the pharmaceutical composition is placed in a USP dissolution apparatus II complying with USP <711> for 4 hours, wherein the dissolution apparatus comprises an aqueous phosphate buffer (optionally 1000 mL of said buffer) at pH 6.8 and at a temperature of 37 ± 0.5 °C, and wherein the aqueous solution is stirred at a rate of 100 rpm.

Optionally, the oral pharmaceutical compositions of the present invention have a dissolution rate lower than about 50% over about 1 hour, when measured at about pH 6.8.

Optionally, the oral pharmaceutical compositions of the present invention have a dissolution rate lower than about 45% over about 1 hour, when measured at about pH 6.8.

Optionally, the oral pharmaceutical compositions of the present invention have a dissolution rate lower than about 40% over about 1 hour, when measured at about pH 6.8.

Optionally, the oral pharmaceutical compositions of the present invention have a dissolution rate lower than about 35% over about 1 hour, when measured at about pH 6.8.

Optionally, the oral pharmaceutical compositions of the present invention have a dissolution rate lower than about 30% over about 1 hour, when measured at about pH 6.8.

Optionally, the oral pharmaceutical compositions of the present invention have a dissolution rate lower than about 25% over about 1 hour, when measured at about pH 6.8.

Optionally, the oral pharmaceutical compositions of the present invention have a dissolution rate lower than about 20% over about 1 hour, when measured at about pH 6.8.

Optionally, the oral pharmaceutical compositions of the present invention have a dissolution rate of greater than or equal to about 80% of colesevelam, or a pharmaceutically acceptable salt thereof, over about 2 hours, when measured at about pH 6.8.

Optionally, the oral pharmaceutical compositions of the present invention have a dissolution rate of greater than or equal to about 80% of colesevelam, or a pharmaceutically acceptable salt thereof, over about 3 hours, when measured at about pH 6.8.

Optionally, the oral pharmaceutical compositions of the present invention have a dissolution rate of greater than or equal to about 80% of colesevelam, or a pharmaceutically acceptable salt thereof, over about 4 hours, when measured at about pH 6.8.

The method of manufacture can be employed to provide a unit dose pharmaceutical composition for oral administration according to the present invention.

There is also provided the use of a pharmaceutical composition as described herein in the manufacture of a medicament for treating bile acid diarrhea, irritable bowel syndrome, optionally, irritable bowel syndrome subtype diarrhea (IBS-D), colitis, such as microscopic colitis, and/or bile acid malabsorption.

The pharmaceutical compositions of the present invention can be used in the treatment of bile acid diarrhea. Tablets comprising colesevelam that are known in the art are typically administered either in a single, non-fractionated administration (e.g., six tablets per day) or in multiple fractionated administrations, (e.g., three tablets twice per day, or two tablets thrice per day).

Given the chronicity of bile acid diarrhea, and the high number of tablets required for treatment per day, patient compliance issues often occur when treating with colesevelam. The present invention allows for a reduction in the number of tablets and frequency of administration per day (such as, for example, two tablets twice per day, or one tablet twice per day), which leads to an improvement in patient compliance.

The pharmaceutical compositions of the present invention can be used in the treatment of bile acid diarrhea. This bile acid diarrhea may be idiopathic (e.g., primary bile acid diarrhea) or secondary to an underlying disease. The underlying disease can be: Crohn's disease, ileal resection or radiation ileitis, chronic pancreatitis, celiac disease, cholecystectomy, small intestine bacterial overgrowth, irritable bowel syndrome subtype diarrhea (IBS-D) and similar.

The method of treating bile acid diarrhea comprises administering to a subject in need thereof, a therapeutically effective amount of a pharmaceutical composition described herein. This bile acid diarrhea may be idiopathic (e.g., primary bile acid diarrhea) or secondary to an underlying disease. The underlying disease can be: Crohn's disease, ileal resection or radiation ileitis, chronic pancreatitis, celiac disease, cholecystectomy, small intestine bacterial overgrowth, irritable bowel syndrome subtype diarrhea (IBS-D) and similar.

The pharmaceutical compositions of the present invention can be used in the treatment of irritable bowel syndrome subtype diarrhea (IBS-D).

The method of treating irritable bowel syndrome subtype diarrhea (IBS-D) comprises administering to a subject in need thereof, a therapeutically effective amount of a pharmaceutical composition described herein.

The pharmaceutical compositions of the present invention can be used in the treatment of microscopic colitis.

The method of treating microscopic colitis comprises administering to a subject in need thereof, a therapeutically effective amount of a pharmaceutical composition described herein.

The pharmaceutical compositions of the present invention can be used in the treatment of bile acid malabsorption.

The method of treating bile acid malabsorption comprises administering to a subject in need thereof, a therapeutically effective amount of a pharmaceutical composition described herein.

The pharmaceutical compositions of the present invention can be used in the treatment of bile acid diarrhea. Tablets comprising colesevelam that are known in the art are typically administered either in a single, non-fractionated administration (e.g., six tablets per day) or in multiple fractionated administrations, (e.g., three tablets twice per day, or two tablets thrice per day). Given the chronicity of the disease, and the high number of tablets required for treatment per day, patient compliance issues often occur when treating with colesevelam. The present invention allows for a reduction in the number of tablets and frequency of administration per day (such as, for example, two tablets twice per day, or one tablet twice per day), which leads to an improvement in patient compliance. The present invention allows to reduce the number of tablets and frequency of administration per day (e.g., two tablets twice per day, or one tablet twice per day), with an improvement in terms of patients' compliance.

The invention will be more readily appreciated by a review of the examples which follow.

### DEFINITIONS

As used herein the term "active pharmaceutical ingredient" ("API") or "pharmaceutically active agent" is a drug or agent which can be employed for the compositions and methods of the disclosure and is intended to be used in the human or animal body in order to heal, to alleviate, to prevent or to diagnose diseases, ailments, physical damage or pathological symptoms; allow the state, the condition or the functions of the body or mental states to be identified; to replace active substances produced by the human or animal body, or body fluids; to defend against, to eliminate or to render innocuous pathogens, parasites or exogenous substances or to influence the state, the condition or the functions of the body or mental states. Drugs in use can be found in reference works such as, for example, the Rote Liste or the Merck Index. Examples which may be mentioned include, for example, colesevelam.

As used herein, "pharmaceutically acceptable salts" refer to derivatives of the disclosed compounds wherein the therapeutic compound is modified by making acid or base salts thereof. Examples of pharmaceutically acceptable salts include, but are not limited to, mineral or organic acid salts of the active agent. The pharmaceutically acceptable salts include the conventional non-toxic salts, for example, from non-toxic inorganic or organic acids. For example, such conventional non-toxic salts include those derived from inorganic acids such as hydrochloric, hydrobromic, sulfuric, sulfonic, sulfamic, phosphoric, nitric and the like; and the salts prepared from organic acids such as amino acids, acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, isethionic, and other known to those of ordinary skill in the pharmaceutical sciences. Lists of suitable salts are found in texts such as Remington 's Pharmaceutical Sciences, 18th Ed. (Alfonso R. Gennaro, ed.; Mack Publishing Company, Easton, Pa., 1990); Remington: the Science and Practice of Pharmacy 19th Ed. (Lippincott, Williams & Wilkins, 1995); Handbook of Pharmaceutical Excipients, 3K| Ed. (Arthur H. Kibbe, ed.; Amer. Pharmaceutical Assoc., 1999); the Pharmaceutical Codex: Principles and Practice of Pharmaceutics 12th Ed. (Walter Lund ed.; Pharmaceutical Press, London, 1994); The United States Pharmacopeia: The National Formulary (United States Pharmacopeial Convention); and Goodman and Gilman's: the Pharmacological Basis of Therapeutics (Louis S. Goodman and Lee E. Limbird, eds.; McGraw Hill, 1992), the disclosures of which are hereby incorporated by reference.

An amount is "effective" as used herein, when the amount provides an effect in the subject. As used herein, the term "effective amount" means an amount of a compound or composition sufficient to significantly induce a positive benefit, including independently or in combinations the benefits disclosed herein, but low enough to avoid serious side effects, i.e., to provide a reasonable benefit to risk ratio, within the scope of sound judgment of the skilled artisan. For those skilled in the art, the effective amount, as well as dosage and frequency of administration, may be determined according to their knowledge and standard methodology of merely routine experimentation based on the present disclosure.

As used herein, the terms "subject" and "patient" are used interchangeably. As used herein, the term "patient" refers to an animal, preferably a mammal such as a non-primate (e.g., cows, pigs, horses, cats, dogs, rats etc.) and a primate (e.g., monkey and human), and most preferably a human. In some embodiments, the subject is a non-human animal such as a farm animal (e.g., a horse, pig, or cow) or a pet (e.g., a dog or cat). In a specific embodiment, the subject is an elderly human. In another embodiment, the subject is a human adult. In another embodiment, the subject is a human child. In yet another embodiment, the subject is a human infant.

As used herein, the phrase "pharmaceutically acceptable" means approved by a regulatory agency of the federal or a state government, or listed in the U.S. Pharmacopeia, European Pharmacopeia, or other generally recognized pharmacopeia for use in animals, and more particularly, in humans.

As used herein, the terms "prevent," "preventing" and "prevention" in the context of the administration of a therapy to a subject refer to the prevention or inhibition of the recurrence, onset, and/or development of a disease or condition, or a combination of therapies (e.g., a combination of prophylactic or therapeutic agents).

As used herein, the terms "therapies" and "therapy" can refer to any method(s), composition(s), and/or agent(s) that can be used in the prevention, treatment and/or management of a disease or condition, or one or more symptoms thereof.

As used herein, the terms "treat," "treatment," and "treating" in the context of the administration of a therapy to a subject refer to the reduction or inhibition of the progression and/or duration of a disease or condition, the reduction or amelioration of the severity of a disease or condition, and/or the amelioration of one or more symptoms thereof resulting from the administration of one or more therapies.

As used herein, the term "about" when used in conjunction with a stated numerical value or range has the meaning reasonably ascribed to it by a person skilled in the art, i.e., denoting somewhat more or somewhat less than the stated value or range. For example, for each specified value "about" may be ±10%, suitably ±5%, suitably ±2%, suitably ±1%.

As used herein, the symbol "wt%" or "% w/w" indicates the weight percent of a substance in a mixture (e.g., a mixture of solid substances, or in a solution), which is calculated as the mass of the component divided by the total mass of the mixture and then multiplied by 100 to provide the percentage. Usually, mass is expressed in grams, but any unit of measurement is acceptable as long as the same units for both the component or solute mass and the total or solution mass are used in the calculation.

The words "comprises/comprising" and the words "having/including" when used herein with reference to the present invention are used to specify the presence of stated features, integers, steps or components but do not preclude the presence or addition of one or more other features, integers, steps, components or groups thereof.

Colesevelam hydrochloride is poly(allylamine hydrochloride) cross-linked with epichlorohydrin and alkylated with 1-bromodecane and (6-bromohexyl)- trimethylammonium bromide. The chemical name (IUPAC) of colesevelam hydrochloride is allylamine polymer with I-chloro-2, 3 -epoxypropane, [6-(allylamino)- hexyljtrimethylammonium chloride and N-allyldecylamine, hydrochloride. The chemical structure of colesevelam hydrochloride is represented by the following formula: 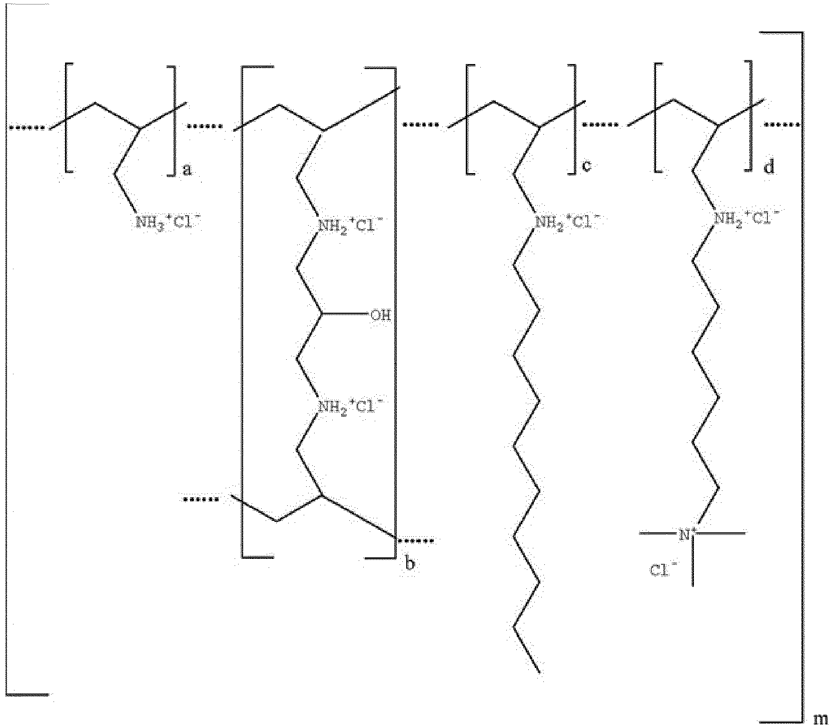 wherein (a) represents allyl amine monomer units that have not been alkylated by either of the 1-bromodecane or (6-bromohexyl)-trimethylammonium bromide alkylating agents or cross-linked by epichlorohydrin; (b) represents allyl amine units that have undergone cross-linking with epichlorohydrin; (c) represents allyl amine units that have been alkylated with a decyl group; (d) represents allyl amine units that have been alkylated with a (6-trimethylammonium) hexyl group, and m represents a number ≥ 100 to indicate an extended polymer network. A small amount of the amines are dialkylated and are not depicted in the formula above. No regular order of the groups is implied by the structure; cross-linking and alkylation are expected to occur randomly along the polymer chains. A large amount of the amines are protonated. The polymer is depicted in the hydrochloride form; a small amount of the halides are bromide. Colesevelam hydrochloride is hydrophilic and insoluble in water.

Preferably, the present invention employs colesevelam hydrochloride, however, the person skilled in the art will appreciate that alternative pharmaceutically acceptable salts, or the free base version of colesevelam may be employed in the present invention.

### Examples

Obtaining tablets with a very high content of active substance is difficult, in particular due to the physical properties of colesevelam. Colesevelam is a cross linked polymer which is insoluble in aqueous and organic solvents and has poor flowability and compressibility properties.

WO 2021/163007 discloses colesevelam hydrochloride formulation having the following composition:

| **Ingredient** | **Qty/mg** | **%w/w** |
|---|---|---|
| Colesevelam hydrochloride (expressed as anhydrous weight) | 625 | 63.13 |
| Microcrystalline cellulose (AVICEL^{™} PH-101) | 220 | 22.22 |
| Hydroxy propyl methylcellulose (HPMC E5LV) | 20 | 2.02 |
| Hydroxy propyl methylcellulose (K4M) | 100 | 10.10 |
| Colloidal silicon dioxide | 15 | 1.52 |
| Magnesium stearate | 10 | 1.01 |
| **Core tablet weight** | **990** | **100** |
| | | |

| | **Coating** | |
|---|---|---|
| Hydroxypropyl methyl cellulose (HPMC E50SLV) | 69.8 | 6.53 |
| Diacylated monoglyceride | 9.4 | 0.88 |
| **Coated tablet total weight** | **1069.2** | **100** |

Using the same components as described in WO 2021/163007 and similar weight percentage amounts, the present inventors assessed whether a higher dose formulation (i.e., a unit dose comprising more than 625 mg colesevelam hydrochloride (anhydrous)) could be manufactured.

### Comparative Example 1: Preparation of Compositions at higher loading than WO 2021/163007.

| | **Comparative Example 1** | |
|---|---|---|
| **Components** | **% w/w** | **Tablet (mg)** |
| **Colesevelam HCl anhydrous** | 63.13 | 900.00 |
| **Microcrystalline cellulose** | | |
| **(AVICEL^{™} PH-101)** | 22.22 | 316.80 |
| **Hydroxy propyl methyl cellulose E5LV** | 2.02 | 28.80 |
| **Hydroxy propyl methyl cellulose K4M** | 10.10 | 144.00 |
| **Colloidal silicon dioxide** | 1.52 | 21.60 |
| **Magnesium stearate** | 1.01 | 14.40 |
| **Total Cores** | 100.00 | 1425.60 |

The inventors attempted to follow the same manufacturing process as is described in WO 2021/163007 in order to manufacture tablet cores comprising 900 mg colesevelam HCl (anhydrous):
1. Colesevelam hydrochloride microcrystalline cellulose, HPMC ES LV(R), hydroxypropyl methyl cellulose (K4M(R) and Aerosil R-200 were sifted through #40 mesh and mixed for 10 minutes in a bin blender. Magnesium stearate was sifted through #60 mesh and added to the above blend and mixed for another 5 min.
2. The above lubricated blend was then compressed into tablets.
3. A coating solution was prepared by dissolving acetylated monoglyceride in purified water under stirring followed by addition of HPMC E50LV(R) into it, under stirring until a uniform dispersion formed.
4. The coating solution of 3) was sprayed onto the tablet core of 2) using a suitable coating machine to provide a weight gain between 6-4% w/w per tablet.

However, during tableting of the composition of Comparative Example 1 (i.e., during step 2 above), it was not possible to reach the target tablet weight.

The formulation of Comparative Example 1 could not be tableted at 900 mg strength using 22.5 - 10.5 mm punches. In fact, the maximum weight reached corresponded to 91.1% of theoretical amount of colesevelam HCl for each tablet.

The theoretical weight could only be achieved by increasing the punches dimension. In doing so, the tablet size would be increased which would negatively impact patient compliance. Furthermore, the resulting tablet had poor friability and crumbled during the hardness test.

In order to overcome the tableting issues, and in the hope of achieving a higher drug loading of colesevelam, the inventors investigated a plethora of different excipient formulations, and drug loadings.

### Comparative Example 2: Direct Compression of Colesevelam using alternative excipients

The use of sodium carboxymethyl starch, and poloxamer or xanthan gum was investigated in this example.

| | **Formulation A** | | **Formulation B** | |
|---|---|---|---|---|
| **Components** | **% w/w** | **Tablet (mg)** | **% w/w** | **Tablet (mg)** |
| **Colesevelam HCl anhydrous** | 78.00 | 900.00 | 78.00 | 900.00 |
| **Silicified Microcrystalline Cellulose** | 12.00 | 138.46 | 11.00 | 126.92 |
| **Sodium Carboxymethyl Starch** | 6.50 | 75.00 | 6.50 | 75.00 |
| **Poloxamer** | 3.50 | 40.38 | 0.00 | 0.00 |
| **Xanthan gum** | 0.00 | 0.00 | 3.50 | 40.38 |
| **Colloidal Silicon Dioxide** | 0.00 | 0.00 | 0.00 | 0.00 |
| **Magnesium Stearate** | 0.00 | 0.00 | 1.00 | 11.54 |
| **Total** | 100.00 | 1153.85 | 100.00 | 1153.85 |

In Comparative Example 2, a similar method of manufacture was employed as in Comparative Example 1. The components of each composition were sifted, sieved and blended prior to tabletting to form tablet cores. In each of the compositions (of formulations A and B), the powder mixture formed after combining the components flowed very poorly, and tablet cores could only be formed with mandatory use of forced loading. Though tablet cores could be formed by direct compression, the tablet cores formed were friable with damage at the edges. Thus, the tablet cores were unsuitable for bulk manufacture.

### Comparative Example 3: Characterization of tablets of Comparative Example 2

| | **Formulation A** | **Formulation B** |
|---|---|---|
| **Targeted Dose (mg)** | 900.0 | 900.0 |
| **Dose obtained (mg)** | 900.0 | 900.0 |
| **Hardness (N)** | 49.7 | 42.2 |
| **Thickness (mm)** | 9.40 | 9.25 |
| **Friability (%)** | Broken tablet during the test - test failed | Broken tablet during the test - test failed |

After investigating different manufacturing processes, as well as different amounts and combinations of various excipients, the inventors identified a combination of excipients that enables the formation of high loading colesevelam tablets, comprising over 700 mg colesevelam, preferably over 850 mg colesevelam, at loadings as high as 76%, 82% and 85%.

### Example 1

| | **Formulation C1** | |
|---|---|---|
| **Components** | **% w/w** | **Tablet (mg)** |
| **Colesevelam HCl anhydrous** | 76.91 | 900.00 |
| **Microcrystalline cellulose** | 15.67 | 183.37 |
| **Copovidone** | 5.30 | 62.00 |
| **Colloidal silicon dioxide** | 1.06 | 12.40 |
| **Magnesium stearate** | 1.06 | 12.40 |
| **Total Cores** | 100.00 | 1170.17 |

| | **Formulation C2** | |
|---|---|---|
| **Components** | **% w/w (considering coated tablet weight)** | **Tablet (mg)** |
| **Colesevelam HCl anhydrous** | 69.92 | 900.00 |
| **Microcrystalline cellulose** | 14.24 | 183.37 |
| **Copovidone** | 4.82 | 62.00 |
| **Colloidal silicon dioxide** | 0.96 | 12.40 |
| **Magnesium stearate** | 0.96 | 12.40 |
| **Total Cores** | 90.9 | 1170.17 |
| **Hydroxypropyl cellulose** | 2.74 | 37.20 |
| **Total sub-coated tablets** | 93.79 | 1207.37 |
| **Poly(methacrylic acid, methyl methacrylate) 1:1 (EUDRAGIT L100)** | 2.70 | 34.70 |
| **Poly(methacrylic acid, methyl methacrylate) 1:2 (EUDRAGIT S100)** | 1.35 | 17.35 |
| **Triethyl citrate** | 0.81 | 10.40 |
| **Talc** | 1.35 | 17.35 |

### Example 2: Characterization of Compositions of Example 1

### Test Methods Employed:

Tablet breaking force was measured according to USP <1217> and EP 2.9.8. Tablet friability was measured according to USP <1216> and EP 2.9.7. Uniformity of mass was measured according to EP 2.9.5.

| | **Formulation C1** |
|---|---|
| **Average mass (mg)** | 1241.4 |
| **Uniformity of mass** | Complies |
| | (min./max.: -1.6%/+1.1%) |
| **Tablet breaking force (N)** | 243 |
| | (min./max.: 211-268) |
| **Thickness (mm)** | 8.70 |
| | (min./max.: 8.61-8.79) |
| **Friability (%)** | < 0.1% |

### Method of Manufacture:

- Colesevelam hydrochloride, microcrystalline cellulose, copovidone and colloidal silicon dioxide were sifted through 20 mesh and mixed. Magnesium stearate was sieved through 30 mesh and mixed.
- The above blend was tabletted using 22.5x10.5 mm punches for 900 mg strength achieving target thickness of 8.5 mm, range 7.8mm - 9.2 mm.
- A sub-coating solution was prepared by dissolving hydroxypropyl cellulose in ethanol under stirring and this solution was sprayed onto the tablet core using a suitable coating machine.
- A coating dispersion was prepared by dissolving the functional coating excipients listed above in ethanol under stirring and this dispersion was sprayed onto the tablets using a suitable coating machine.

### Example 3: API Loading Evaluation in Formulation Development

| | **Formulation D : 82% drug loading (as anhydrous substance)** | | **Formulation E: 85% drug loading (as anhydrous substance)** | |
|---|---|---|---|---|
| **Components** | **% w/w** | **Tablet (mg)** | **% w/w** | **Tablet (mg)** |
| **Colesevelam HCl anhydrous** | 82.00 | 900.00 | 85.00 | 900.00 |
| **Microcrystalline cellulose** | 11.95 | 131.20 | 9.72 | 102.85 |
| **Copovidone** | 4.05 | 44.36 | 3.28 | 34.77 |
| **Colloidal silicon dioxide** | 1.00 | 11.00 | 1.00 | 10.60 |
| **Magnesium stearate** | 1.00 | 11.00 | 1.00 | 10.60 |
| **Total Cores** | 100.00 | 1097.56 | 100.00 | 1058.82 |

### Example 4: Characterization of Compositions of Example 3

| | **Formulation D : 82% drug loading (as anhydrous substance)** | **Formulation E: 85% drug loading (as anhydrous substance)** |
|---|---|---|
| **Bulk Density (g/ml)** | 0.467 | 0.466 |
| **Tapped Density (g/ml)** | 0.585 | 0.599 |
| **Carr's Index** | 20 | 22 |
| | Fair | Passable |

| | **Formulation D : 82% drug loading (as anhydrous substance)** | | **Formulation E: 85% drug loading (as anhydrous substance)** | | |
|---|---|---|---|---|---|
| | **Set 1** | **Set 2** | **Set 1** | **Set 2** | **Set 3** |
| **Average mass (mg)** | 1177.6 | 1162.6 | 1128.5 | 1124.4 | 1119.4 |
| **Uniformity of mass** | Complies (min./max.: - 0.4%/+0.4%) | Complies (min./max.: - 0.7%/+0.3%) | Complies (min./max.: - 0.3%/+0.4%) | Complies (min./max.: - 0.6%/+0.4%) | Complies (min./max.: - 0.7%/+0.7%) |
| **Tablet** | 197 | 261 | 158 | 251 | 306 |
| **breaking force (N)** | (min./max.: 193-203) | (min./max.: 246-270) | (min./max.: 149-168) | (min./max.: 237-270) | (min./max.: 272-337) |
| **Thickness (mm)** | 8.41 (min./max.: 8.39-8.44) | 8.14 (min./max.: 8.11-8.16) | 8.55 (min./max.: 8.51-8.60) | 8.26 (min./max.: 8.24-8.29) | 8.13 (min./max.: 8.11-8.17) |
| **Friability (%)** | < 0.1% | < 0.1% | <0.1% | < 0.1% | < 0.1% |

Advantageously, the present inventors have managed to formulate high drug loading colesevelam tablets that are sufficiently hard, and have low friability to permit manufacture thereof on an industrial scale (i.e., to permit bulk manufacture). The present inventors have identified combinations of excipients that permit manufacture of 900 mg colesevelam dose tablets that are suitable for oral administration. Advantageously, the tablets are not overly large (e.g., 22.5 mm x 10.5 mm and having a thickness of about 8.5 mm), thereby facilitating easier administration, with a consequent improvement in patient compliance in comparison to bulkier tablets.

A side by side comparison of the properties of the unit oral dosage forms of the present invention and that of Comparative Example 1 is provided below:

| | **Comparative Example 1** | | **Formulation C1** | |
|---|---|---|---|---|
| **Components** | **% w/w** | **Tablet (mg)** | **% w/w** | **Tablet (mg)** |
| **Colesevelam HCl (anhydrous)** | 63.13 | 900.00 | 76.91 | 900.00 |
| **Microcrystalline cellulose** | | | 15.67 | 183.37 |
| **Copovidone** | | | 5.30 | 62.00 |
| **Microcrystalline cellulose (AVICEL^{™} PH-101)** | 22.22 | 316.80 | | |
| **Hydroxy propyl methyl cellulose E5LV** | 2.02 | 28.80 | | |
| **Hydroxy propyl methyl cellulose K4M** | 10.10 | 144.00 | | |
| **Colloidal silicon** | 1.52 | 21.60 | 1.06 | 12.40 |
| **dioxide** | | | | |
| **Magnesium stearate** | 1.01 | 14.40 | 1.06 | 12.40 |
| **Total Cores** | 100.00 | 1425.60 | 100.00 | 1170.17 |

| | **Comparative Example 1** | **Formulation C1** |
|---|---|---|
| **Average mass (mg)** | - | 1241.4 |
| **Uniformity of mass** | - | Complies |
| | | (min./max.: -1.6%/+1.1%) |
| **Tablet breaking force (N)** | - | 243 |
| | | (min./max.: 211-268) |
| **Thickness (mm)** | - | 8.70 |
| | | (min./max.: 8.61-8.79) |
| **Friability (%)** | failed - crumbled | < 0.1% |

Unlike the unit oral dosage forms of the present invention, the composition of Comparative Example 1 crumbled when tabletting was attempted. The composition of Comparative Example 1 had inferior hardness, was friable and was not suitable for bulk manufacture in tablet form. A particularly surprising and satisfying aspect to the formulation of the unit oral dosage form of the present invention was that in bulk manufacture, the uniformity of mass was particularly good (less than 2%).

Providing tablets with certain advanced characteristics, such as a targeted and/or delayed release profile, is very difficult due to the very low content of excipients in the tablet, which give rise to the desired release properties.

### Embodiments of the present invention are described herein:

1. A **unit dose** pharmaceutical composition for oral administration comprising **a tablet core** and a gastro-resistant coating covering the tablet core,
   wherein the tablet core comprises colesevelam or a pharmaceutically acceptable salt thereof,
   wherein the colesevelam or a pharmaceutically acceptable salt thereof is at least 66 wt% of the tablet core, and
   wherein the colesevelam or a pharmaceutically acceptable salt thereof is present in the **tablet core** in an amount of from about 700 mg to 1400 mg.
2. The **unit dose** pharmaceutical composition according to embodiment 1, wherein the colesevelam or a pharmaceutically acceptable salt thereof is at least 70 wt%, or at least 71 wt%, or at least 75 wt%, or at least 76 wt%, or at least 80 wt%, or at least 81 wt% of the tablet core., optionally, at least 82 wt% of the tablet core.
3. The **unit dose** pharmaceutical composition of embodiment 1 or 2, wherein the tablet core further comprises one or more of a diluent, a binder, a disintegrant, a glidant or a lubricant, preferably wherein the tablet core comprises a diluent, a binder, a glidant and a lubricant, and optionally a disintegrant.
4. The **unit dose** pharmaceutical composition of embodiment 3, wherein the tablet core comprises a diluent or mixture of two or more diluents selected from the group comprising: lactose, sugars such as sucrose and fructose, dextrose, dextrates, dextrans, a polyol such as sorbitol, mannitol and xylitol, dibasic calcium phosphate, tribasic calcium phosphate, calcium sulphate, sodium chloride, starch, such as pregelatinized starch, cellulose or derivatives thereof, such as methyl, ethyl, or hydroxyethyl cellulose; optionally wherein the diluent is selected from cellulose or a derivative thereof, lactose, calcium phosphate, starch, modified starch and mannitol.
5. The **unit dose** pharmaceutical composition of embodiment 3 or 4, wherein the tablet core comprises a diluent, optionally the diluent is microcrystalline cellulose, optionally the diluent is a porous microcrystalline cellulose having an average particle size of in the range of from 80 to 110 µm and an angle of repose of less than about 40°, preferably, wherein the porous microcrystalline cellulose has an average particle size of about 90 µm and an angle of repose of about 34°.
6. The **unit dose** pharmaceutical composition of any one of embodiments 3 to 5, wherein the tablet comprises a diluent and wherein the diluent is present in an amount of from 6 to 21 wt% of the tablet core, optionally 8 to 18 wt% of the tablet core, optionally the diluent is present in an amount of from 9 to 13 wt% of the tablet core.
7. The **unit dose** pharmaceutical composition of any one of embodiments 3 to 6, wherein the tablet core comprises a binder selected from the group comprising: polyvinyl alcohol, a natural gum, gelatin, a polymethacrylate, cellulose or a derivate thereof (e.g., ethyl cellulose) and copovidone, or any combination thereof; optionally wherein the binder is selected from copovidone and polyvinyl alcohol.
8. The **unit dose** pharmaceutical composition of any one of embodiments 3 to 7, wherein the tablet core comprises a binder and wherein the binder is present in an amount of from 1 to 7 wt%, of the tablet core, optionally, wherein the binder is present in an amount of from 2 to 6 wt%, of the tablet core, optionally wherein the binder is present in an amount of from 3 to 5 wt% of the tablet core.
9. The **unit dose** pharmaceutical composition of any one of embodiments 3 to 8, wherein the tablet core comprises a disintegrant selected from the group comprising: sodium carboxymethyl cellulose, cross-linked sodium carboxymethyl cellulose, bentonite, alginic acid or a derivative or salt thereof, and crospovidone, or any combination thereof, optionally wherein the disintegrant is selected from crospovidone and cross-linked sodium carboxymethyl cellulose.
10. The **unit dose** pharmaceutical composition of any one of embodiments 3 to 9, wherein the tablet core comprises a disintegrant and wherein the disintegrant is present in an amount of from 0.01 to 2 wt% of the tablet core; optionally wherein the disintegrant is present in an amount of from 0.1 to 1 wt% of the tablet core.
11. The **unit dose** pharmaceutical composition of any one of embodiments 3 to 10, wherein the tablet core comprises a glidant selected from the group comprising: talc, starch, magnesium oxide, silica, silicon dioxide, a silicate or a salt thereof such as magnesium or calcium silicate, or any combination thereof; optionally wherein the glidant is selected from silica, colloidal silicon dioxide, talc and magnesium oxide.
12. The **unit dose** pharmaceutical composition of any one of embodiments 3 to 11, wherein the tablet core comprises a glidant and wherein the glidant is present in an amount of from 0.1 to 2 wt% of the tablet core, optionally wherein the glidant is present in an amount of from 0.5 to 1.5 wt% of the tablet core, further optionally, wherein the glidant is present in an amount of from 0.7 to 1.2 wt% of the tablet core.
13. The **unit dose** pharmaceutical composition of any one of embodiments 3 to 12, wherein the tablet core comprises a lubricant selected from the group comprising: stearic acid or a salt thereof such as magnesium, calcium or zinc stearate, polyethylene glycol, fumaric acid or a salt thereof, glyceryl behenate, glyceryl palmitostearate, wax, and sodium benzoate, or any combination thereof; optionally wherein the lubricant is selected from stearic acid or a salt thereof, fumaric acid or a salt thereof, and polyethylene glycol.
14. The **unit dose** pharmaceutical composition of any one of embodiments 3 to 13, wherein the tablet core comprises a lubricant and the lubricant is present in an amount of from 0.1 to 2 wt% of the tablet core, optionally wherein the lubricant is present in an amount of from 0.5 to 1.5 wt% of the tablet core, further optionally, wherein the lubricant is present in an amount of from 0.7 to 1.2 wt% of the tablet core.
15. The **unit dose** pharmaceutical composition of any one of embodiments 3 to 14, wherein the tablet core comprises microcrystalline cellulose, copovidone, colloidal silicon dioxide, magnesium stearate, and optionally crospovidone.
16. The **unit dose** pharmaceutical composition according to any preceding embodiment,
   wherein the tablet core comprises:
   microcrystalline cellulose in an amount of from 9 to 13 wt% of the tablet core,
   copovidone in an amount of from 3 to 4 wt% of the tablet core,
   colloidal silicon dioxide in an amount of from 0.5 to 1.5 wt% of the tablet core,
   magnesium stearate in an amount of from 0.5 to 1.5 wt% of the tablet core, and optionally, crospovidone in an amount of from 0 to 1 wt% of the tablet core.
17. The **unit dose** pharmaceutical composition of any preceding embodiment, further comprising a sub-coating between the tablet core and the gastro-resistant coating, optionally, wherein the sub-coating comprises hydroxypropyl cellulose.
18. The **unit dose** pharmaceutical composition of any preceding embodiment, wherein said gastro-resistant coating comprises methacrylic acid/methyl methacrylate (1:1) copolymer.
19. The **unit dose** pharmaceutical composition of any preceding embodiment, wherein said gastro-resistant coating comprises methacrylic acid/methyl methacrylate (1:2) copolymer.
20. The **unit dose** pharmaceutical composition of any preceding embodiment, wherein said gastro-resistant coating comprises a mixture of methacrylic acid/methyl methacrylate (1:1) copolymer and of methacrylic acid/methyl methacrylate (1:2) copolymer, optionally, the weight ratio of methacrylic acid/methyl methacrylate (1:1) copolymer to methacrylic acid/methyl methacrylate (1:2) copolymer is in the range of from 0.5:1 to 3:1, suitably, in a weight range of from 1:1 to 2:1.
21. The **unit dose** pharmaceutical composition of any preceding embodiment, wherein the gastro-resistant coating of the unit dose starts breaking or dissolving at or above a pH of 5, suitably at or above a pH of 6, preferably at or above a pH of 6.5, more preferably at a pH of 6.8.
22. The **unit dose** pharmaceutical composition of any preceding embodiment, wherein the tablet core has a hardness of greater than 70 N, suitably greater than 90 N, preferably greater than 120 N as determined in accordance with European Pharmacopoeia 2.9.8 or with US Pharmacopoeia monograph <1217>.
23. The **unit dose** pharmaceutical composition of any preceding embodiment, wherein the tablet has a friability of less than 1%, preferably less than 0.5%, more preferably of less than 0.1% as determined in accordance with US Pharmacopoeia monograph <1216> or with European Pharmacopoeia monograph 2.9.7
24. The **unit dose** pharmaceutical composition of any preceding embodiment, wherein the tablet core has a length of less than about 25 ± 0.5 mm, preferably less than about 24 ± 0.5 mm, more preferably of less than 23 mm ± 0.5.
25. The **unit dose** pharmaceutical composition of any preceding embodiment, wherein the tablet core has a thickness of less than about 11.5 mm ± 0.5 mm, preferably of less than about 10.5 mm ± 0.5 mm, more preferably of less than about 9.5 mm ± 0.5 mm, more preferably of less than or equal to about 8.5 mm ± 0.5 mm.
26. The **unit dose** pharmaceutical composition of any preceding embodiment, wherein the tablet core is formed by direct compression.
27. The **unit dose** pharmaceutical composition of any preceding embodiment, wherein the gastro-resistant coating is formulated for delayed release, optionally, wherein the gastro-resistant coating is formulated for release in the ileum.
28. The **unit dose** pharmaceutical composition of any preceding embodiment, wherein the tablet core comprises colesevelam hydrochloride.
29. The unit dose pharmaceutical composition of any preceding embodiment, wherein the pharmaceutical composition releases not more than about 50% of the amount of colesevelam, or a pharmaceutically acceptable salt thereof, when the pharmaceutical composition is placed in a USP dissolution apparatus II complying with USP <711> for 1 hour, optionally wherein the pharmaceutical composition releases not less than about 80% of the amount of colesevelam, or a pharmaceutically acceptable salt thereof, when the pharmaceutical composition is placed in a USP dissolution apparatus II complying with USP <711> for 2 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.
30. The unit dose pharmaceutical composition of any preceding embodiment, wherein the pharmaceutical composition releases not more than about 45% of the amount of colesevelam, or a pharmaceutically acceptable salt thereof, when the pharmaceutical composition is placed in a USP dissolution apparatus II complying with USP <711> for 1 hour, optionally wherein the pharmaceutical composition releases not less than about 80% of the amount of colesevelam, or a pharmaceutically acceptable salt thereof, when the pharmaceutical composition is placed in a USP dissolution apparatus II complying with USP <711> for 2 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.
31. The unit dose pharmaceutical composition of any preceding embodiment, wherein the pharmaceutical composition releases not more than about 40% of the amount of colesevelam, or a pharmaceutically acceptable salt thereof, when the pharmaceutical composition is placed in a USP dissolution apparatus II complying with USP <711> for 1 hour, optionally wherein the pharmaceutical composition releases not less than about 80% of the amount of colesevelam, or a pharmaceutically acceptable salt thereof, when the pharmaceutical composition is placed in a USP dissolution apparatus II complying with USP <711> for 2 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.
32. The unit dose pharmaceutical composition of any preceding embodiment, wherein the pharmaceutical composition releases not more than about 35% of the amount of colesevelam, or a pharmaceutically acceptable salt thereof, when the pharmaceutical composition is placed in a USP dissolution apparatus II complying with USP <711> for 1 hour, optionally wherein the pharmaceutical composition releases not less than about 80% of the amount of colesevelam, or a pharmaceutically acceptable salt thereof, when the pharmaceutical composition is placed in a USP dissolution apparatus II complying with USP <711> for 2 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.
33. The unit dose pharmaceutical composition of any preceding embodiment, wherein the pharmaceutical composition releases not more than about 30% of the amount of colesevelam, or a pharmaceutically acceptable salt thereof, when the pharmaceutical composition is placed in a USP dissolution apparatus II complying with USP <711> for 1 hour, optionally wherein the pharmaceutical composition releases not less than about 80% of the amount of colesevelam, or a pharmaceutically acceptable salt thereof, when the pharmaceutical composition is placed in a USP dissolution apparatus II complying with USP <711> for 2 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.
34. The unit dose pharmaceutical composition of any preceding embodiment, wherein the pharmaceutical composition releases not more than about 25% of the amount of colesevelam, or a pharmaceutically acceptable salt thereof, when the pharmaceutical composition is placed in a USP dissolution apparatus II complying with USP <711> for 1 hour, optionally wherein the pharmaceutical composition releases not less than about 80% of the amount of colesevelam, or a pharmaceutically acceptable salt thereof, when the pharmaceutical composition is placed in a USP dissolution apparatus II complying with USP <711> for 2 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.
35. The unit dose pharmaceutical composition of any preceding embodiment, wherein the pharmaceutical composition releases not more than about 20% of the amount of colesevelam, or a pharmaceutically acceptable salt thereof, when the pharmaceutical composition is placed in a USP dissolution apparatus II complying with USP <711> for 1 hour, optionally wherein the pharmaceutical composition releases not less than about 80% of the amount of colesevelam, or a pharmaceutically acceptable salt thereof, when the pharmaceutical composition is placed in a USP dissolution apparatus II complying with USP <711> for 2 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.
36. The unit dose pharmaceutical composition of any one of embodiments 1 to 28, wherein the pharmaceutical composition releases not more than about 50% of the amount of colesevelam, or a pharmaceutically acceptable salt thereof, when the pharmaceutical composition is placed in a USP dissolution apparatus II complying with USP <711> for 1 hour, optionally wherein the pharmaceutical composition releases not less than about 80% of the amount of colesevelam, or a pharmaceutically acceptable salt thereof, when the pharmaceutical composition is placed in a USP dissolution apparatus II complying with USP <711> for 3 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.
37. The unit dose pharmaceutical composition of any one of embodiments 1 to 28, wherein the pharmaceutical composition releases not more than about 45% of the amount of colesevelam, or a pharmaceutically acceptable salt thereof, when the pharmaceutical composition is placed in a USP dissolution apparatus II complying with USP <711> for 1 hour, optionally wherein the pharmaceutical composition releases not less than about 80% of the amount of colesevelam, or a pharmaceutically acceptable salt thereof, when the pharmaceutical composition is placed in a USP dissolution apparatus II complying with USP <711> for 3 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.
38. The unit dose pharmaceutical composition of any one of embodiments 1 to 28, wherein the pharmaceutical composition releases not more than about 40% of the amount of colesevelam, or a pharmaceutically acceptable salt thereof, when the pharmaceutical composition is placed in a USP dissolution apparatus II complying with USP <711> for 1 hour, optionally wherein the pharmaceutical composition releases not less than about 80% of the amount of colesevelam, or a pharmaceutically acceptable salt thereof, when the pharmaceutical composition is placed in a USP dissolution apparatus II complying with USP <711> for 3 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.
39. The unit dose pharmaceutical composition of any one of embodiments 1 to 28, wherein the pharmaceutical composition releases not more than about 35% of the amount of colesevelam, or a pharmaceutically acceptable salt thereof, when the pharmaceutical composition is placed in a USP dissolution apparatus II complying with USP <711> for 1 hour, optionally wherein the pharmaceutical composition releases not less than about 80% of the amount of colesevelam, or a pharmaceutically acceptable salt thereof, when the pharmaceutical composition is placed in a USP dissolution apparatus II complying with USP <711> for 3 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.
40. The unit dose pharmaceutical composition of any one of embodiments 1 to 28, wherein the pharmaceutical composition releases not more than about 30% of the amount of colesevelam, or a pharmaceutically acceptable salt thereof, when the pharmaceutical composition is placed in a USP dissolution apparatus II complying with USP <711> for 1 hour, optionally wherein the pharmaceutical composition releases not less than about 80% of the amount of colesevelam, or a pharmaceutically acceptable salt thereof, when the pharmaceutical composition is placed in a USP dissolution apparatus II complying with USP <711> for 3 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.
41. The unit dose pharmaceutical composition of any one of embodiments 1 to 28, wherein the pharmaceutical composition releases not more than about 25% of the amount of colesevelam, or a pharmaceutically acceptable salt thereof, when the pharmaceutical composition is placed in a USP dissolution apparatus II complying with USP <711> for 1 hour, optionally wherein the pharmaceutical composition releases not less than about 80% of the amount of colesevelam, or a pharmaceutically acceptable salt thereof, when the pharmaceutical composition is placed in a USP dissolution apparatus II complying with USP <711> for 3 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.
42. The unit dose pharmaceutical composition of any one of embodiments 1 to 28, wherein the pharmaceutical composition releases not more than about 20% of the amount of colesevelam, or a pharmaceutically acceptable salt thereof, when the pharmaceutical composition is placed in a USP dissolution apparatus II complying with USP <711> for 1 hour, optionally wherein the pharmaceutical composition releases not less than about 80% of the amount of colesevelam, or a pharmaceutically acceptable salt thereof, when the pharmaceutical composition is placed in a USP dissolution apparatus II complying with USP <711> for 3 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.
43. The unit dose pharmaceutical composition of any one of embodiments 1 to 28, wherein the pharmaceutical composition releases not more than about 50% of the amount of colesevelam, or a pharmaceutically acceptable salt thereof, when the pharmaceutical composition is placed in a USP dissolution apparatus II complying with USP <711> for 1 hour, optionally wherein the pharmaceutical composition releases not less than about 80% of the amount of colesevelam, or a pharmaceutically acceptable salt thereof, when the pharmaceutical composition is placed in a USP dissolution apparatus II complying with USP <711> for 4 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.
44. The unit dose pharmaceutical composition of any one of embodiments 1 to 28, wherein the pharmaceutical composition releases not more than about 45% of the amount of colesevelam, or a pharmaceutically acceptable salt thereof, when the pharmaceutical composition is placed in a USP dissolution apparatus II complying with USP <711> for 1 hour, optionally wherein the pharmaceutical composition releases not less than about 80% of the amount of colesevelam, or a pharmaceutically acceptable salt thereof, when the pharmaceutical composition is placed in a USP dissolution apparatus II complying with USP <711> for 4 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.
45. The unit dose pharmaceutical composition of any one of embodiments 1 to 28, wherein the pharmaceutical composition releases not more than about 40% of the amount of colesevelam, or a pharmaceutically acceptable salt thereof, when the pharmaceutical composition is placed in a USP dissolution apparatus II complying with USP <711> for 1 hour, optionally wherein the pharmaceutical composition releases not less than about 80% of the amount of colesevelam, or a pharmaceutically acceptable salt thereof, when the pharmaceutical composition is placed in a USP dissolution apparatus II complying with USP <711> for 4 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.
46. The unit dose pharmaceutical composition of any one of embodiments 1 to 28, wherein the pharmaceutical composition releases not more than about 35% of the amount of colesevelam, or a pharmaceutically acceptable salt thereof, when the pharmaceutical composition is placed in a USP dissolution apparatus II complying with USP <711> for 1 hour, optionally wherein the pharmaceutical composition releases not less than about 80% of the amount of colesevelam, or a pharmaceutically acceptable salt thereof, when the pharmaceutical composition is placed in a USP dissolution apparatus II complying with USP <711> for 4 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.
47. The unit dose pharmaceutical composition of any one of embodiments 1 to 28, wherein the pharmaceutical composition releases not more than about 30% of the amount of colesevelam, or a pharmaceutically acceptable salt thereof, when the pharmaceutical composition is placed in a USP dissolution apparatus II complying with USP <711> for 1 hour, optionally wherein the pharmaceutical composition releases not less than about 80% of the amount of colesevelam, or a pharmaceutically acceptable salt thereof, when the pharmaceutical composition is placed in a USP dissolution apparatus II complying with USP <711> for 4 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.
48. The unit dose pharmaceutical composition of any one of embodiments 1 to 28, wherein the pharmaceutical composition releases not more than about 25% of the amount of colesevelam, or a pharmaceutically acceptable salt thereof, when the pharmaceutical composition is placed in a USP dissolution apparatus II complying with USP <711> for 1 hour, optionally wherein the pharmaceutical composition releases not less than about 80% of the amount of colesevelam, or a pharmaceutically acceptable salt thereof, when the pharmaceutical composition is placed in a USP dissolution apparatus II complying with USP <711> for 4 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.
49. The unit dose pharmaceutical composition of any one of embodiments 1 to 28, wherein the pharmaceutical composition releases not more than about 20% of the amount of colesevelam, or a pharmaceutically acceptable salt thereof, when the pharmaceutical composition is placed in a USP dissolution apparatus II complying with USP <711> for 1 hour, optionally wherein the pharmaceutical composition releases not less than about 80% of the amount of colesevelam, or a pharmaceutically acceptable salt thereof, when the pharmaceutical composition is placed in a USP dissolution apparatus II complying with USP <711> for 4 hours, wherein the dissolution apparatus comprises an aqueous phosphate buffer (optionally 1000 ml of said buffer) at pH 6.8 and at a temperature of 37 ± 0.5 °C, and wherein the aqueous solution is stirred at a rate of 100 rpm.
50. A method of manufacturing a unit dose pharmaceutical composition for oral administration comprising the steps of:
   providing a tablet core comprising colesevelam or a pharmaceutically acceptable salt thereof,
   wherein the colesevelam or a pharmaceutically acceptable salt thereof is at least 66 wt% of the tablet core,
   wherein the colesevelam or a pharmaceutically acceptable salt thereof is present in the **tablet core** in an amount of from 850 mg to 1200 mg; and
   coating the tablet core with a gastro-resistant coating, and optionally a subcoating.
51. The method of manufacture of embodiment 50, comprising the steps of:
   (i) combining colesevelam and/or pharmaceutically acceptable salts thereof, with a diluent, a binder, a glidant, a lubricant and optionally a disintegrant to form a mixture;
   (ii) tableting the mixture of step (i) to form tablet cores; and
   (iii) coating the tablet cores formed in step (ii) with a gastro-resistant coating, and optionally a subcoating.
52. The method of manufacture of embodiment 50 or 51, comprising bulk manufacture of a plurality of unit dose pharmaceutical compositions for oral administration, wherein the plurality of unit doses has a uniformity of mass of less than ±3%, preferably less than ±2%, more preferably less than ±1.7%.
53. The method of manufacture of any one of embodiments 50 to 52, comprising providing a unit dose pharmaceutical composition for oral administration according to any one of embodiments 1 to 34.
54. A unit dose pharmaceutical composition for oral administration according to any one of embodiments 1 to 49 for use in a method of treating bile acid diarrhea, irritable bowel syndrome, optionally, irritable bowel syndrome subtype diarrhea (IBS-D), colitis, such as microscopic colitis, and/or bile acid malabsorption in a patient in need thereof, optionally, wherein the method comprises administering one of said unit dose pharmaceutical compositions for oral administration to said patient, optionally, wherein the method comprises administering more than one of said unit dose pharmaceutical compositions for oral administration to said patient.
55. A method of treating bile acid diarrhea, irritable bowel syndrome, optionally, irritable bowel syndrome subtype diarrhea (IBS-D), colitis, such as microscopic colitis, and/or bile acid malabsorption in a patient in need thereof, comprising administering to said patient a unit dose pharmaceutical composition for oral administration according to any one of embodiments 1 to 49, optionally, wherein the method comprises administering one of said unit dose pharmaceutical compositions for oral administration to said patient, optionally, wherein the method comprises administering more than one of said unit dose pharmaceutical compositions for oral administration to said patient.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

## Claims

1. A unit dose pharmaceutical composition for oral administration comprising a tablet core and a gastro-resistant coating covering the tablet core,
wherein the tablet core comprises colesevelam or a pharmaceutically acceptable salt thereof,
wherein the colesevelam or a pharmaceutically acceptable salt thereof is at least 66 wt% of the tablet core, and
wherein the colesevelam or a pharmaceutically acceptable salt thereof is present in the tablet core in an amount of from about 700 mg to 1400 mg;
optionally wherein the colesevelam or a pharmaceutically acceptable salt thereof is at least 70 wt%, or at least 71 wt%, or at least 75 wt%, or at least 76 wt%, or at least 80 wt%, or at least 81 wt% of the tablet core, optionally, at least 82 wt% of the tablet core;
optionally wherein the tablet core comprises colesevelam hydrochloride.

2. The **unit dose** pharmaceutical composition of claim 1, wherein the tablet core comprises a diluent or mixture of two or more diluents selected from the group comprising: lactose, sugars such as sucrose and fructose, dextrose, dextrates, dextrans, a polyol such as sorbitol, mannitol and xylitol, dibasic calcium phosphate, tribasic calcium phosphate, calcium sulphate, sodium chloride, starch, such as pregelatinized starch, cellulose or a derivative thereof, such as methyl, ethyl, or hydroxyethyl cellulose; optionally wherein the diluent is selected from cellulose or derivatives thereof, lactose, calcium phosphate, starch, modified starch and mannitol;
optionally wherein the diluent is microcrystalline cellulose, optionally the diluent is a porous microcrystalline cellulose having an average particle size of in the range of from 80 to 110 µm and an angle of repose of less than about 40°, preferably, wherein the porous microcrystalline cellulose has an average particle size of about 90 µm and an angle of repose of about 34;
optionally wherein the diluent is present in an amount of from 6 to 21 wt% of the tablet core, optionally 8 to 18 wt% of the tablet core, optionally the diluent is present in an amount of from 9 to 13 wt% of the tablet core.

3. The **unit dose** pharmaceutical composition of claim 1 or 2, wherein the tablet core comprises a binder, wherein the binder is selected from the group comprising: polyvinyl alcohol, a natural gum, gelatin, a polymethacrylate, cellulose or a derivative thereof such as ethyl cellulose, and copovidone, or any combination thereof; optionally wherein the binder is selected from copovidone and polyvinyl alcohol;
optionally wherein the binder is present in an amount of from 1 to 7 wt%, of the tablet core, optionally wherein the binder is present in an amount of from 2 to 6 wt%, of the tablet core, optionally wherein the binder is present in an amount of from 3 to 5 wt% of the tablet core.

4. The **unit dose** pharmaceutical composition of any one of claims 1 to 3, wherein the tablet core comprises a disintegrant selected from the group comprising: sodium carboxymethyl cellulose, cross-linked sodium carboxymethyl cellulose, bentonite, alginic acid or a derivative or salt thereof, and crospovidone, or any combination thereof, optionally wherein the disintegrant is selected from crospovidone and cross-linked sodium carboxymethyl cellulose; optionally wherein the disintegrant is present in an amount of from 0.01 to 2 wt% of the tablet core; optionally wherein the disintegrant is present in an amount of from 0.1 to 1 wt% of the tablet core.

5. The **unit dose** pharmaceutical composition of any one of claims 1 to 4, wherein the tablet core comprises a glidant selected from the group comprising: talc, starch, magnesium oxide, silica, silicon dioxide, a silicate or a salt thereof such as magnesium or calcium silicate, or any combination thereof; optionally wherein the glidant is selected from silica, colloidal silicon dioxide, talc and magnesium oxide;
optionally wherein the glidant is present in an amount of from 0.1 to 2 wt% of the tablet core, optionally wherein the glidant is present in an amount of from 0.5 to 1.5 wt% of the tablet core, further optionally, wherein the glidant is present in an amount of from 0.7 to 1.2 wt% of the tablet core.

6. The **unit dose** pharmaceutical composition of any one of claims 1 to 5, wherein the tablet core comprises a lubricant selected from the group comprising: stearic acid or a salt thereof such as magnesium, calcium or zinc stearate, polyethylene glycol, fumaric acid or a salt thereof, glyceryl behenate, glyceryl palmitostearate, wax, and sodium benzoate, or any combination thereof; optionally wherein the lubricant is selected from stearic acid or a salt thereof, fumaric acid or a salt thereof, and polyethylene glycol;
optionally wherein the lubricant is present in an amount of from 0.1 to 2 wt% of the tablet core, optionally wherein the lubricant is present in an amount of from 0.5 to 1.5 wt% of the tablet core, further optionally, wherein the lubricant is present in an amount of from 0.7 to 1.2 wt% of the tablet core.

7. The **unit dose** pharmaceutical composition of any one of claims 1 to 6, wherein the tablet core comprises microcrystalline cellulose, copovidone, colloidal silicon dioxide, magnesium stearate, and optionally crospovidone;
optionally wherein the tablet core comprises:
microcrystalline cellulose in an amount of from 9 to 13 wt% of the tablet core,
copovidone in an amount of from 3 to 4 wt% of the tablet core,
colloidal silicon dioxide in an amount of from 0.5 to 1.5 wt% of the tablet core,
magnesium stearate in an amount of from 0.5 to 1.5 wt% of the tablet core, and optionally, crospovidone in an amount of from 0 to 1 wt% of the tablet core.

8. The **unit dose** pharmaceutical composition of any preceding claim, further comprising a subcoating between the tablet core and the gastro-resistant coating, optionally, wherein the subcoating comprises hydroxypropyl cellulose;
optionally wherein said gastro-resistant coating comprises methacrylic acid/methyl methacrylate (1:1) copolymer;
optionally wherein said gastro-resistant coating comprises methacrylic acid/methyl methacrylate (1:2) copolymer;
optionally wherein said gastro-resistant coating comprises a mixture of methacrylic acid/methyl methacrylate (1:1) copolymer and of methacrylic acid/methyl methacrylate (1:2) copolymer, optionally, the weight ratio of methacrylic acid/methyl methacrylate (1:1) copolymer to methacrylic acid/methyl methacrylate (1:2) copolymer is in the range of from 0.5:1 to 3:1, suitably, in a weight range of from 1:1 to 2:1;
optionally wherein the gastro-resistant coating of the unit dose starts breaking or dissolving at or above a pH of 5, suitably at or above a pH of 6, preferably at or above a pH of 6.5, more preferably at a pH of 6.8
optionally wherein the gastro-resistant coating is formulated for delayed release, optionally, wherein the gastro-resistant coating is formulated for release in the ileum.

9. The **unit dose** pharmaceutical composition of any preceding claim, wherein the tablet core has a hardness of greater than 70 N, suitably greater than 90 N, preferably greater than 120 N as determined in accordance with European Pharmacopoeia 2.9.8 or with US Pharmacopoeia monograph <1217>.

10. The unit dose pharmaceutical composition of any preceding claim, wherein the tablet core has a friability of less than 1%, preferably less than 0.5%, more preferably of less than 0.1% as determined in accordance with US Pharmacopoeia monograph <1216> or with European Pharmacopoeia monograph 2.9.7.

11. The **unit dose** pharmaceutical composition of any preceding claim, wherein the tablet core has a length of less than about 25 ± 0.5 mm, preferably less than about 24 ± 0.5 mm, more preferably of less than 23 mm ± 0.5; optionally wherein the tablet core has a thickness of less than about 11.5 mm ± 0.5 mm, preferably of less than about 10.5 mm ± 0.5 mm, more preferably of less than about 9.5 mm ± 0.5 mm, more preferably of less than or equal to about 8.5 mm ± 0.5 mm; optionally wherein the tablet core is formed by direct compression.

12. The **unit dose** pharmaceutical composition of any preceding claim, wherein the pharmaceutical composition releases not more than about 50% of the amount of colesevelam, or a pharmaceutically acceptable salt thereof, when the pharmaceutical composition is placed in a USP dissolution apparatus II complying with USP <711> for 1 hour, optionally wherein the pharmaceutical composition releases not less than about 80% of the amount of colesevelam, or a pharmaceutically acceptable salt thereof, when the pharmaceutical composition is placed in a USP dissolution apparatus II complying with USP <711> for 2 hours, wherein the dissolution apparatus comprises an aqueous phosphate buffer (optionally 1000 mL of said buffer) at pH 6.8 and at a temperature of 37 ± 0.5 °C, and wherein the aqueous solution is stirred at a rate of 100 rpm.

13. The unit dose pharmaceutical composition of any preceding claim wherein the colesevelam or a pharmaceutically acceptable salt thereof is present in an amount of from 76 wt% to 90 wt% of the tablet core, optionally, from 80 wt% to 90 wt%, optionally from 81 wt% to 90 wt%, such as from 82 wt% to 90 wt% of the tablet core and wherein the colesevelam or a pharmaceutically acceptable salt thereof is present in the tablet core in an amount of from about 700 mg to 1400 mg, wherein the tablet core has a hardness in the range of from 70 N to 450 N, optionally, from 80 N to 400 N, further optionally, from 90 N to 370 N, such as from 100 N to 330 N as determined in accordance with European Pharmacopoeia 2.9.8 or with US Pharmacopoeia monograph <1217>; and wherein the tablet core has a friability of from 0.001% to 1%, optionally, from 0.01 to 0.5%, optionally from 0.01 to 0.1% as determined in accordance with US Pharmacopoeia monograph <1216> or with European Pharmacopoeia monograph 2.9.7.

14. The unit dose pharmaceutical composition of any preceding claim wherein the colesevelam or a pharmaceutically acceptable salt thereof is present in an amount of from 76 wt% to 90 wt% of the tablet core, optionally, from 80 wt% to 90 wt%, optionally from 81 wt% to 90 wt%, such as from 82 wt% to 90 wt% of the tablet core and wherein the colesevelam or a pharmaceutically acceptable salt thereof is present in the tablet core in an amount of from about 700 mg to 1400 mg;
wherein tablet core comprises a diluent, wherein the diluent comprises a porous microcrystalline cellulose having an average particle size of in the range of from 80 to 110 µm and an angle of repose of less than about 40°, preferably, wherein the porous microcrystalline cellulose has an average particle size of about 90 µm and an angle of repose of about 34°, wherein the diluent is present in an amount of from 8 to 18 wt% of the tablet core;
wherein the tablet core has a hardness in the range of from 70 N to 450 N, optionally, from 80 N to 400 N, further optionally, from 90 N to 370 N, such as from 100 N to 330 N as determined in accordance with European Pharmacopoeia 2.9.8 or with US Pharmacopoeia monograph <1217>; and wherein the tablet core has a friability of from 0.001% to 1%, optionally, from 0.01 to 0.5%, optionally from 0.01 to 0.1% as determined in accordance with US Pharmacopoeia monograph <1216> or with European Pharmacopoeia monograph 2.9.7.

15. A method of manufacturing a unit dose pharmaceutical composition for oral administration comprising the steps of:
providing a tablet core comprising colesevelam or a pharmaceutically acceptable salt thereof, wherein the colesevelam or a pharmaceutically acceptable salt thereof is at least 66 wt% of the tablet core, wherein the colesevelam or a pharmaceutically acceptable salt thereof is present in the **tablet core** in an amount of from 850 mg to 1200 mg; and
coating the tablet core with a gastro-resistant coating, and optionally a subcoating;
optionally wherein the method further comprises the steps of:
(i) combining colesevelam and/or pharmaceutically acceptable salts thereof, with a diluent, a binder, a glidant, a lubricant and optionally a disintegrant to form a mixture;
(ii) tableting the mixture of step (i) to form tablet cores; and
(iii) coating the tablet cores formed in step (ii) with a gastro-resistant coating, and optionally a subcoating.

16. The method of manufacture of claim 15, comprising bulk manufacture of a plurality of unit dose pharmaceutical compositions for oral administration, wherein the plurality of unit doses has a uniformity of mass of less than ±3%, preferably less than ±2%, more preferably less than ±1.7%.

17. A **unit dose** pharmaceutical composition for oral administration according to any one of claims 1 to 14 for use in a method of treating bile acid diarrhea, irritable bowel syndrome, optionally, irritable bowel syndrome subtype diarrhea (IBS-D), colitis, such as microscopic colitis, and/or bile acid malabsorption in a patient in need thereof, optionally, wherein the method comprises administering one of said unit dose pharmaceutical compositions for oral administration to said patient, optionally, wherein the method comprises administering more than one of said unit dose pharmaceutical compositions for oral administration to said patient.
